(19) Europäisches Patentamt
European Patent Office
Office européen des brevets

(11) Veröffentlichungsnummer : **0 542 685 A1**

# (12) EUROPÄISCHE PATENTANMELDUNG

(21) Anmeldenummer : **92810853.9**

(22) Anmeldetag : **04.11.92**

(51) Int. Cl.[5] : **C07D 239/54,** C07D 239/70, C07D 405/10, C07D 239/36, C07C 275/42, C07C 237/18, A01N 43/54

(30) Priorität : **13.11.91 CH 3302/91**

(43) Veröffentlichungstag der Anmeldung : **19.05.93 Patentblatt 93/20**

(84) Benannte Vertragsstaaten : **AT BE CH DE DK ES FR GB GR IE IT LI LU NL PT SE**

(71) Anmelder : **CIBA-GEIGY AG**
**Klybeckstrasse 141**
**CH-4002 Basel (CH)**

(72) Erfinder : **Wenger, Jean, Dr.**
**Zwischen den Rainen 374**
**CH-4323 Wallbach (CH)**
Erfinder : **Winternitz, Paul, Dr.**
**Am Pfisterhölzli 50**
**CH-8606 Greifensee (CH)**
Erfinder : **Zeller, Martin, Dr.**
**Kronengasse 7**
**CH-5400 Baden (CH)**

## (54) Neue 3-Aryluracil-Derivate und deren Verwendung zur Unkrautbekämpfung.

(57) 3-Aryluracil-Derivate der Formel I

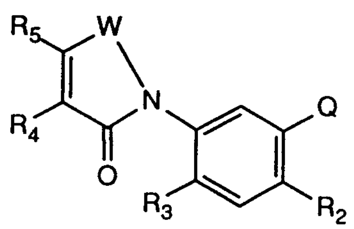

(I),

worin
-W- die Gruppe

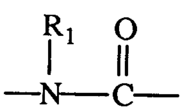 oder 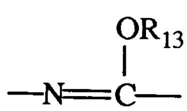

bedeutet, wobei die Bindung an das Ring-Stickstoffatom über das C-Atom erfolgt ;

$R_1$ Wasserstoff, $C_1$-$C_4$-Alkyl, $C_1$-$C_4$-Haloalkyl, $C_3$- oder $C_4$-Alkenyl oder $C_3$- oder $C_4$-Alkinyl ;
$R_2$ Halogen oder Cyano ;
$R_3$ Wasserstoff oder Halogen ;
$R_4$ Wasserstoff, Halogen oder $C_1$-$C_4$-Alkyl ;
$R_5$ $C_1$-$C_4$-Alkyl oder $C_1$-$C_4$-Haloalkyl ist ; oder $R_4$ und $R_5$ zusammen $-(CH_2)_n-$ sind ;
n die Zahl 3 oder 4 ;
$R_{13}$ $C_1$-$C_4$-Alkyl, $C_3$- oder $C_4$-Alkenyl oder $C_3$- oder $C_4$-Alkinyl ist ; und
Q eine der Gruppen a) bis e) bedeutet :

a)

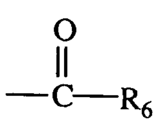 (1),

wobei $R_6$ Wasserstoff, $C_1$-$C_8$-Alkyl, $C_3$-$C_7$-Cycloalkyl oder $C_3$-$C_7$-Cycloalkyl-$C_1$-$C_4$-alkyl ist ;

b)

EP 0 542 685 A1

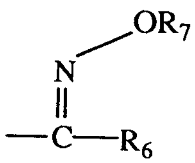 (2),

wobei $R_6$, die unter a) angegebene Bedeutung hat;

$R_7$ Wasserstoff, $C_1$-$C_8$-Alkyl, $C_2$-$C_8$-Alkenyl, $C_2$-$C_8$-Alkinyl, $C_3$-$C_7$-Cycloalkyl, Phenyl, mit Halogen, $C_1$-$C_4$-Alkyl oder $C_1$-$C_4$-Alkoxy substituiertes Phenyl, Benzyl oder mit Halogen, $C_1$-$C_4$-Alkyl oder $C_1$-$C_4$-Alkoxy substituiertes Benzyl ist;

c)

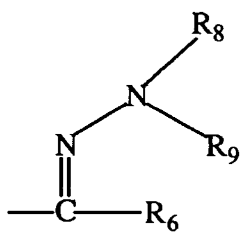 (3),

wobei $R_6$ die unter a) angegebene Bedeutung hat;

$R_6$ und $R_9$, unabhängig voneinander, Wasserstoff, $C_1$-$C_8$-Alkyl, $C_2$-$C_8$-Alkenyl, $C_2$-$C_8$-Alkinyl, Phenyl, mit Halogen, $C_1$-$C_4$-Alkyl oder $C_1$-$C_4$-Alkoxy substituiertes Phenyl, Benzyl, mit Halogen, $C_1$-$C_4$-Alkyl oder $C_1$-$C_4$-Alkoxy substituiertes Benzyl; oder $R_6$ und $R_9$ zusammen $-(CH_2)_m-$ sind; und

m die Zahl 3, 4 oder 5 ist;

d)

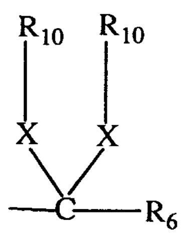 (4),

wobei $R_6$ die unter a) angegebene Bedeutung hat;

$R_{10}$ $C_1$-$C_8$-Alkyl, $C_2$-$C_8$-Alkenyl, $C_2$-$C_8$-Alkinyl, $C_1$-$C_4$-Alkoxy-$C_1$-$C_6$-alkyl, Hydroxy-$C_1$-$C_6$-alkyl oder $C_1$-$C_8$-Haloalkyl; und

X Sauerstoff oder Schwefel ist;

e)

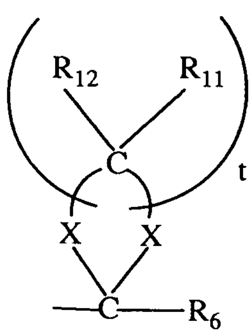 (5),

wobei $R_6$ die unter a) angegebene Bedeutung hat;

$R_{11}$, und $R_{12}$, unabhängig voneinander, Wasserstoff oder $C_1$-$C_4$-Alkyl sind;

t die Zahl 2, 3 oder 4; und

X Sauerstoff oder Schwefel bedeutet; und,

sofern $R_1$ Wasserstoff ist, auch die agrochemisch verträglichen Salze von Verbindungen der Formel I zeigen herbizide Wirkung. Sie eignen sich als Wirkstoff in Unkrautbekämpfungsmitteln.

Die vorliegende Erfindung betrifft neue 3-Aryluracile der Formel I

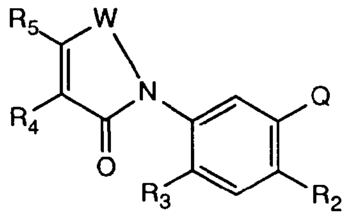

(I),

worin
-W- die Gruppe

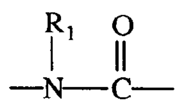 oder 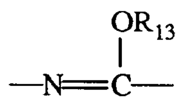

bedeutet, wobei die Bindung an das Ring-Stickstoffatom über das C-Atom erfolgt;

- $R_1$ Wasserstoff, $C_1$-$C_4$-Alkyl, $C_1$-$C_4$-Haloalkyl, $C_3$- oder $C_4$-Alkenyl oder $C_3$- oder $C_4$-Alkinyl;
- $R_2$ Halogen oder Cyano;
- $R_3$ Wasserstoff oder Halogen;
- $R_4$ Wasserstoff, Halogen oder $C_1$-$C_4$-Alkyl;
- $R_5$ $C_1$-$C_4$-Alkyl oder $C_1$-$C_4$-Haloalkyl ist; oder $R_4$ und $R_5$ zusammen $-(CH_2)_n-$ sind;
- n die Zahl 3 oder 4;
- $R_{13}$ $C_1$-$C_4$-Alkyl, $C_3$- oder $C_4$-Alkenyl oder $C_3$- oder $C_4$-Alkinyl ist; und
- Q eine der Gruppen a) bis e) bedeutet:

a)

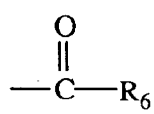 (1),

wobei $R_6$ Wasserstoff, $C_1$-$C_8$-Alkyl, $C_3$-$C_7$-Cycloalkyl oder $C_3$-$C_7$-Cycloalkyl-$C_1$-$C_4$-alkyl ist;

b)

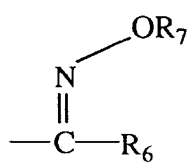 (2),

wobei $R_6$ die unter a) angegebene Bedeutung hat;

$R_7$ Wasserstoff, $C_1$-$C_8$-Alkyl, $C_2$-$C_8$-Alkenyl, $C_2$-$C_8$-Alkinyl, $C_3$-$C_7$-Cycloalkyl, Phenyl, mit Halogen, $C_1$-$C_4$-Alkyl oder $C_1$-$C_4$-Alkoxy substituiertes Phenyl, Benzyl oder mit Halogen, $C_1$-$C_4$-Alkyl oder $C_1$-$C_4$-Alkoxy substituiertes Benzyl ist;

c)

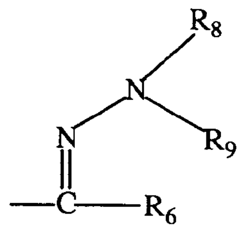

(3),

wobei $R_6$ die unter a) angegebene Bedeutung hat;

$R_8$ und $R_9$, unabhängig voneinander, Wasserstoff, $C_1$-$C_8$-Alkyl, $C_2$-$C_8$-Alkenyl, $C_2$-$C_8$-Alkinyl, Phenyl, mit Halogen, $C_1$-$C_4$-Alkyl oder $C_1$-$C_4$-Alkoxy substituiertes Phenyl, Benzyl, mit Halogen, $C_1$-$C_4$-Alkyl oder $C_1$-$C_4$-Alkoxy substituiertes Benzyl; oder $R_8$ und $R_9$ zusammen $-(CH_2)_m-$ sind; und

m die Zahl 3, 4 oder 5 ist;

d)

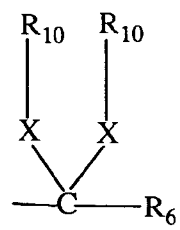

(4),

wobei $R_6$ die unter a) angegebene Bedeutung hat;

$R_{10}$ $C_1$-$C_8$-Alkyl, $C_2$-$C_8$-Alkenyl, $C_2$-$C_8$-Alkinyl, $C_1$-$C_4$-Alkoxy-$C_1$-$C_6$-alkyl, Hydroxy-$C_1$-$C_6$-alkyl oder $C_1$-$C_8$-Haloalkyl; und

X Sauerstoff oder Schwefel ist;

e)

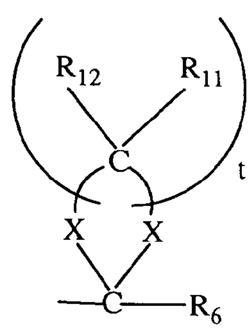

(5),

wobei $R_6$ die unter a) angegebene Bedeutung hat;

$R_{11}$ und $R_{12}$, unabhängig voneinander, Wasserstoff oder $C_1$-$C_4$-Alkyl sind;

t die Zahl 2, 3 oder 4; und

X Sauerstoff oder Schwefel bedeutet; und

sofern $R_1$ Wasserstoff ist, auch die agrochemisch verträglichen Salze von Verbindungen der Formel I.

Die erfindungsgemässen 3-Aryluracile der Formel I, in denen die Gruppe -W- in der Amid-Form Ia

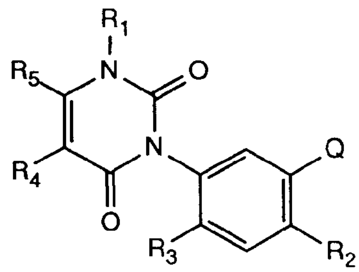

(Ia),

oder in der Iminoether-Form Ib

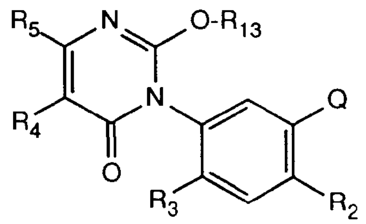

(Ib) vorliegen,

worin $R_1$, $R_2$, $R_3$, $R_4$, $R_5$, $R_{13}$ und Q die unter Formel I angegebenen Bedeutungen besitzen, sowie die möglichen Salze dieser Verbindungen, sind herbizid wirksam und eignen sich als Wirkstoffe von Unkrautbekämpfungsmitteln. Somit umfasst die vorliegende Erfindung auch Unkrautbekämpfungsmittel, welche die erfindungsgemässen Verbindungen als Wirkstoffe enthalten, Verfahren zur Herstellung dieser Verbindungen, sowie die Verwendung solcher Verbindungen beziehungsweise Mittel zur Bekämpfung von Unkräutern.

Andere herbizid wirksame 3-Aryluracil-Derivate sind z.B. bekannt aus der EP-A-0,195,346, worin Uracile mit Q als Carbonsäure- und Ester-Gruppen offenbart werden; EP-A-0,255,047, worin Uracile mit Q als Ether, (Thio) Carbonyloxy- und Sulfonyloxy-Gruppen offenbart werden; und EP-A-0,260,621, worin Uracile mit Q als Ether, Alkyl-carbonyloxy-, Alkoxy-carbonyloxy- und Alkoxycarbonyl-Gruppen offenbart werden.

In den Formeln I, Ia und Ib der erfindungsgemässen 3-Aryluracile beudeutet Halogen in den Definitionen der Reste $R_2$, $R_3$ und $R_4$ Fluor, Chlor, Brom und Jod. Die Alkyl-, Alkenyl- und Alkinyl-Reste $R_1$, $R_4$, $R_5$ und die entsprechenden als Substituenten in Q in Betracht kommenden Radikale $R_6$, $R_7$, $R_8$, $R_9$, $R_{10}$, $R_{11}$ und $R_{12}$ können geradkettig oder verzweigt sein, wobei dies auch für den Alkyl-Teil der Haloalkyl- und Alkoxy-Gruppen gilt. Als Beispiele solcher Alkyle seien Methyl, Ethyl, n-Propyl, i-Propyl, n-Butyl, i-Butyl, s-Butyl oder t-Butyl, als Beispiele für Alkenyle seien Allyl, Methallyl oder But-2-en-1-yl und als Beispiele für Alkinyle seien Propargyl, But-2-in-1-yl, But-3-in-1-yl und Pent-4-in-1-yl genannt. Alkoxyalkyl in den Definitionen des Restes $R_{10}$ bedeutet beispielsweise Methoxymethyl, Methoxyethyl oder Ethoxyethyl. Die Reste $R_{10}$ sind identisch. Eine Haloalkyl-Gruppe kann ein- oder mehrere (gleiche oder verschiedene) Halogenatome aufweisen, wobei als Beispiele einer mehrfach halogenierten Alkylgruppe Trifluormethyl und Pentafluorethyl genannt seien. Bei den als Substituenten in Betracht kommenden Cycloalkyl-Resten $R_6$ und $R_7$ handelt es sich beispielsweise um Cyclopropyl, Cyclobutyl, Cyclopentyl, Cyclohexyl oder Cycloheptyl.

Bei den Salzen der Verbindungen der Formel I handelt es sich insbesondere um Alkalimetallsalze, z.B. Natrium- und Kaliumsalze; Erdalkalimetallsalze, z.B. Calcium- und Magnesiumsalze; Ammoniumsalze, d.h. unsubstituierte Ammoniumsalze und mono- oder mehrfach-substituierte Ammoniumsalze, Z.B. Triethylammonium- und Methylammoniumsalze, sowie um Salze mit anderen organischen Basen, z.B. mit Pyridin.

Das mögliche Vorhandensein mindestens eines asymmetrischen Kohlenstoffatoms in den Verbindungen der Formel I hat zur Folge, dass die Verbindungen in optisch aktiven Einzelisomeren, als auch in Form von racemischen Gemischen auftreten können. In der vorliegenden Erfindung sind unter den Wirkstoffen der Formel I sowohl die reinen optischen Antipoden als auch die Racemate zu verstehen. Sofern nicht speziell auf die einzelnen optischen Antipoden hingewiesen wird, sind diejenigen racemischen Gemische unter der gegebenen Formel zu verstehen, die beim angegebenen Herstellungsverfahren entstehen. Durch das Vorliegen einer allfälligen aliphatischen C=C-Doppelbindung kann auch geometrische Isomerie auftreten. Zudem ist bei denjenigen Verbindungen der Formel I, in denen $R_1$ Wasserstoff bedeutet, nicht ausgeschlossen, dass Keto Enol-Tautomerie

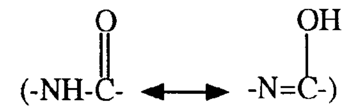

auftritt. Die Formel I soll alle diese möglichen isomeren Formen sowie Gemische davon umfassen.

Unter den Verbindungen der Formel I sind folgende Bedeutungen der folgenden Reste herauszuheben:

a) $R_1$ Wasserstoff, $C_1$-$C_4$-Alkyl, $C_1$-$C_4$-Haloalkyl, $C_3$- oder $C_4$-Alkenyl, oder $C_3$- oder $C_4$-Alkinyl, insbesondere Methyl, Allyl oder Propargyl; oder/und

b) $R_2$ Cyano, insbesondere Fluor, Chlor oder Brom; oder/und

c) $R_3$ Wasserstoff oder Fluor, oder/und

d) $R_4$ Methyl, insbesondere Wasserstoff oder Fluor, oder/und

e) $R_5$ Methyl, Trifluormethyl, Pentafluorethyl; oder $R_4$ und $R_5$ bilden zusammen eine Alkylenbrücke, wobei n die Zahl 4, insbesondere 3; oder/und

f) $R_7$ Wasserstoff, $C_1$-$C_4$-Alkyl, $C_3$-$C_5$-Alkenyl, $C_3$-$C_5$-Alkinyl oder Benzyl; oder/und

g) $R_{10}$ $C_1$-$C_5$-Alkyl, $C_3$-$C_5$-Alkenyl, $C_3$-$C_5$-Alkinyl oder Hydroxy-ethyl; X ist vorzugsweise Sauerstoff; oder/und

h) $R_{11}$ und $R_{12}$ unabhängig voneinander Methyl, insbesondere Wasserstoff, und t die Zahl 2 oder 3; oder/und

i) $R_{13}$ $C_1$- oder $C_2$-Alkyl, Allyl oder Propargyl.

Bevorzugt sind die 3-Aryluracil-Derivate der Formel I, worin $R_1$ $C_1$-$C_4$-Alkyl, $C_1$-$C_4$-Haloalkyl, $C_3$- oder $C_4$-Alkenyl oder $C_3$- oder $C_4$-Alkinyl bedeutet; insbesondere worin $R_1$ Methyl, Allyl oder Propargyl bedeutet; vorzugsweise ist $R_1$ Methyl.

Bevorzugt sind ebenfalls die 3-Aryluracil-Derivate der Formel I, worin Q einen Rest der Formel 1 bedeutet, wobei $R_5$ die unter der Formel I angegebene Bedeutung hat; insbesondere worin $R_5$ Wasserstoff, $C_1$-$C_5$-Alkyl oder $C_3$-$C_6$-Cycloalkyl bedeutet.

Ferner sind bevorzugt 3-Aryluracil-Derivate der Formel I, worin Q einen Rest der Formel 2 bedeutet, wobei $R_5$ und $R_7$ die unter der Formel I angegebene Bedeutung besitzen; insbesondere worin $R_6$ Wasserstoff oder $C_1$-$C_5$-Alkyl; und $R_7$ Wasserstoff, $C_1$-$C_5$-Alkyl, $C_3$-$C_5$-Alkenyl, $C_3$-$C_5$-Alkinyl oder Benzyl bedeuten.

Ferner sind bevorzugt 3-Aryluracil-Derivate der Formel I, worin Q einen Rest der Formel 3 bedeutet, wobei $R_5$, $R_5$ und $R_9$ die unter der Formel I angegebene Bedeutung besitzen; insbesondere worin $R_5$ Wasserstoff oder $C_1$-$C_5$-Alkyl; und $R_8$ und $R_9$ je Methyl bedeuten.

Ferner sind bevorzugt 3-Aryluracil-Derivate der Formel I, worin Q einen Rest der Formel 4 bedeutet, wobei $R_5$, $R_{10}$ und X die unter der Formel I angegebene Bedeutung besitzen; insbesondere worin $R_6$ Wasserstoff, $C_1$-$C_5$-Alkyl oder $C_3$-$C_6$-Cycloalkyl; und $R_{10}$ $C_1$-$C_5$-Alkyl, $C_3$-$C_5$-Alkenyl, $C_3$-$C_5$-Alkinyl oder Hydroxy-$C_2$-$C_4$-alkyl bedeuten.

Ferner sind bevorzugt 3-Aryluracil-Derivate der Formel I, worin Q einen Rest der Formel 5 bedeutet, wobei $R_5$, $R_{11}$, $R_{12}$, t und X die unter der Formel I angegebene Bedeutung besitzen; insbesondere worin $R_5$ Wasserstoff, $C_1$-$C_5$-Alkyl oder $C_3$-$C_6$-Cycloalkyl ist; $R_{11}$ und $R_{12}$, unabhängig voneinander, Wasserstoff oder Methyl sind; und t die Zahl 2 oder 3 bedeutet; vorzugsweise sind $R_{11}$ und $R_{12}$ Wasserstoff.

Besonders bevorzugt sind 3-Aryluracil-Derivate der Formel Ia, worin $R_1$ Wasserstoff, Methyl, Allyl oder Propargyl; $R_2$ Fluor, Chlor, Brom oder Cyano; $R_3$ Wasserstoff oder Fluor, $R_4$ Wasserstoff, Fluor oder Methyl; $R_5$ Methyl, Trifluormethyl oder Pentafluorethyl ist; oder $R_4$ und $R_5$ zusammen $-(CH_2)_n-$ sind; und n die Zahl 3 oder 4 bedeutet; und worin insbesondere $R_1$ Wasserstoff oder Methyl; $R_2$ Fluor, Chlor, Brom oder Cyano; $R_4$ Fluor oder Wasserstoff ist; oder $R_4$ und $R_5$ zusammen $-(CH_2)_n-$ sind, wobei n die Zahl 3 bedeutet.

Ferner sind besonders bevorzugt 3-Aryluracil-Derivate der Formel Ib, worin $R_2$ Fluor, Chlor, Brom oder Cyano; $R_3$ Wasserstoff oder Fluor, $R_4$ Wasserstoff, Fluor oder Methyl; $R_5$ Methyl, Trifluormethyl oder Pentafluorethyl ist; oder $R_4$ und $R_5$ zusammen $-(CH_2)_n-$ sind; n die Zahl 3 oder 4; und $R_{13}$ Methyl, Ethyl, Allyl oder Propargyl bedeutet; und worin insbesondere $R_2$ Fluor, Chlor, Brom oder Cyano; $R_4$ Wasserstoff oder Fluor und n die Zahl 3 bedeutet.

Ganz besonders bevorzugt sind 3-Aryluracil-Derivate der Formel I, worin -W- die Gruppe

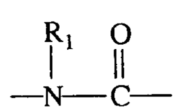

bedeutet; $R_1$ Wasserstoff oder Methyl; $R_2$ Fluor, Chlor, Brom oder Cyano; $R_3$ Wasserstoff oder Fluor, $R_4$ Was-

serstoff; $R_5$ Methyl, Trifluormethyl, Pentafluorethyl ist; oder $R_4$ und $R_5$ zusammen $-(CH_2)_n-$ sind; n die Zahl 3; und Q eine der Gruppen a) bis e) bedeutet:

a)

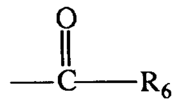

(1),

wobei $R_6$ Wasserstoff, $C_1$-$C_5$-Alkyl oder $C_3$-$C_6$-Cycloalkyl;

b)

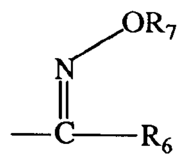

(2),

wobei $R_6$ die unter a) angegebene Bedeutung besitzt; und $R_7$ Wasserstoff, $C_1$-$C_4$-Alkyl, Allyl oder Benzyl;

c)

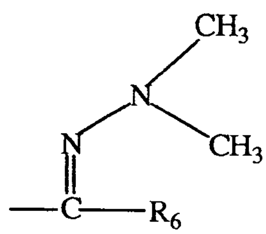

(3),

wobei $R_6$ die unter a) angegebene Bedeutung besitzt;

d)

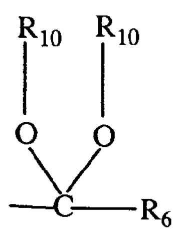

(4),

wobei $R_6$ die unter a) angegebene Bedeutung besitzt; und $R_{10}$ $C_1$-$C_5$-Alkyl oder Hydroxy-ethyl;

e)

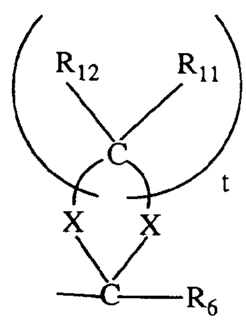

(5),

wobei $R_6$ die unter a) angegebene Bedeutung besitzt; $R_{11}$ und $R_{12}$ je Wasserstoff sind; t die Zahl 2 oder

3; oder $R_{11}$ Wasserstoff und $R_{12}$ Methyl; n die Zahl 2; und X Sauerstoff oder Schwefel, bedeutet.

Als bevorzugte Einzelverbindungen der Formel I sind zu nennen:

2-Chlor-5-[3,6-dihydro-2,6-dioxo-3-methyl-4-trifluormethyl-1-(2H)-pyrimidinyl]-benzaldehyd,
2-Chlor-5-[3,6-dihydro-2,6-dioxo-3-methyl-4-trifluormethyl-1(2H)-pyrimidinyl]-acetophenon-oxim-methylether,
2-Chlor-5-[3,6-dihydro-2,6-dioxo-3-methyl-4-trifluormethyl-1(2H)-pyrimidinyl]-acetophenonethylenketal,
2-Chlor-5-[3,6-dihydro-2,6-dioxo-3-methyl-4-trifluormethyl-1(2H)-pyrimidinyl]-benzophenonethylenketal,
3-[4-Chlor-3-(3-methylbutyryl)phenyl]-1-methyl-6-trifluormethyl-2,4(1H,3H)-pyrimidindion,
2-Chlor-5-[3,6-dihydro-2,6-dioxo-3-methyl-4-trifluormethyl-1(2H)-pyrimidinyl]-acetophenon,
2-Chlor-5-[3,6-dihydro-2,6-dioxo-3-methyl-4-trifluormethyl-1(2H)-pyrimidinyl]-phenyl-1-(i)propyl-keton,
2-Chlor-5-[3,6-dihydro-2,6-dioxo-3-methyl-4-trifluormethyl-1(2H)-pyrimidinyl]-phenyl-1-(n)butyl-keton,
2-Chlor-5-[3,6-dihydro-2,6-dioxo-3-methyl-4-trifluormethyl-1(2H)-pyrimidinyl]-phenyl-1-(3-methylbutyl)-keton,
2-Chlor-5-[3,6-dihydro-2,6-dioxo-3-methyl-4-trifluormethyl-1(2H)-pyrimidinyl]-phenyl-1-cyclopropyl-keton,
2-Chlor-5-[3,6-dihydro-2,6-dioxo-3-methyl-4-trifluormethyl-1-(2H)-pyrimidinyl]-phenyl-1-cyclopentyl-keton,
2-Chlor-5-[3,6-dihydro-2,6-dioxo-3-methyl-4-trifluormethyl-1(2H)-pyrimidinyl]-phenyl-1-cyclohexyl-keton,
2-Chlor-4-fluor-5-[3,6-dihydro-2,6-dioxo-3-methyl-4-trifluormethyl-1(2H)-pyrimidinyl]-acetophenon,
2-Chlor-4-fluor-5-[3,6-dihydro-2,6-dioxo-3,4-dimethyl-1(2H)-pyrimidinyl]-acetophenon,
2,4-Difluor-5-[3,6-dihydro-2,6-dioxo-3-methyl-4-trifluormethyl-1(2H)-pyrimidinyl]-acetophenon,
2-Chlor-5-[3,6-dihydro-2,6-dioxo-3-methyl-4-pentafluorethyl-1(2H)-pyrimidinyl]-phenyl-1-(i)propyl-keton,
2-Chlor-5-[3,6-dihydro-2,6-dioxo-3-methyl-4-trifluormethyl-1(2H)-pyrimidinyl]-propiophenon,
2-Chlor-5-[3,6-dihydro-2,6-dioxo-3-methyl-4-pentafluorethyl-1(2H)-pyrimidinyl]-propiophenon,
2-Chlor-5-[3,6-dihydro-2,6-dioxo-3-methyl-4-trifluormethyl-1(2H)-pyrimidinyl]-phenyl-1-(n)propyl-keton,
2-Brom-5-[3,6-dihydro-2,6-dioxo-3-methyl-4-trifluormethyl-1(2H)-pyrimidinyl]-acetophenon,
2-Fluor-5-[3,6-dihydro-2,6-dioxo-3-methyl-4-trifluormethyl-1(2H)-pyrimidinyl]-acetophenon,
2-Chlor-4-fluor-5-[3,6-dihydro-2,6-dioxo-3,4-dimethyl-1(2H)-pyrimidinyl]-propiophenon,
2-Chlor-4-fluor-5-[1,2,4,5,6,7-hexahydro-1-methyl-2,4-dioxo-3H-cyclopenta[d]-pyrimidin-3-yl]-acetophenon,
2-Chlor-5-[3,6-dihydro-2,6-dioxo-3-methyl-4-trifluormethyl-1(2H)-pyrimidinyl]-4-fluorbenzaldehyd,
2-Chlor-5-[3,6-dihydro-2,6-dioxo-3-methyl-4-trifluormethyl-1(2H)-pyrimidinyl]-acetophenonoxim,
2-Chlor-5-[3,6-dihydro-2,6-dioxo-3-methyl-4-trifluormethyl-1(2H)-pyrimidinyl]-acetophenonoxim-ethylether,
2-Chlor-5-[3,6-dihydro-2,6-dioxo-3-methyl-4-trifluormethyl-1(2H)-pyrimidinyl]-acetophenonoxim-isopropylether,
2-Chlor-5-[3,6-dihydro-2,6-dioxo-3-methyl-4-trifluormethyl-1(2H)-pyrimidinyl]-acetophenonoxim-allylether,
2-Chlor-5-[3,6-dihydro-2,6-dioxo-3-methyl-4-trifluormethyl-1(2H)-pyrimidinyl]-acetophenonoxim-benzylether,
2-Chlor-5-[3,6-dihydro-2,6-dioxo-3-methyl-4-trifluormethyl-1(2H)-pyrimidinyl]-benzaloxim
2-Chlor-5-[3,6-dihydro-2,6-dioxo-3-methyl-4-trifluormethyl-1(2H)-pyrimidinyl]-acetophenonoxim-(i)butylether,
2-Chlor-5-[3,6-dihydro-2,6-dioxo-3-methyl-4-trifluormethyl-1(2H)-pyrimidinyl]-acetophenon-N,N-dimethylhydrazon,
2-Chlor-5-[3,6-dihydro-2,6-dioxo-3-methyl-4-trifluormethyl-1(2H)-pyrimidinyl]-benzaldehyd-N,N-dimethylhydrazon,
2-Chlor-5-[3,6-dihydro-2,6-dioxo-3-methyl-4-trifluormethyl-1(2H)-pyrimidinyl]-acetophenon-methylethylenacetal,
2-Chlor-5-[3,6-dihydro-2,6-dioxo-3-methyl-4-trifluormethyl-1(2H)-pyrimidinyl]-acetophenon-propylenacetal,
2-Chlor-5-[3,6-dihydro-2,6-dioxo-3-methyl-4-trifluormethyl-1(2H)-pyrimidinyl]-benzaldehyd-dimethoxyacetal,
2-Chlor-5-[3,6-dihydro-2,6-dioxo-3-methyl-4-trifluormethyl-1(2H)-pyrimidinyl]-benzaldehyd-ethylenacetal,
2-Chlor-5-[3,6-dihydro-2,6-dioxo-3-methyl-4-trifluormethyl-1(2H)-pyrimidinyl]-acetophenon-propylendithioacetal,
2-Chlor-5-[3,6-dihydro-2,6-dioxo-3-methyl-4-trifluormethyl-1(2H)-pyrimidinyl]-acetophenon-ethylendithioacetal,
2-Chlor-5-[3,6-dihydro-2,6-dioxo-3-methyl-4-trifluormethyl-1(2H)-pyrimidinyl]-acetophenon-methylethylendithioacetal,
2-Chlor-5-[3,6-dihydro-2,6-dioxo-3-methyl-4-trifluormethyl-1(2H)-pyrimidinyl]-benzaldehyd-isopropyloxim.

Das erfindungsgemässe Verfahren zur Herstellung der Verbindungen der Formel I und ihrer Salze erfolgt in Analogie zu bekannten Verfahren und ist dadurch gekennzeichnet, dass man

a) zwecks Herstellung derjenigen 3-Aryluracil-Derivate der Formel I, in denen $R_1$ Wasserstoff bedeutet, sowie gegebenenfalls von Metallsalzen dieser Verbindungen, eine Verbindung der Formel IIa oder IIb

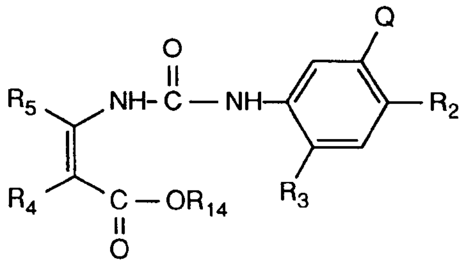

(IIa) oder

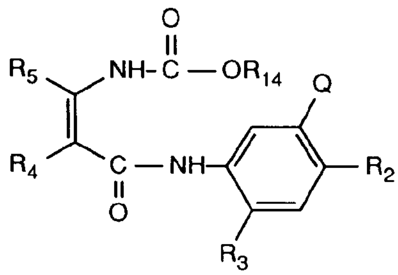

(IIb),

worin $R_2$, $R_3$, $R_4$, $R_5$ und Q die unter Formel I angegebenen Bedeutungen besitzen, und $R_{14}$ $C_1$-$C_6$-Alkyl, bevorzugt $C_1$-$C_4$-Alkyl, bedeutet, einer Cyclisierung durch Behandlung mit einer Base unterwirft und gegebenenfalls ein erhaltenes Salz des Uracil-Derivates der Formel IIc

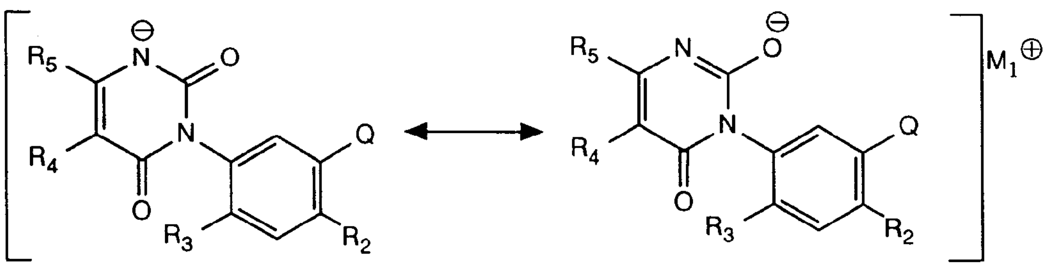

(IIc),

worin $R_2$, $R_3$, $R_4$, $R_5$ und Q die angegebene Bedeutung besitzen und $M_1^{\oplus}$ ein Kation, z.B. Alkalimetallion ist, durch Behandlung mit einer Säure in die Verbindungen der Formel I mit $R_1$ Wasserstoff (protonierte Form) überführt;

b) zwecks Herstellung derjenigen 3-Aryluracil-Derivate der Formel I, in denen $R_1$ $C_1$-$C_4$-Alkyl, $C_3$- oder $C_4$-Alkenyl, $C_3$- oder $C_4$-Alkinyl oder $C_1$-$C_4$-Haloalkyl bedeutet, ein Uracil-Derivat der Formel Ic

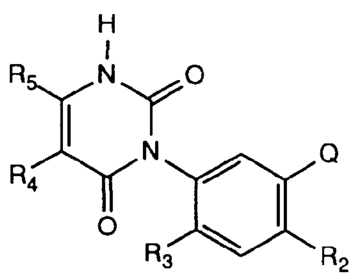

(Ic),

worin $R_2$, $R_3$, $R_4$, $R_5$ und Q die unter Formel I angegebenen Bedeutungen haben, in Gegenwart eines eine entsprechende $C_1$-$C_4$-Alkyl-, $C_3$- oder $C_4$-Alkenyl-, $C_3$- oder $C_4$-Alkinyl- oder $C_1$-$C_4$-Haloalkyl-Gruppe enthaltenden Alkylierungsmittels alkyliert; als weiteres Produkt kann auch das O-Alkylierungsprodukt der Formel Id

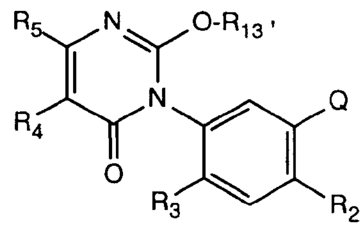

(Id)

erhalten werden, wobei $R_{13'}$ die für $R_1$ angegebene Bedeutung hat, mit Ausnahme von Wasserstoff;

c) zwecks Herstellung derjenigen 3-Aryluracil-Derivate der Formel I, in denen $R_1$ $C_1$-$C_4$-Alkyl, $C_1$-$C_4$-Haloalkyl, $C_3$- oder $C_4$-Alkenyl oder $C_3$- oder $C_4$-Alkinyl und Q eine Gruppe

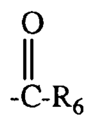 (1) ist,

worin $R_6$ $C_1$-$C_8$-Alkyl, $C_3$-$C_7$-Cycloalkyl oder $C_3$-$C_7$-Cycloalkyl-$C_1$-$C_4$-alkyl bedeutet, ein Carbonsäure-Derivat der Formel III

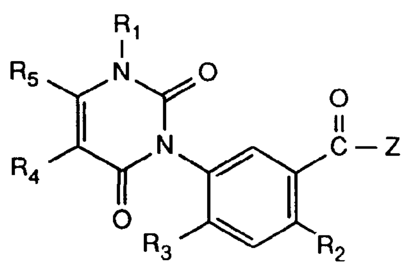

(III),

worin $R_1$, $R_2$, $R_3$, $R_4$ und $R_5$ die unter Formel I angegebenen Bedeutungen haben, und Z Halogen, vorzugsweise Chlor, oder eine Abgangsgruppe, vorzugsweise N,O-Dimethylhydroxyl-amino bedeutet, mit einer metallorganischen Verbindung der Formel

$R_{6'}$-$M_2$ (VIIa) oder $R_{6'}$-$M_3$-G (VIIb),

worin $R_{6'}$ $C_1$-$C_8$-Alkyl, $C_3$-$C_7$-Cycloalkyl oder $C_3$-$C_7$-Cycloalkyl-$C_1$-$C_4$-alkyl; $M_2$ ein Alkalimetallion, bevorzugt Lithium; $M_3$ ein Erdalkalimetallion oder ein Metall der ersten oder zweiten Nebengruppe des Periodensystems, bevorzugt Magnesium; und G Halogen, bevorzugt Chlor oder Brom bedeuten, umsetzt;

d) zwecks Herstellung derjenigen 3-Aryluracil-Derivate der Formel I, in denen $R_1$ $C_1$-$C_4$-Alkyl, $C_1$-$C_4$-Haloalkyl, $C_3$- oder $C_4$-Alkenyl oder $C_3$- oder $C_4$-Alkinyl und Q eine Gruppe

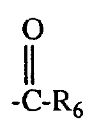 (1) ist,

worin $R_6$ Wasserstoff, $C_1$-$C_8$-Alkyl, $C_3$-$C_7$-Cycloalkyl oder $C_3$-$C_7$-Cycloalkyl-$C_1$-$C_4$-alkyl bedeutet, ein Carbinol der Formel IV

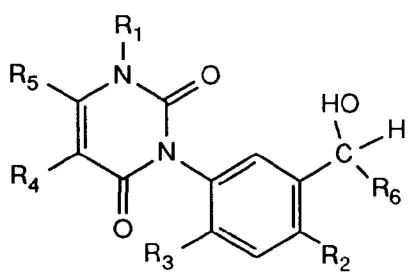

(IV),

worin $R_1$, $R_2$, $R_3$, $R_4$ und $R_5$ die unter Formel I angegebenen Bedeutungen haben, oxidiert;
e) zwecks Herstellung derjenigen 3-Aryluracil-Derivate der Formel I, in denen Q eine Gruppe

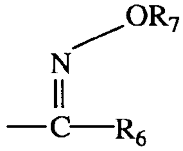 (2) ist,

worin $R_6$ und $R_7$ die unter Formel I angegebenen Bedeutungen haben, einen Aldehyd oder ein Keton der Formel I, in denen Q eine Gruppe

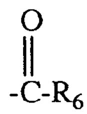 (1) ist,

mit einem Hydroxylamin der Formel $H_2N\text{-}O\text{-}R_7$ (VIII), reagieren lässt;
f) zwecks Herstellung derjenigen 3-Aryluracil-Derivate der Formel I, in denen Q eine Gruppe

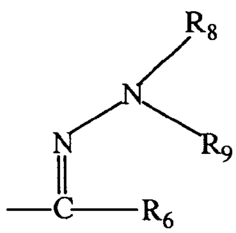 (3) ist,

worin $R_6$, $R_8$ und $R_9$ die unter Formel I angegebenen Bedeutungen haben, einen Aldehyd oder ein Keton der Formel I, in denen Q eine Gruppe

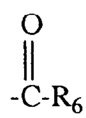 (1) ist,

mit einem Hydrazin der Formel

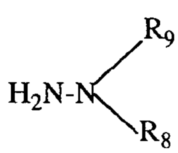 (IX),

reagieren lässt;
g) zwecks Herstellung derjenigen 3-Aryluracil-Derivate der Formel I, in denen Q eine Gruppe

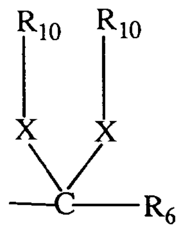 (4) oder 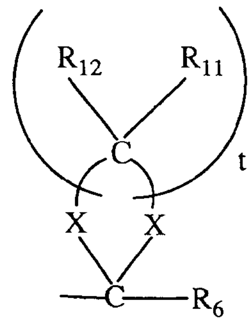 (5) ist,

worin $R_6$, $R_{10}$, $R_{11}$, $R_{12}$ und X die unter Formel I angegebenen Bedeutungen haben, einen Aldehyd oder ein Keton der Formel I, in denen Q eine Gruppe

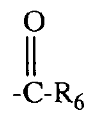 (1) ist,

mit einem Alkohol oder Thiol der Formel $R_{10}$-X-H (Xa) oder

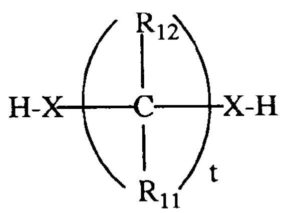 (Xb),

umsetzt;

h) zwecks Herstellung derjenigen 3-Aryluracil-Derivate der Formel Ib (Imino-Ether-Form)

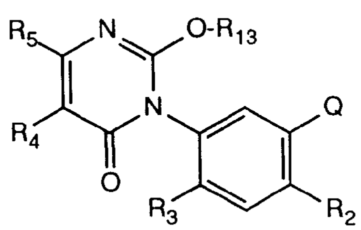

(Ib),

ein Uracil der Formel Ic

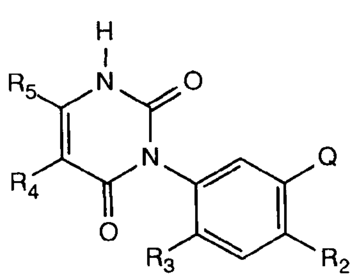

(Ic),

worin $R_2$, $R_3$, $R_4$, $R_5$ und Q die unter Formel I angegebenen Bedeutungen haben, zuerst einer Halogenierung unterwirft und das Uracil der Formel V erhält,

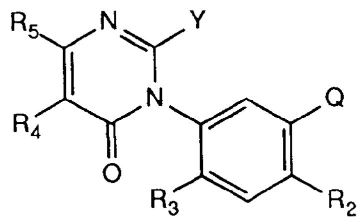

(V),

worin Y Halogen, bevorzugt Chlor, bedeutet, und dieses anschliessend basenkatalysiert mit einem Alkohol der Formel $R_{13}$-OH (XIa) oder mit dem entsprechenden Alkoholat der Formel $R_{13}$-$O^{\ominus}M_4^{\oplus}$ (XIb), worin $R_{13}$ die unter Formel I angegebene Bedeutung hat, und $M_4^{\oplus}$ ein Alkalimetall- oder Eralkalimetallion ist, umsetzt;

i) zwecks Herstellung derjenigen 3-Aryluracil-Derivate der Formel I, in denen $R_2$ Cyano ist, eine Verbindung der Formel I, worin $R_2$ Halogen bedeutet, mit einem Metallcyanid der Formel XII

$M_5(CN)_s$ (XII),

worin $M_5$ ein Alkalimetallion oder ein Metall der ersten oder zweiten Nebengruppe des Periodensystems; und s die Zahl 1 oder 2, bedeuten, umsetzt;

j) zwecks Herstellung derjenigen 3-Aryluracil-Derivate der Formel I, in denen $R_1$ $C_1$-$C_4$-Alkyl, $C_1$-$C_4$-Haloalkyl, $C_3$- oder $C_4$-Alkenyl oder $C_3$- oder $C_4$-Alkinyl und Q eine Gruppe

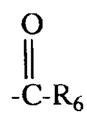

(1),

worin $R_6$ $C_1$-$C_8$-Alkyl oder $C_3$-$C_7$-Cycloalkyl-$C_1$-$C_4$-alkyl bedeutet, ein reaktives Säurederivat der Formel III

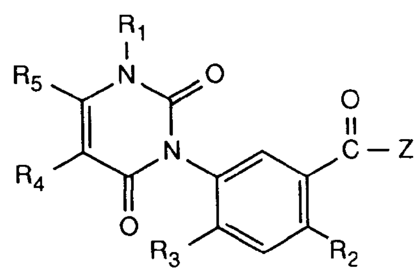

(III),

worin Z ein Halogen, vorzugsweise Chlor, bedeutet, mit einer metallorganischen Verbindung der Formel XIII

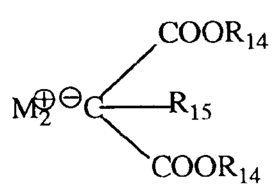

(XIII),

worin $R_{14}$ $C_1$-$C_6$-Alkyl, vorzugsweise $C_1$-$C_4$-Alkyl, $R_{15}$ Wasserstoff, $C_1$-$C_7$-Alkyl, $C_3$-$C_7$-Cycloalkyl, $C_3$-$C_7$-Cycloalkyl-$C_1$-$C_3$-alkyl, und $M_2^{\oplus}$ ein Alkalimetallion, vorzugsweise Lithium oder Natrium, bedeuten, umsetzt, und man die erhaltenen Zwischenprodukte der Formel VI

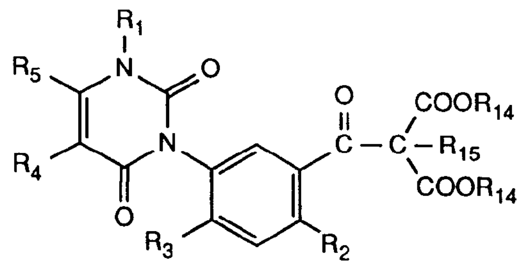

(VI),

worin $R_2$, $R_3$, $R_4$ und $R_5$ die unter Formel I angegebenen Bedeutungen haben, in einer säurekatalysierten Folgereaktion zu den 3-Aryluracilen der Formel I hydrolysiert und decarboxyliert;

k) zwecks Herstellung derjenigen 3-Aryluracil-Derivate der Formel I, in denen Q eine Gruppe

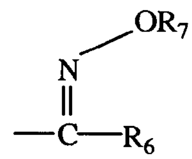

(2) ist,

worin $R_6$ die unter Formel I angegebene Bedeutung hat, und $R_7$ $C_1$-$C_8$-Alkyl, $C_2$-$C_8$-Alkenyl, $C_2$-$C_8$-Alkinyl, $C_3$-$C_7$-Cycloalkyl, Benzyl oder mit Halogen, $C_1$-$C_4$-Alkyl oder $C_1$-$C_4$-Alkoxy substituiertes Benzyl bedeutet, ein Oxim der Formel I, worin Q eine Gruppe

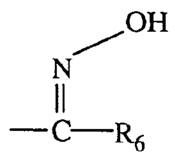

ist,

in ein reaktives Alkalimetallsalz überführt, und dieses anschliessend mit einer Verbindung der Formel XIV

$R_7$-Z' (XIV),

worin Z' eine Abgangsgruppe, bevorzugt Halogen, bedeutet, umsetzt; und eine gemäss diesen aufgeführten Verfahrensvarianten erhaltene Verbindung der Formel I, vorausgesetzt $R_1$ ist Wasserstoff, gegebenenfalls in ein Salz überführt.

Die Cyclisierung nach Verfahrensvariante a) kann zweckmässigerweise durchgeführt werden, indem man die Verbindung der Formel IIa oder IIb in einem inerten protischen organischen Lösungsmittel, wie einem Alkohol, beispielsweise Methanol, Ethanol oder Isopropanol; einem inerten aprotischen organischen Lösungsmittel wie einem aliphatischen oder cyclischen Ether, beispielsweise 1,2-Dimethoxyethan, Tetrahydrofuran oder Dioxan oder einem Aromaten, beispielsweise Benzol oder Toluol; einem inerten aprotischen, polaren organischen Lösungsmittel, beispielsweise Dimethylformamid oder Dimethylsulfoxid, wobei solche Lösungsmittel gegebenenfalls im Zweiphasen-Gemisch mit einem Kohlenwasserstoff, beispielsweise n-Hexan oder Toluol, verwendet werden können; oder Wasser mit einer Base bei Temperaturen zwischen -78°C und der Rückflusstemperatur des Reaktionsgemisches, behandelt. Als Basen kommen vorzugsweise Natriumalkoholate, Alkalimetallhydroxide, insbesondere Natriumhydroxid oder Kaliumhydroxid, Alkalimetallcarbonate, insbesondere Natriumcarbonat oder Kaliumcarbonat oder Natriumhydrid in Betracht. Bei der Verwendung von Natriumhydrid als Base ist das Lösungsmittel vorzugsweise ein aliphatischer oder cyclischer Ether, Dimethylformamid oder Dimethylsulfoxid, wobei jedes dieser Lösungsmittel im Gemisch mit Toluol verwendet werden kann.

Nach Beendigung der Cyclisierung liegt das Produkt im Falle der Verwendung einer der obenerwähnten oder verwandten Basen in Form des entsprechenden Alkalimetallsalzes vor. Dieses kann in an sich bekannter Weise isoliert und gereinigt werden, oder man kann das Gemisch ansäuern, um die jeweilige Verbindung der Formel I zu isolieren. Zu diesem Zwecke verwendet man vorzugsweise eine Mineralsäure, wie Salzsäure, oder eine starke organische Säure, wie Essigsäure oder p-Toluolsulfonsäure.

Bei der Verfahrensvariante b) steht der Ausdruck "alkyliert" für die Einführung einer $C_1$-$C_4$-Alkyl-, $C_3$- oder $C_4$-Alkenyl-, $C_3$- oder $C_4$-Alkinyl- oder $C_1$-$C_4$-Haloalkyl-Gruppe am unsubstituierten Stickstoffatom des Uracilkerns. Als Alkylierungsmittel wird zweckmässigerweise ein $C_1$-$C_4$-Alkyl-, $C_3$- oder $C_4$-Alkenyl- oder $C_3$- oder $C_4$-Alkinylhalogenid, insbesondere das diesbezügliche Chlorid oder Bromid, oder Sulfonat bzw. ein mehrfach

halogeniertes $C_1$-$C_4$-Alkan, wie beispielsweise Chlordifluormethan, oder ein mono- oder mehrfach halogeniertes Alken, wie beispielsweise Tetrafluorethen verwendet.

Die Alkylierung wird zweckmässigerweise in Gegenwart eines inerten, protischen oiganischen Lösungsmittels, wie eines niederen Alkanols, beispielsweise Ethanol, gegebenenfalls im Gemisch mit Wasser, eines inerten, aprotischen organischen Lösungsmittels, wie eines aliphatischen oder cyclischen Ethers, beispielsweise 1,2-Dimethoxyethan, Tetrahydrofuran oder Dioxan; eines Ketons, beispielsweise Aceton oder Butan-2-on; oder eines inerten, aprotischen, polaren organischen Lösungmittels, beispielsweise Dimethylformamid, Dimethylsulfoxid oder Acetonitril, sowie in Gegenwart einer Base, wie Natriumhydrid, eines Alkalimetallhydroxids, insbesondere Natrium- oder Kaliumhydroxid, eines Alkalimetallalkoholats, insbesondere Natriumalkoholat, oder eines Alkalimetallcarbonats oder -hydrogencarbonats, insbesondere Natriumcarbonat, Kaliumcarbonat, Natriumhydrogencarbonat oder Kaliumhydrogencarbonat, bei Temperaturen zwischen 0°C und der Rückflusstemperatur des Reaktionsgemisches, vorzugsweise bei Temperaturen zwischen 50°C und 100°C, durchgeführt. In einer bevorzugten Ausführungsform wird das Uracilderivat der Formel Ic zunächst mit der Base wie Natriumhydrid, -ethanolat oder -carbonat, im Lösungsmittel behandelt und nach einer kurzen Reaktionszeit mit dem Halogenid im gleichen Lösungsmittel versetzt. In einer weiteren Ausführungsform wird das Uracilderivat Ic zusammen mit einem Dialkylsulfat in Gegenwart eines Alkalimetallhydrogencarbonats, insbesondere Natrium- oder Kaliumhydrogencarbonat, im Lösungsmittel, beispielsweise Aceton bei Rückflusstemperatur zur Reaktion gebracht. Die Reaktion ist in der Regel je nach verwendetem Lösungsmittel innert relativ kurzer Zeit oder nach wenigen Stunden beendet.

Die Umsetzung nach Verfahrensvariante c) erfolgt zweckmässigerweise unter Verwendung eines Carbonsäurehalogenids der Formel III, worin Z bevorzugt Chlor ist, und einer metallorganischen Verbindung der Formel VIIa oder VIIb, in einem aprotischen organischen Lösungsmittel, vorzugsweise aliphatische oder cyclische Ether, beispielsweise Diethylether, Dimethoxyethan oder Tetrahydrofuran. Die Reaktionstemperaturen sind im allgemeinen zwischen -80°C und 0°C, vorzugsweise bei Temperaturen um -70°C. Für die Reaktion der reaktiven Carbonsäure-Derivate der Formel III, worin Z eine N,O-Dimethylhydroxylamino-Gruppe ist, wird eine metallorganische Verbindung der Formel VIIa oder VIIb, vorzugsweise aber die Grignard-Verbindung VIIb in Tetrahydrofuran bei 0°C eingesetzt.

Bei der Oxidation gemäss Verfahrensvariante d) wird zweckmässigerweise ein Benzylalkohol der Formel IV mit einem geeigneten Oxidationsmittel, wie beispielsweise Pyridiniumdichromat, Pyridinium-chlorchromat, Kaliumpermanganat, Rutheniumtetroxyd oder Mangandioxyd in einem inerten, organischen Lösungsmittel, wie beispielsweise in chlorierten Kohlenwasserstoffen, wie Tetrachlorkohlenstoff oder Dichlormethan oder in einem aliphatischen Nitril wie Acetonitril, oxydiert. Als weitere Lösungsmittel können auch Essigsäure oder Mineralsäuren, wie beispielsweise Schwefelsäure eingesetzt werden Die angewendeten Reaktionstemperaturen liegen zwischen -20°C und +50°C, vorzugsweise zwischen 0°C und +30°C. In einer bevorzugten Ausführungsform wird das Uracilderivat der Formel IV mit Pyridinium-chlorchromat in Dichlormethan oxydiert.

Bei der Oximierung eines 3-Aryluracil-Derivats der Formel I, worin Q eine Aldehyd- oder Keton-Gruppe darstellt, mit gegebenenfalls O-substituiertem Hydroxylamin der Formel VIII gemäss Verfahrensvariante e), wird zweckmässigerweise in einem organischen Lösungsmittel wie beispielsweise in einem niederen Alkohol, vorzugsweise Methanol, Ethanol oder Isopropanol, gegebenenfalls in einem Gemisch mit Wasser bei Temperaturen zwischen 0°C und der Siedetemperatur des Reaktionsgemisches verfahren. Das Reagens Hydroxylamin der Formel VIII kann in Form der freien Base oder in Form eines Säureadditionssalzes wie beispielsweise als Hydrochlorid- oder als Hydrogensulfat-Salz eingesetzt werden. Die Oximierung verläuft spontan oder basenkatalysiert durch Zugabe einer organischen Base wie beispielsweise Pyridin, Triethylamin oder 4-Dimethylaminopyridin oder durch Zugabe eines Alkalimetall- oder Erdalkalimetallcarbonats oder - hydrogencarbonats wie beispielsweise Natrium - oder Kalium-Carbonat.

Bei der Hydrazonbildung eines 3-Aryluracil-Derivats der Formel I, worin Q eine Aldehyd- oder Keton-Gruppe darstellt, mit gegebenenfalls substituiertem Hydrazin der Formel IX gemäss Verfahrensvariante f) wird zweckmässigerweise in einem organischen Lösungsmittel wie in Verfahrensvariante e) für die Oximierung angegeben, verfahren. Das Hydrazin der Formel IX kann in Form der freien Base oder als Säureadditionssalz wie beispielsweise des Hydrochlorids oder des Hydrogensulfats eingesetzt werden. Die Hydrazonbildung verläuft spontan oder basenkatalysiert durch Zugabe einer organischen Base wie beispielsweise Pyridin, Triethylamin oder 4-Dimethylaminopyridin, oder eines Alkalimetall- oder Erdalkalimetall-carbonats oder -hydrogencarbonats, wie beispielsweise Natrium-, Kaliumcarbonat oder Kaliumhydrogencarbonat.

Die (Thio) Acetalisierung eines 3-Aryluracil-Derivats der Formel I, worin Q eine Aldehyd- oder Keton-Gruppe darstellt, mit einem Alkohol oder Thiol der Formel Xa oder Xb gemäss Verfahrensvariante g) erfolgt zweckmässigerweise in einem inerten, organischen Lösungsmittel, beispielsweise in einem aromatischen Kohlenwasserstoff, vorzugsweise Benzol, Toluol oder Xylol, oder in einem halogenierten Kohlenwasserstoff, vorzugsweise Chloroform bei Temperaturen zwischen -30°C und der Siedetemperatur des Reaktionsgemisches unter

Zusatz einer entweder katalyrischen, stöchiometrischen oder überschüssigen Menge einer Carbonsäure oder organischen Sulfonsäure, beispielsweise p-Toluolsulfonsäure oder einer Mineralsäure, beispielsweise Chlorwasserstoff- oder Schwefelsäure. Das dabei entstehende Reaktionswasser kann beispielsweise azeotrop abdestilliert oder durch Absorption in einem Alkali-Erdalkalialuminiumsilikat (Molekularsieb) aus dem Reaktionsgemisch entfernt werden.

Die Halogenierung nach Verfahrensvariante h) erfolgt zweckmässigerweise durch Einsatz eines Halogenierungsmittels, beispielsweise Thionylchlorid, Phosphorpentachlorid oder Phosphoroxychlorid bzw. Phosphorpentabromid oder Phosphorylbromid, gegebenenfalls in einem inerten, organischen Lösungsmittel, beispielsweise n-Hexan, Benzol, Toluol, Xylol, Dichlormethan, Chloroform, 1,2-Dichlorethan oder Chlorbenzol, bei Reaktionstemperaturen zwischen 0°C und der Rückflusstemperatur des Reaktionsgemisches, vorzugsweise zwischen 80°C und 120°C.

Der Reaktion kann gegebenenfalls eine organische Base, beispielsweise Triethylamin, Pyridin oder 4-Dimethylaminopyridin zugesetzt werden. Zweckmässigerweise kann beim Arbeiten mit Thionylchlorid auch eine katalytische Menge N,N-Dimethylformamid zugesetzt werden.

Das 2-Halopyrimidinon der Formel V wird in einem Ueberschuss des entsprechenden Alkohols der Formel XIa als Lösungsmittel bei Temperaturen zwischen 0°C und +50°C, vorzugsweise bei Raumtemperatur in Gegenwart einer geeigneten organischen Base, beispielsweise Pyridin, umgesetzt. Erfolgt die Reaktion des 2-Halopyrimidin-ons der Formel V mit dem entsprechenden Alkoholat der Formel XIb, bedeutet $M_4^{\oplus}$ vorzugsweise ein Alkalimetallion beispielsweise Natrium- oder Kaliumion, oder ein Erdalkalimetallion, beispielsweise Calcium- oder Magnesiumion. Bevorzugt ist das Natriumion.

Bei der Herstellung der 3-Aryluracil-Derivate der Formel I, worin $R_2$ eine Cyanogruppe ist, gemäss der Verfahrensvariante i) wird ein aromatischer Halogensubstituent ($R_2$ = Halogen) durch eine Cyanogruppe ausgetauscht mittels eines Metallcyanids der Formel XII. Letzteres ist insbesondere ein Uebergangsmetallcyanid, vorzugsweise Kupfer (I)-cyanid. Die Austauschreaktion erfolgt zweckmässigerweise in Gegenwart eines aprotischen, polaren Lösungsmittels, wie Alkylnitril, beispielsweise Aceto-, Propio- oder Butyronitril, oder in Gegenwart eines Alkylharnstoffs, beispielsweise Tetramethylharnstoff, oder eines Dialkylamids, beispielsweise Dimethylformamid, oder eines Dialkylysulfoxids, beispielsweise Dimethylsulfoxid oder in N-Methyl-2-pyrrolidon, 1,3-Dimethyl-imidazolidin-2-on, 1,3-Dimethyl-3,4,5,6-tetrahydro-2(1H)-pyrimidinon, Hexamethylphosphorsäuretriamid bei Temperaturen zwischen +80°C und +200°C, vorzugsweise zwischen +150°C bis +200°C.

Bei der Herstellung der 3-Aryluracile der Formel I gemäss Verfahrensvariante j) kann zweckmässigerweise eine Verbindung der Formel III in einem inerten, aprotischen organischen Lösungsmittel, beispielsweise einem aliphatischen oder alicyclischen Ether, vorzugsweise 1,2-Dimethoxyethan, Tetrahydrofuran oder Dioxan, oder in einem inerten, aprotischen polaren Lösungsmittel, beispielsweise Dimethylformamid, N-Methylpyrrolidon, 1,3-Dimethyl-3,4,5,6-tetrahydro-2(1H)-pyrimidinon oder Dimethylsulfoxid, wobei solche Lösungsmittel gegebenenfalls im Zweiphasengemisch mit Kohlenwasserstoffen, beispielsweise n-Hexan, Cyclohexan oder Toluol verwendet werden können, mit einer metallorganischen Verbindung der Formel XIII zur Reaktion gebracht werden. Als Basen werden Alkalimetallhydride, vorzugsweise Natrium- oder Lithiumhydrid, eingesetzt. Die Reaktionstemperatur liegt zwischen -20°C und +50°C, vorzugsweise bei Raumtemperatur. Die saure Hydrolyse und nachfolgende Decarboxylierung der β-Ketodiester-Zwischenprodukte der Formel VI erfolgt zweckmässigerweise in Gegenwart einer Mineralsäure, beispielsweise Schwefel- oder Bromwasserstoffsäure, mit oder ohne Lösungsmittel. Als mögliche Lösungsmittel kommen niedere Carbonsäuren, vorzugsweise Essigsäure, in Betracht. Die Reaktionstemperaturen liegen zwischen +80°C und + 140°C, bevorzugt zwischen +100°C und +120°C.

Die Herstellung der Oximderivate der Formel I, worin Q eine Gruppe

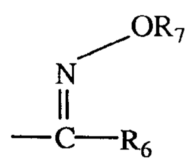 (2)

und $R_7$ $C_1$-$C_8$-Alkyl, $C_2$-$C_8$-Alkenyl, $C_2$-$C_8$-Alkinyl, $C_3$-$C_7$-Cycloalkyl oder gegebenenfalls substituiertes Phenyl oder Benzyl bedeutet, gemäss Verfahrensvariante k) erfolgt zweckmässigerweise durch Alkylierung des entsprechenden Oxims der Formel I, wobei $R_7$ Wasserstoff ist, mit einem geeigneten Alkylierungsmittel, beispielsweise mit Alkylhalogenid, vorzugsweise mit Alkylchlorid, -bromid oder -jodid, oder einem Dialkylsulfat in einem niederen Alkohol, beispielsweise Methanol oder Ethanol, oder in einem aliphatischen oder cyclischen Ether, beispielsweise Dimethoxyethan, Tetrahydrofuran oder Dioxan, bevorzugt in einem aprotischen inerten, polaren Lösungsmittel, beispielsweise Dimethylformamid, N-Methyl-pyrrolidon oder 1,3-Dimethyl-3,4,5,6-tetrahydro-2(1H)-pyrimidinon oder Dimethylsulfoxid bei Temperaturen zwischen -10°C und +70°C, vorzugsweise zwischen 0°C und +80°C. Als Base werden Alkalimetallalkoholate oder vorzugsweise Alkalimetallhydride, wie Natriumhydrid, eingesetzt.

Die Salze der so erhaltenen Verbindungen der Formel I, in denen $R_1$ Wasserstoff bedeutet, können in an sich bekannter Weise hergestellt werden, wie beispielsweise durch Auflösen der Verbindungen der Formel I in einer Lösung einer diesbezüglichen anorganischen oder organischen Base. Die Salzbildung erfolgt in der Regel innert kurzer Zeit bei Raumtemperatur. In einer Ausführungsform wird das Natriumsalz durch Auflösen des Uracilderivats I in wässriger Natriumhydroxidlösung bei Raumtemperatur hergestellt, wobei äquivalente Mengen des Uracilderivats und des Natriumhydroxids verwendet werden. Das feste Salz kann dann durch Fällen mit einem geeigneten inerten Lösungsmittel oder durch Abdampfen des Lösungsmittels isoliert werden. Eine weitere Ausführungsform besteht darin, eine wässrige Lösung eines Alkalimetallsalzes des Uracilderivats I in eine wässrige Lösung eines Salzes, das ein anderes Metallkation als ein Alkalimetallkation aufweist, einzuführen, wobei das zweite Metallsalz des Uracilderivats hergestellt wird. Diese Ausführungsform dient im allgemeinen zur Herstellung von Uracil-Metallsalzen, die in Wasser unlöslich sind.

Die erhaltenen Verbindungen der Formel I sowie deren Salze können nach an sich bekannten Methoden isoliert und gereinigt werden. Ferner ist dem Fachmann geläufig, in welcher Reihenfolge gewisse Umsetzungen unter den Verfahrensvarianten a) bis k) zweckmässig durchzuführen sind, um mögliche, unerwünschte konkurrierende Reaktionen zu vermeiden.

Sofern keine gezielte Synthese zur Isolierung reiner Isomerer durchgeführt wird, kann das Produkt als Gemisch zweier oder mehrerer Isomerer anfallen. Die Isomeren können nach an sich bekannten Methoden aufgetrennt werden. Gewünschtenfalls können beispielsweise reine optisch aktive Isomere auch durch Synthese aus entsprechenden optisch aktiven Ausgangsmaterialien hergestellt werden.

Die Ausgangsverbindungen der Formeln na und IIb sind neu und können in Analogie zu bekannten Verfahren, z.B. EP-A-0,260,621 hergestellt werden, zum Beispiel gemäss den nachfolgenden Reaktionsschemata 1 und 2 (Methoden aa), bb), cc), dd), ee) und ff)).

<u>Reaktionsschema 1</u>

Methode aa):

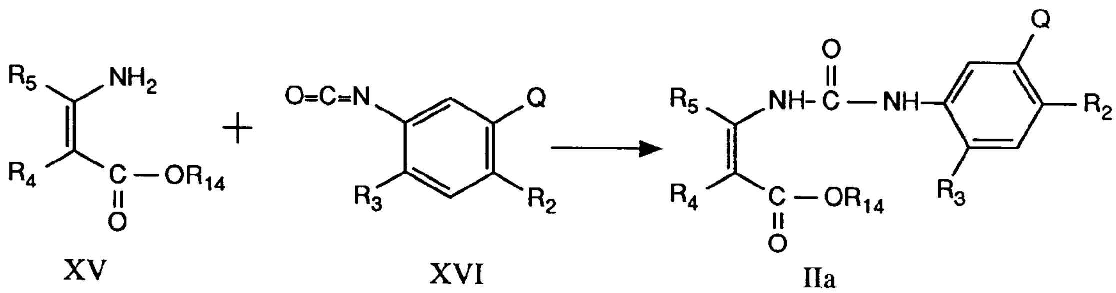

XV XVI IIa

Methode bb):

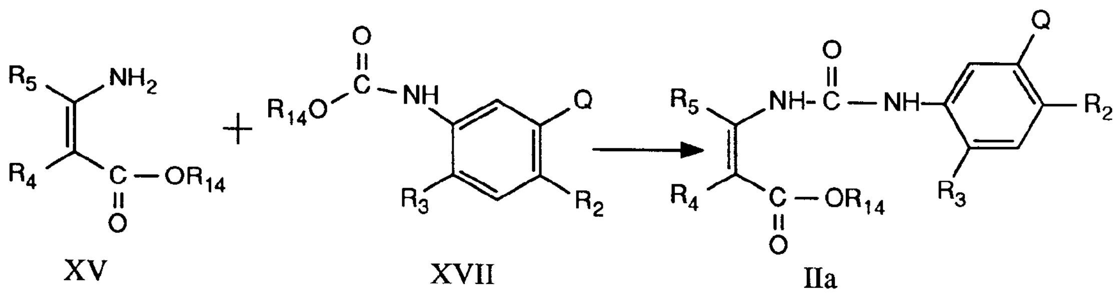

XV XVII IIa

Methode cc):

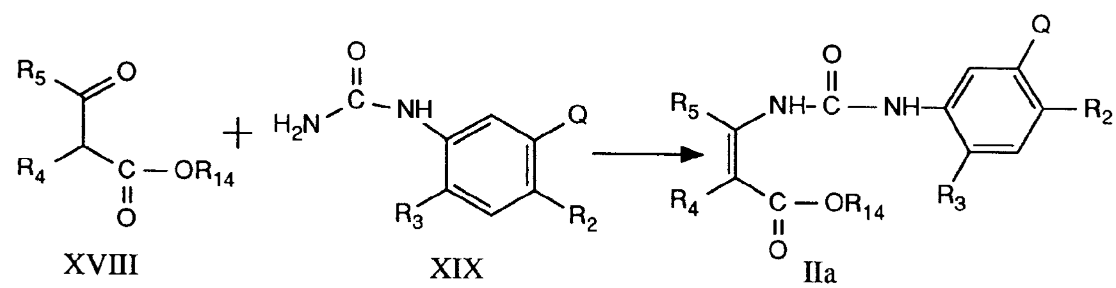

XVIII XIX IIa

Methode dd)

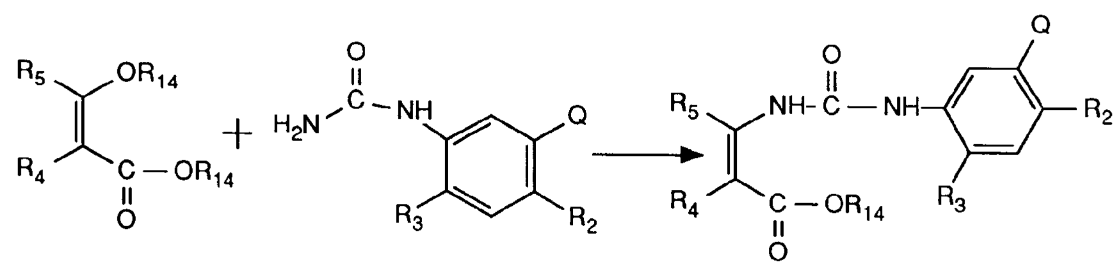

XX XIX IIa

Reaktionsschema 2

Methode ee):

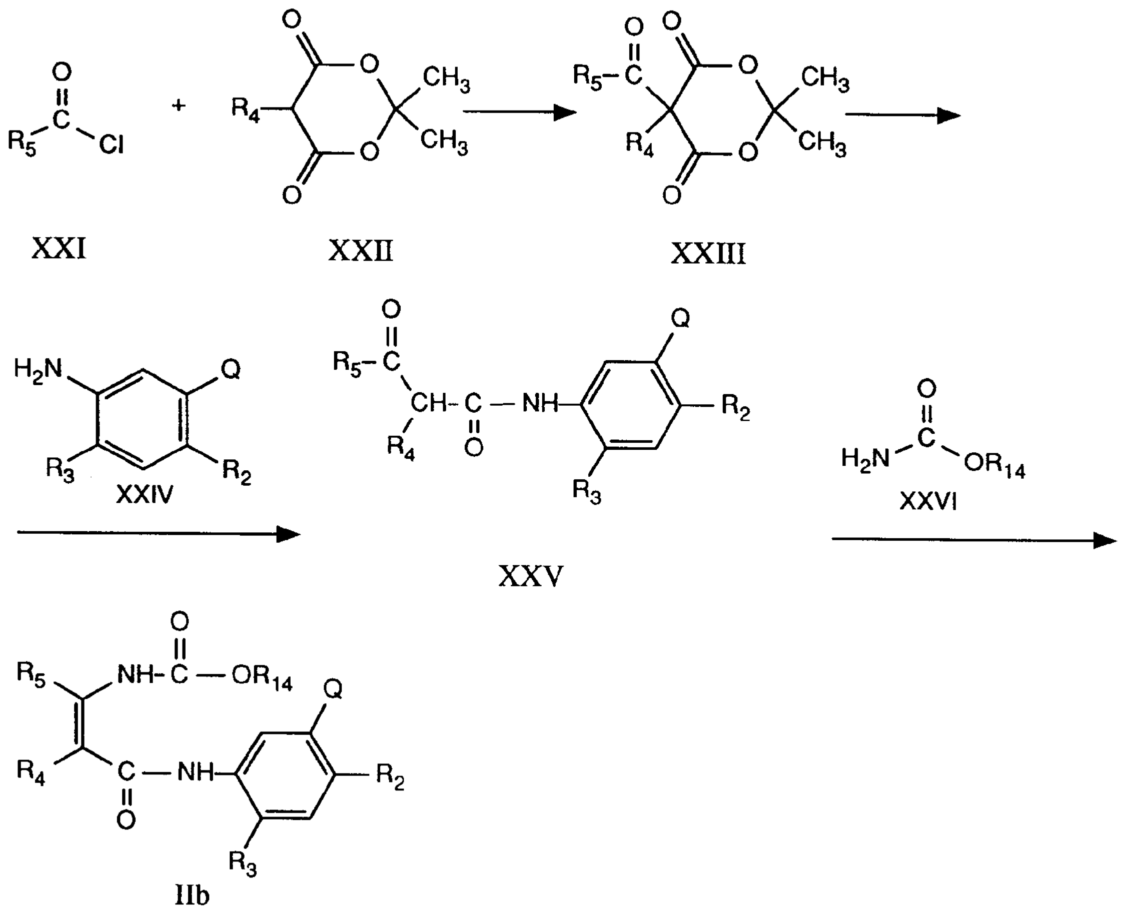

XXI XXII XXIII XXIV XXV XXVI IIb

Methode ff):

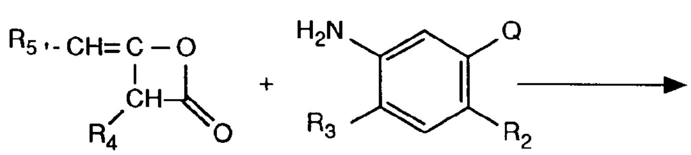

XXVII XXIV

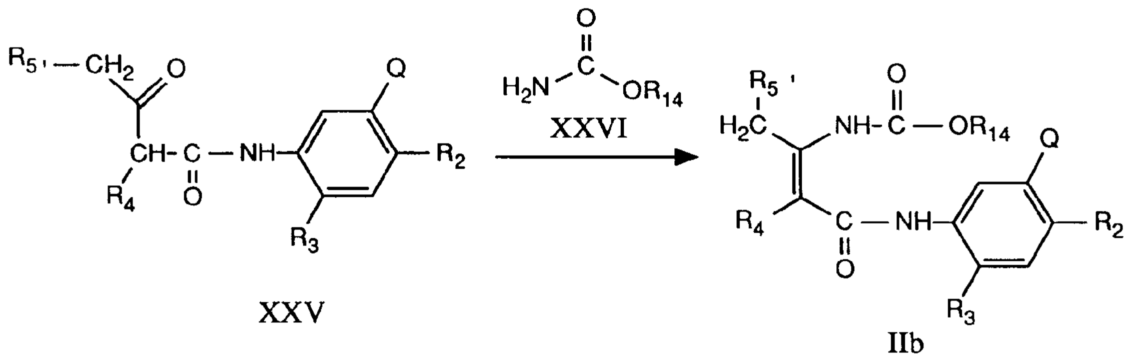

XXV XXVI IIb

Die Umsetzung nach der Methode aa) in Reaktionsschema 1 erfolgt zweckmässigerweise in Gegenwart eines wasserfreien, aprotischen, organischen Lösungsmittels, beispielsweise eines aliphatischen oder cyclischen Ethers, vorzugsweise Diethylether, 1,2-Dimethoxyethan, Tetrahydrofuran oder Dioxan; eines aliphatischen oder aromatischen Kohlenwasserstoffs, beispielsweise n-Hexan, Benzol, Toluol oder Xylol; oder eines inerten aprotischen, polaren, organischen Lösungsmittels, beispielsweise Dimethylformamid oder Dimethylsulfoxid; oder eines halogenierten, aliphatischen Kohlenwasserstoffs, beispielsweise Methylenchlorid, Chloroform, Tetrachlorkohlenstoff oder 1,2-Dichlorethan, sowie gegebenenfalls in Gegenwart einer Base, beispielsweise Triethylamin oder Pyridin, wobei letzteres sowohl als Lösungsmittel wie auch als Base dienen kann, oder eines Metallhydrids, beispielsweise Natrium- oder Kaliumhydrid. Die Reaktionstemperaturen sind vorzugsweise im Bereich von etwa -80°C bis +50°C, vorzugsweise bei -30°C und Raumtemperatur.

Die Umsetzung nach der Methode bb) erfolgt analog zur Methode aa) mit dem Unterschied allerdings, dass die Reaktionstemperatur im Bereich von -80°C bis +150°C, bevorzugt zwischen 0°C und +130°C liegt.

Die Umsetzung nach der Methode cc) erfolgt zweckmässigerweise, indem man die Verbindungen der Formeln XVIII und XIX in einem wasserfreien Lösungsmittel und säurekatalysiert bei erhöhter Temperatur miteinander reagieren lässt. Als Lösungsmittel kommen insbesondere mit Wasser Azeotrope bildende organische Lösungsmittel, beispielsweise Benzol, Toluol und Xylole; oder halogenierte Kohlenwasserstoffe, beispielsweise Methylenchlorid, Chloroform, Tetrachlorkohlenstoff und Chlorbenzol; oder aliphatische oder cyclische Ether, beispielsweise 1,2-Dimethoxyethan, Tetrahydrofuran oder Dioxan in Frage. Als saure Katalysatoren kommen insbesondere starke Mineralsäuren, beispielsweise Schwefel- oder Salzsäure, organische Säuren, beispielsweise p-Toluolsulfonsäure und Polyphosphorsäure, oder saure Kationentauscher, beispielsweise "Amberlyst 15" (Fluka), in Frage. Der Temperaturbereich liegt für diese Umsetzung zwischen +70°C und +120°C, vorzugsweise bei der Siedetemperatur des Reaktionsgemisches. Unter diesen Reaktionsbedingungen wird gleichzeitig eine rasche Entfernung des gebildeten Reaktionswassers erreicht.

Die Umsetzung nach der Methode dd) erfolgt zweckmässigerweise in einem inerten und mit Wasser mischbaren, organischen Lösungsmittel, beispielsweise in einem aliphatischen oder cyclischen Ether, vorzugsweie 1,2-Dimethoxyethan, Tetrahydrofuran oder Dioxan; oder in einem niederen Alkohol, vorzugsweise Ethanol, bei Temperaturen die zwischen +50°C und +100°C, vorzugsweise bei der Siedetemperatur des Reaktionsgemisches liegen; oder in einem aromatischen Lösungsmittel, beispielsweise Benzol, Toluol oder einem Xylol, in Gegenwart eines sauren Katalysators, beispielsweise Salzsäure oder p-Toluolsulfonsäure, und bei Temperaturen zwischen +50°C und +100°C, vorzugsweise zwischen +60°C und +80°C.

Die Umsetzung der Verbindungen der Formeln XXI und XXII nach der Methode ee) wird zweckmässigerweise in einem wasserfreien, inerten, aprotischen Lösungsmittel, einem Kohlenwasserstoff beispielsweise Benzol, Toluol oder Xylol, einem Halogenkohlenwasserstoff, beispielsweise Dichlormethan oder Chloroform, oder einem Ether, beispielsweise Diethylether, 1,2-Dimethoxyethan, Tetrahydrofuran oder Dioxan, in Gegenwart einer Base, beispielsweise Pyridin, Triethylamin oder 4-Dimethylaminopyridin bei Temperaturen zwischen -50°C und der Rückflusstemperatur des Reaktionsgemisches, vorzugsweise zwischen -5°C und 35°C, durchgeführt.

Die Umsetzung der Verbindungen der Formeln XXIII und XXIV wird zweckmässigerweise in einem wasserfreien, inerten aprotischen Lösungsmittel bei Temperaturen zwischen +70°C und +140°C, vorzugsweise zwischen +100°C und +120°C durchgeführt.

Geeignete Lösungsmittel sind aromatische Kohlenwasserstoffe, beispielsweise Benzol, Toluol und Xylole; halogenierte Kohlenwasserstoffe, beispielsweise Tetrachlorkohlenstoff, Trichlorethan, Tetrachlorethan und Chlorbenzol; oder aliphatische und cyclische Ether, beispielsweise Dibutylether, 1,2-Dimethoxyethan, Tetrahydrofuran und Dioxan. Die anschliessende Umsetzung der so hergestellten Verbindung der Formel XXV mit Carbaminsäureestern der Formel XXVI erfolgt zweckmässigerweise in einem wasserfreien Lösungsmittel und

in Gegenwart eines sauren Katalysators bei erhöhter Temperatur. Als Lösungsmittel kommen vorzugsweise mit Wasser Azeotrope bildende organische Lösungsmittel, beispielsweise aromatische Kohlenwasserstoffe, vorzugsweise Benzol, Toluol und Xylole; halogenierte Kohlenwasserstoffe, beispielsweise Tetrachlorkohlenstoff und Chlorbenzol, und als saure Katalysatoren vorzugsweise starke Mineralsäuren, beispielsweise Schwefelsäure; organische Säuren, beispielsweise p-Toluolsulfonsäure; Phosphor enthaltende Säuren, beispielsweise Orthophosphorsäure und Polyphosphorsäure; oder saure Kationentauscher, beispielsweise "Amberlyst 15" (Fluka), in Betracht. Die angewendeten Temperaturen sind im Bereich von +70°C bis 150°C, vorzugsweise bei der Siedetemperatur des Reaktionsgemisches. Dadurch wird die Entfernung des Reaktionswassers erzielt.

Die Umsetzung des Amins der Formel XXIV mit dem Diketen der Formel XXVII gemäss Methode ff) erfolgt zweckmässigerweise in einem wasserfreien, inerten aprotischen Lösungsmittel, beispielsweise einem halogenierten Kohlenwasserstoff, vorzugsweise Methylenchlorid, Chloroform, Tetrachlorkohlenstoff oder Chlorbenzol; oder einem aromatischen Kohlenwasserstoff, beispielsweise Benzol, Toluol oder einem Xylol; oder einem aliphatischen oder cyclischen Ether, beispielsweise Diethylether, 1,2-Dimethoxyethan, Tetrahydrofuran oder Dioxan. Die Reaktion verläuft basenkatalysiert, beispielsweise in Gegenwart von 4-Pyrrolidino-pyridin, 4-Dimethylaminopyridin, 1,4-Diazabicyclo[2,2,2]octan, 1,5-Diazabicyclo[4,3,0]non-5-en, 1,8-Diazabicyclo[5,4,0]undec-7-en oder Diethylamin und exotherm, weshalb im Temperaturbereich von - 10°C bis +50°C, vorzugsweise bei Raumtemperatur gearbeitet wird. Die anschliessende Umsetzung der so erhaltenen Verbindung der Formel XXV mit dem Carbaminsäureester der Formel XXVI erfolgt in analoger Weise wie unter der Methode ee) beschrieben.

Die in den Verfahrensvarianten aa) bis ff) benötigten Ausgangsverbindungen XV, XVIII, XX, XXI, XXII und XXVI sind vorbekannt.

Die Ausgangsverbindung der Formel XVI (Variante aa)) lässt sich nach bekannten, analogen Methoden aus der Verbindung der Formel XXIV durch Umsetzung mit Phosgen oder Diphosgen herstellen.

Die Ausgangsverbindung der Formel XVII (Variante bb)) lässt sich nach bekannten, analogen Methoden aus der Verbindung der Formel XXIV durch Umsetzung mit Chlorameisensäureestern herstellen.

Die Ausgangsverbindung der Formel XIX (Variante cc)) lässt sich nach bekannten, analogen Methoden aus der Verbindung der Formel XXIV durch Umsetzung mit Metallcyanaten herstellen.

Die Ausgangsverbindung der Formel XXIV (Variante ee) und ff)) lässt sich aus bekannten, analogen Methoden aus der Verbindung der Formel XXIX

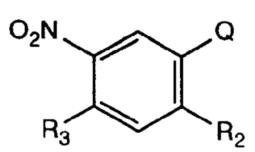

(XXIX)

druch Reduktion der Nitrogruppe herstellen.

Die Ausgangsverbindung der Formel XXIX lässt sich aus bekannten, analogen Methoden aus der Verbindung der Formel XXX

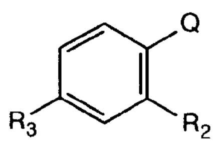

(XXX)

durch Nitrierung herstellen.

Die Zwischenprodukte der Formeln IIa, IIb, IV, V und VI sind neu; sie wurden speziell für die Synthese der erfindungsgemässen Wirkstoffe der Formel I synthetisiert und bilden einen Bestandteil der vorliegenden Erfindung.

Es wurde nun gefunden, dass die erfindungsgemässen Verbindungen der Formel I (im folgenden auch als "Wirkstoffe" bezeichnet), sowie ihre Enolether oder Salze wertvolle Wirkstoffe mit herbiziden Eigenschaften sind und sich zur Bekämpfung von Unkräutern, einschliesslich Ungräsern, so unter anderem von Agropyron repens, Alopecurus myosuroides, Avena fatua, Bromus inermis, Echinochloa crus-galli, Poa annua, Sorghum halepense, Abutilon theophrasti, Amaranthus retroflexus, Cassia obtusifolia, Chenopodium album, Galium aparine, Matricaria chamomilla, Sinapis arvensis und Stellaria media, in verschiedenen Nutzpflanzenkulturen, so u.a. Kulturen von Reis, insbesondere Wasserreis, Weizen, Mais, Soja, Raps und Baumwolle eignen. Zudem sind die Verbindungen sowohl Vorauflauf- als auch Nachauflauf-Herbizide. Bei einigen Vertretern der Verbindungen der Formel I hat sich eine gute Selektivität gezeigt, beispielsweise bei der Bekämpfung von Unkräutern in Soja- und Baumwolle-Kulturen.

In der Praxis genügt üblicherweise eine Konzentration von 1 g bis 3 kg der Verbindung der Formel I pro ha, vorzugsweise von 10 g bis 1 kg/ha, um den gewünschten herbiziden Effekt zu erzielen. Um den gewünschten herbiziden Effekt bei optimaler Nutzpflanzenverträglichkeit zu erzielen, ist der Bereich von 10 bis 100 g/ha in der Vorauflaufbehandlung und von 100 bis 1000 g/ha in der Nachauflaufbehandlung besonders günstig.

Die erfindungsgemässen Unkrautbekämpfungsmittel enthalten eine wirksame Menge mindestens einer Verbindung der Formel I, oder eines Enolethers oder Salzes davon, sowie in aller Regel ausserdem Formulierungshilfsstoffe. Zweckmässigerweise enthalten sie zumindest je einen Formulierungshilfsstoff aus jeder der folgenden Gruppen:

- feste Trägerstoffe;
- Lösungs- bzw. Dispersionsmittel;
- Tenside (Netzmittel und Emulgatoren);
- Dispergiermittel (ohne Tensidwirkung); und
- Stabilisatoren.

Die pestiziden Zubereitungen enthalten in der Regel neben den Wirkstoffen der Formel I 1 bis 99 % eines Formulierungshilfsstoffs aus der Gruppe

- feste Trägerstoffe;
- Lösungs- bzw. Dispersionsmittel;
- Dispergiermittel (ohne Tensidwirkung); und
- Stabilisatoren; und 0 bis 25 %, insbesondere 0,1 bis 25 % eines Tensides (Netzmittel und Emulgatoren).

Unter Verwendung solcher und anderer Hilfsstoffe können die Verbindungen der Formel I, d.h. die herbiziden Wirkstoffe, in die üblichen Formulierungen, wie Stäube, Pulver, Granulate, Lösungen, Emulsionen, Suspensionen, emulgierbare Konzentrate, Pasten und dgl. übergeführt werden.

Die Verbindungen der Formel I und ihre Enolether sind im allgemeinen wasserunlöslich, die Salze hingegen, insbesondere die Alkalimetallsalze und Ammoniumsalze, im all- die Salze hingegen, insbesondere die Alkalimetallsalze und Ammoniumsalze, im allgemeinen wasserlöslich, und können nach den für wasserunlösliche bzw. wasserlösliche Verbindungen üblichen Methoden unter Verwendung der bekannten Formulierungshilfsstoffe konfektioniert werden. Die Herstellung der Mittel kann in an sich bekannter Weise durchgeführt werden, beispielsweise durch Vermischen des jeweiligen Wirkstoffes mit festen Trägerstoffen, durch Auflösen oder Suspendieren in geeigneten Lösungs- bzw. Dispersionsmitteln, gegebenenfalls unter Verwendung von Tensiden als Netzmitteln oder Emulgatoren und/oder von Dispergiermitteln, oder durch Verdünnen bereits vorbereiteter emulgierbarer Konzentrate mit Lösungs- bzw. Dispersionsmitteln.

Als feste Trägerstoffe kommen im wesentlichen in Frage:

- natürliche Mineralstoffe, wie Kreide, Dolomit, Kalkstein, Tonerden und Kieselsäure oder deren Salze, beispielsweise Kieselgur, Kaolin, Bentonit, Talkum, Attapulgit oder Montmorillonit;
- synthetische Mineralstoffe, wie hochdisperse Kieselsäure, Aluminiumoxid oder Silikate;
- organische Stoffe, wie Cellulose, Stärke, Harnstoffe oder Kunstharze; oder
- Düngemittel, wie Phosphate oder Nitrate. Solche Trägerstoffe können beispielsweise als Pulver oder als Granulate vorliegen.

Als Lösungs- bzw. Dispersionmittel kommen im wesentlichen in Frage: -Aromaten, wie Benzol, Toluol, Xylole und Alkylnaphthaline; - chlorierte Aromaten und chlorierte aliphatische Kohlenwasserstoffe, wie Chlorbenzole, Chlorethylene oder Methylenchlorid; - aliphatische Kohlenwasserstoffe, wie Cyclohexan oder Paraffine, beispielsweise Erdölfraktionen; - Alkohole, wie Butanol oder Glykol und deren Ether und Ester; - Ketone, wie Aceton, Methylethylketon, Methylisobutylketon oder Cyclohexanon; oder - stark polare Lösungs- bzw. Dispersionsmittel, wie Dimethylformamid, N-Methylpyrrolidon oder Dimethylsulfoxid (wobei solche Lösungsmittel vorzugsweise Flammpunkte von mindestens 30°C und Siedepunkte von mindestens 50°C aufweisen) oder Wasser. Weiter kommen als Lösungs- bzw. Dispersionsmittel auch sogenannte verflüssigte gasförmige Streckmittel oder Trägerstoffe, welche Produkte sind, die bei Raumtemperatur und unter Normaldruck gasförmig sind, in Frage. Beispiele solcher Produkte sind insbesondere Aerosol-Treibgase, wie Halogenkohlenwasserstoffe, beispielsweise Dichlordifluormethan. Liegt das erfindungsgemässe Unkrautbekämpfungsmittel in Form einer Druckgaspackung vor, so wird zweckmässigerweise zusätzlich zum Treibgas ein Lösungsmittel eingesetzt.

Die Tenside (Netzmittel und Emulgatoren) können nichtionische Verbindungen sein, wie: - Kondensationsprodukte von Fettsäuren, Fettalkoholen oder fettsubstituierten Phenolen mit Ethylenoxid; - Fettsäureester und -ether von Zuckern oder mehrwertigen Alkoholen; - die Produkte, welche aus Zuckern oder mehrwertigen Alkoholen durch Kondensation mit Ethylenoxid erhalten werden; - Blockpolymere von Ethylenoxid und Propylenoxid; oder - Alkyldimethylaminoxide.

Die Tenside können auch anionische Verbindungen sein, wie: -Seifen; - Fettsulfatester, beispielsweise Natrium-dodecylsulfat, Natrium-octadecylsulfat und Natrium-cetylsulfat; - Alkylsulfonate, Arylsulfonate oder fettaromatische Sulfonate, wie Alkylbenzolsulfonate, beispielsweise Calcium-dodecylbenzolsulfonat, oder Butylnaphthalinsulfonate; oder - komplexere Fettsulfonate, beispielsweise die Amidkondensationsprodukte von Oelsäure und N-Methyltaurin oder das Natriumsulfonat von Dioctylsuccinat.

Schliesslich können die Tenside kationische Verbindungen sein, wie Alkyldimethylbenzylammoniumchloride, Dialkyldimethylammoniumchloride, Alkyltrimethylammoniumchloride oder ethoxylierte Ammoniumchloride.

Als Dispergiermittel (ohne Tensidwirkung) kommen im wesentlichen in Frage: Lignin, Natrium- und Ammoniumsalze von Ligninsulfonsäuren, Natriumsalze von Maleinsäureanhydrid-Diisobutylen-Copolymeren, Natrium- und Ammoniumsalze von sulfonierten Polykondensationsprodukten aus Naphthalin und Formaldehyd, oder Sulfitablaugen.

Als Dispergiermittel, welche sich insbesondere als Verdickungs- bzw. Antiabsetzmittel eignen, können beispielsweise Methylcellulose, Carboxymethylcellulose, Hydroxyethylcellulose, Polyvinylalkohol, Alignate, Caseinate und Blutalbumin eingesetzt werden.

Beispiele von geeigneten Stabilisatoren sind: - säurebindende Mittel, beispielsweise Epichlorhydrin, Phenylglycidether oder Soyaepoxide; - Antioxidantien, beispielsweise Gallussäureester oder Butylhydroxytoluol; - UV-Absorber, beispielsweise substituierte Benzophenone, Diphenylacrylonitrilsäureester oder Zimtsäureester, oder -Deaktivatoren, beispielsweise Salze der Ethylendiaminotetraessigsäure und Polyglykole.

Die erfindungsgemässen Unkrautbekämpfungsmittel enthalten im allgemeinen zwischen 0,001 und 95 Gewichtsprozent, vorzugsweise zwischen 0,5 und 75 Gewichtsprozent, einer oder mehrerer erfindungsgemässer Verbindungen der Formel I als Wirkstoff(e). Sie können beispielsweise in einer Form vorliegen, die sich für die Lagerung und den Transport eignet. In solchen Formulierungen, beispielsweise emulgierbaren Konzentraten, ist die Wirkstoffkonzentration normalerweise im höheren Bereich, vorzugsweise zwischen 1 und 50 Gewichtsprozent, insbesondere zwischen 5 und 30 Gewichtsprozent. Diese Formulierungen können dann, beispielsweise mit gleichen oder verschiedenen inerten Stoffen, bis zur Wirkstoffkonzentration verdünnt werden, die sich für den praktischen Gebrauch eignet, also vorzugsweise ca. 0.001 bis 10 Gewichtsprozent, insbesondere etwa 0.005 bis 5 Gewichtsprozent. Die Wirkstoffkonzentrationen können jedoch auch kleiner oder grösser sein.

Wie erwähnt, kann die Herstellung der erfindungsgemässen Unkrautbekämpfungsmittel in an sich bekannter Weise durchgeführt werden.

Zur Herstellung pulverförmiger Präparate kann der Wirkstoff, d.h. mindestens eine erfindungsgemässe Verbindung der Formel I mit einem festen Trägerstoff vermischt werden, beispielsweise durch Zusammenmahlen. Oder man kann den festen Trägerstoff mit einer Lösung oder Suspension des Wirkstoffes imprägnieren und dann das Lösungs- bzw. Dispersionsmittel durch Abdunsten, Erhitzen oder Absaugen unter vermindertem Druck entfernen. Durch Zusatz von Tensiden bzw. Dispergiermitteln kann man solche pulverförmige Mittel mit Wasser leicht benetzbar machen, so dass sie in wässrige Suspensionen, die sich beispielsweise als Spritzmittel eignen, übergeführt werden können.

Der Wirkstoff der Formel I kann auch mit einem Tensid und einem festen Trägerstoff zur Bildung eines netzbaren Pulvers, welches in Wasser dispergierbar ist, vermischt werden, oder er kann mit einem festen, vorgranulierten Trägerstoff zur Bildung eines granulatförmigen Produktes vermischt werden.

Gewünschtenfalls kann der Wirkstoff der Formel I in einem mit Wasser nicht mischbaren Lösungsmittel, wie beispielsweise einem hochsiedenden Kohlenwasserstoff, gelöst werden. Dieses enthält zweckmässigerweise gelösten Emulgator, so dass die Lösung bei ihrer Zugabe zu Wasser selbstemulgierend ist. Andererseits kann der Wirkstoff mit einem Emulgator vermischt und das Gemisch dann mit Wasser auf die gewünschte Konzentration verdünnt werden. Zudem kann der Wirkstoff in einem Lösungsmittel gelöst und danach mit einem Emulgator vermischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die erfindungsgemässe Verwendung der beschriebenen Unkrautbekämpfungsmittel kann nach üblichen Applikationsmethoden, wie Spritzen, Sprühen, Stäuben, Giessen oder Streuen, erfolgen.

Die folgenden Beispiele dienen der näheren Erläuterung der Erfindung.

## A. Herstellung der erfindungsgemässen Verbindungen der Formel I

### Beispiel 1: 2-Chlor-4-fluor-5-[3,6-dihydro-2,6-dioxo-4-trifluormethyl-1(2H)-pyrimidinyl]-acetophenon

1,0 g Natriumhydrid (55 %ige Dispersion in Oel) wird in 20 ml Dimethylformamid vorgelegt, mit 1 ml n-Hexan versetzt und auf - 10°C gekühlt. 4,2 g 3-Amino-4,4,4-trifluorcrotonsäureethylester werden innerhalb 1/2 Stunde zugetropft und 1 Stunde nachgerührt. Anschliessend wird die Reaktionslösung auf -40°C abgekühlt und 5,0 g 3-Acetyl-4-chlor-6-fluorphenylisocyanat in 20 ml Diethylether zugetropft. Nach beendeter Zugabe des Isocyanats wird das Kühlbad entfernt und während 4 Stunden bis zum Erreichen der Raumtemperatur nachgerührt.

Die Aufarbeitung des Reaktionsgemisches erfolgt durch Aufgiessen auf 100 ml 1N Salzsäurelösung und Extraktion des wässrigen Gemisches mit Essigsäureethylester. Die abgetrennte organische Phase wird neutralgewaschen, getrocknet und am Rotovap eingeengt, wobei das Produkt auskristallisiert; Smp.: 254°C.
$^1$H-NMR. ($CDCl_3$, 60 MHz): 8,08 ppm (d, 1H), 7,85 ppm (d, 1H), 6,51 ppm (s, 1H), 2,63 ppm (s, 3H).

Beispiel 2: 2-Chlor-5-[3,6-dihydro-2,6-dioxo-4-trifluormethyl-1(2H)-pyrimidinyl]-acetophenon

22,8 g 60-65%ige Natriumhydrid-Suspension in Weissöl werden in 400 ml Dimethylformamid unter Stickstoffatmosphäre vorgelegt. Dazu werden 103,9 g 3-Amino-4,4,4-trifluorcrotonsäureethylester in 400 ml Dimethylformamid innerhalb 2 Stunden bei Raumtemperatur zugetropft. Nach beendeter Wasserstoffentwicklung werden 137,0 g fester 3-Acetyl-4-chlor-carbanilsäureethylester in einer Portion zugegeben und das Reaktionsgemisch während 3 Stunden auf 150°C erhitzt. Das während der Reaktion entstehende Ethanol wird laufend abdestilliert.

Zur Aufarbeitung wird das Reaktionsgemisch auf 1,5 kg Eis und 1,5 l Wasser gegossen und mit 100 ml konzentrierter Salzsäure sauergestellt. Die sich bildenden Kristalle werden abgenutscht und mit 400 ml Wasser nachgewaschen. Der trockengesaugte Kristallbrei wird in 400 ml Ethanol während 15 Minuten aufgerührt und abgenutscht; zweimal wird mit je 70 ml Ethanol nachgewaschen und die so erhaltenen Kristalle im Vakuum bei 80°C getrocknet; Smp.: >240°C.

Analog zu Beispiel 2 werden die in der Tabelle 1 aufgeführten Verbindungen der Formel Ie hergestellt.

Tabelle 1: Verbindungen der Formel Ie

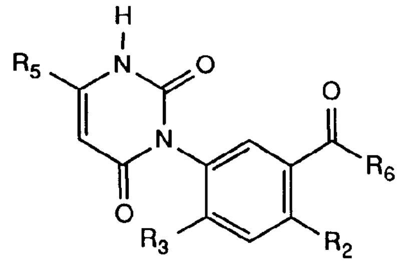

(Ie)

| Beispiel | $R_6$ | $R_3$ | $R_2$ | $R_5$ | Phys.-chem. Daten |
|---|---|---|---|---|---|
| 3 | $-CH(CH_3)_2$ | H | Cl | $-CF_3$ | Smp. >220°C |
| 4 | $-(CH_2)_3CH_3$ | H | Cl | $-CF_3$ | Smp. 156-160°C |
| 5 | $-(CH_2)_4CH_3$ | H | Cl | $-CF_3$ | Smp. 120-122°C |
| 6 | $-CH_2CH_2CH(CH_3)_2$ | H | Cl | $-CF_3$ | Smp. 120-123°C |
| 7 | 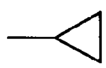 | H | Cl | $-CF_3$ | Smp. 195-199°C |
| 8 | 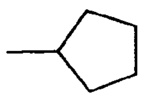 | H | Cl | $-CF_3$ | Smp. 185-188°C |
| 9 | 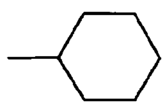 | H | Cl | $-CF_3$ | Smp. 184-186°C |
| 10 | $-CH_3$ | F | F | $-CF_3$ | $^1$H-NMR. ($CDCl_3$, 60 $MH_z$): 8,10 ppm (t, 1H), 7,54 ppm (t, 1H) 6,38 ppm (s, 1H), 2,64 und 2,53 ppm (s, 3H). |
| 11 | $-CH_2CH_3$ | H | Cl | $-CF_2CF_3$ | Smp. 177-180°C |
| 12 | $-CH(CH_3)_2$ | H | Cl | $-CF_2CF_3$ | Smp. 159-161°C |
| 13 | $-CH_2CH_3$ | H | Cl | $-CF_3$ | Smp. 191-194°C |
| 14 | $-CH_2CH_2CH_3$ | H | Cl | $-CF_3$ | Smp. 183-184°C |
| 15 | $-CH_3$ | H | F | $-CF_3$ | Smp. 239-245°C |
| 16 | $-CH_3$ | H | Br | $-CF_3$ | Smp. 255-260°C |

Beispiel 17: 2-Chlor-4-fluor-5-[3,6-dihydro-2,6-dioxo-4-methyl-1(2H)-pyrimidinyl]acetophenon

5,5 g einer 33%igen Natriumhydrid-Suspension werden portionenweise zu 150 ml Isopropanol gegeben. Nach beendeter Wasserstoffentwicklung gibt man 23,2 g Ethyl-[1-[[4-chlor-6-fluor-3-acetophenyl]carbamoyl]methylen-ethyl]-carbamat dazu und lässt die Lösung während 2 Stunden bei Raumtemperatur weiterrühren. Anschliessend wird das Reaktionsgemisch mit 1 N Salzsäurelösung versetzt und mit Essigsäureethylester extrahiert. Die organische Phase wird neutralgewaschen, getrocknet und am Rotovap eingeengt. Zur Reindarstellung wird das Rohprodukt über 1,0 kg Kieselgel und mit Essigsäureethylester/n-Hexan (1:1-Gemisch) als Eluenten chromatographiert; Smp.: 204-206°C. $^1$H-NMR. ($CDCl_3$, 400 MHz): 9,97 ppm (s, 1H), 7,64

ppm (d, 1H), 7,33 ppm (d, 1H), 5,67 ppm (s, 1H), 2,68 ppm (s, 3H), 2,12 ppm (s, 3H).

Beispiel 18: 2-Chlor-5-[3,6-dihydro-2,6-dioxo-4-methyl-1(2H)-pyrimidinyl-propiophenon

13,1 g 5-(Acetoacetylamino)-2-chlor-4-fluor-propiophenon, 8,2 g Urethan und 0,2 g p-Toluolsulfonsäure werden in 300 ml Benzol während 7 Stunden in der Dean-Stark-Apparatur erhitzt. Das Lösungsmittel wird eingedampft und der erhaltene Rückstand in 50 ml Methanol gelöst und darauf zu einer zuvor aus 1,1 g Natrium und 60 ml Methanol zubereiteten Lösung zugegeben. Dieses Reaktionsgemisch wird während 2 Stunden bei 60°C gerührt. Nach dem Abkühlen wird mit Essigsäure angesäuert und eingedampft. Der Rückstand wird in 500 ml Essigsäureethylester gelöst und die organische Phase mit 300 ml Wasser und dann mit 200 ml gesättigter Natriumchloridlösung gewaschen, über Natriumsulfat getrocknet und eingeengt. Die Reindarstellung des Rohproduktes erfolgt mittels Kieselgelsäule und Essigsäureethylester/n-Hexan 1/1 als Laufmittel und anschliessend mittels Umkristallisation aus Essigsäureethylester/n-Hexan; Smp.: 197-199°C.

Beispiel 19: 2-Chlor-5-[3,6-dihydro-2,6-dioxo-3-methyl-4-trifluormethyl-1(2H)-pyrimidinyl]-acetophenon

32,0 g 2-Chlor-5-[3,6-dihydro-2,6-dioxo-4-trifluormethyl-1(2H)-pyrimidinyl]-acetophenon, 12,0 g Dimethylsulfat und 13,3 g Kaliumcarbonat werden in 500 ml Aceton während 3 Stunden am Rückfluss erhitzt. Anschliessend wird das Reaktionsgemisch eingeengt, der Rückstand mit 300 ml Wasser versetzt und dieses Gemisch zweimal mit je 300 ml Essigsäureethylester extrahiert. Die vereinigten organischen Phasen werden zweimal mit je 200 ml gesättigter Natriumchloridlösung gewaschen, über Natriumsulfat getrocknet und eingeengt. Das Dünnschichtchromatogramm des erhaltenen Rückstandes (Laufmittel Essigsäureethylester/n-Hexan 1/1) zeigt im UV-Licht zwei Produkte an. Die Auftrennung mittels Kieselgelsäule und Essigsäureethylester/n-Hexan als Laufmittel liefert als erste Verbindung 2-Chlor-5-[2-methoxy-6-oxo-4-trifluormethyl-1(6H)-pyrimidinyl]-acetophenon als gelbes Oel; $^1$H-NMR. ($d_6$-DMSO, 200 MHz): 7,88 ppm (d,1H), 7,73 ppm (d,1H), 7,62 ppm (dxd,1H), 6,79 ppm (s,1H), 3,90 ppm (s,3H), 2,59 ppm (s,3H); und als zweite Verbindung 2-Chlor-5-[3,6-dihydro-2,6-dioxo-3-methyl-4-trifluormethyl-1(2H)-pyrimidinyl]-acetophenon als Festkörper, der aus Essigsäureethylester/n-Hexan umkristallisiert wird; Smp.: 175-176°C.

Analog zu Beispiel 19 werden die in den Tabellen 2a und 2b zusammengestellten Verbindungen der Formeln If und Ig hergestellt.

Tabelle 2a: Verbindungen der Formel If

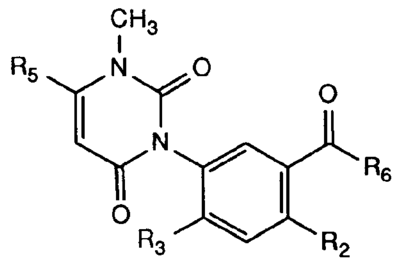

(If)

| Beispiel | $R_6$ | $R_2$ | $R_3$ | $R_5$ | Phys.-chem. Daten |
|---|---|---|---|---|---|
| 20 | $-CH(CH_3)_2$ | Cl | H | $-CF_3$ | Smp. 168-169°C |
| 21 | $-(CH_2)_3CH_3$ | Cl | H | $-CF_3$ | Smp. 70-72°C |
| 22 | $-(CH_2)_4CH_3$ | Cl | H | $-CF_3$ | Smp. 89-90°C |
| 23 | $-CH_2CH_2CH(CH_3)_2$ | Cl | H | $-CF_3$ | Oel; $^1$H-NMR. ($CDCl_3$, 200 MHz): 7,54 ppm (d, 1H), 7,36 ppm (d, 1H), 7,24 ppm (dxd, 1H), 6,37 ppm (s, 1H), 3,55 ppm (s, 3H), 2,86-3,02 ppm (m, 2H), 1,52-1,71 ppm (m, 3H), 0,84-1,00 ppm (m, 6H). |
| 24 | 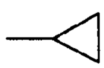 | Cl | H | $-CF_3$ | Smp. 157-159°C |
| 25 | 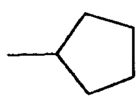 | Cl | H | $-CF_3$ | Smp. 142-144°C |
| 26 | 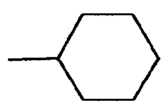 | Cl | H | $-CF_3$ | Smp. 118-121°C |
| 27 | $-CH_3$ | Cl | F | $-CF_3$ | Smp. 128°C |
| 28 | $-CH_3$ | Cl | F | $-CH_3$ | Smp. 47°C |
| 29 | $-CH_3$ | F | F | $-CF_3$ | Smp. 164°C |
| 30 | $-CH(CH_3)_2$ | Cl | H | $-CF_2CF_3$ | Oel; $^1$H-NMR. ($d_6$-DMSO, 400 MHz); 7,70 ppm (d, 1H), 7,58 ppm (d, 1H), 7,46 ppm (dxd, 1H), 6,44 ppm (s, 1H), 3,41 ppm (s, 3H), 3,23 ppm (septett, 1H), 1,10 ppm (d, 6H). |

Tabelle 2a: (Fortsetzung)

| Beispiel | $R_6$ | $R_2$ | $R_3$ | $R_5$ | Phys.-chem. Daten |
|---|---|---|---|---|---|
| 31 | $-CH_2CH_3$ | Cl | H | $-CF_3$ | Smp. 101-103°C |
| 32 | $-CH_2CH_3$ | Cl | H | $-CF_2CF_3$ | Smp. 102-103°C |
| 33 | $-CH_2CH_2CH_3$ | Cl | H | $-CF_3$ | Smp. 135-137°C |
| 34 | $-CH_3$ | Br | H | $-CF_3$ | Smp. 162-163°C |
| 35 | $-CH_3$ | F | H | $-CF_3$ | Smp. 181-183°C |
| 36 | $-CH_2CH_3$ | Cl | F | $-CF_3$ | Smp. 117-120°C |

Tabelle 2b: Verbindungen der Formel Ig

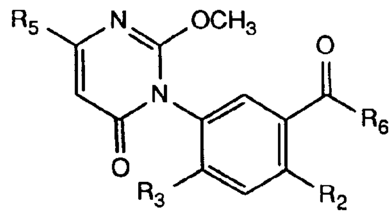

(Ig)

| Beispiel | $R_6$ | $R_2$ | $R_3$ | $R_5$ | Phys.-chem. Daten |
|---|---|---|---|---|---|
| 37 | $-CH_2CH_2CH_3$ | Cl | H | $-CF_3$ | Oel; $^1$H-NMR. ($d_6$-DMSO, 200 MHz): 7,82 ppm (d, 1H), 7,72 ppm (d, 1H), 7,60 ppm (dxd, 1H), 6,78 ppm (s, 1H), 3,91 ppm (s, 3H), 2,90 ppm (t, 2H), 1,62 ppm (sextett, 2H), 0,92 ppm (t, 3H). |
| 38 | $-(CH_2)_3-CH_3$ | Cl | H | $-CF_3$ | Oel; $^1$H-NMR. ($d_6$-DMSO, 200 MHz): 7,82 ppm (d, 1H), 7,72 ppm (d, 1H), 7,61 ppm (dxd, 1H), 6,78 ppm (s, 1H), 3,91 ppm (s, 3H), 2,92 ppm (t, 2H), 1,49-1,66 ppm (m, 2H), 1,22-1,44 ppm (m, 2H), 0,88 ppm (t, 3H). |
| 39 | $-(CH_2)_4-CH_3$ | Cl | H | $-CF_3$ | Oel; $^1$H-NMR. ($CDCl_3$, 200 MHz): 7,56 ppm (d, 1H), 7,32 ppm (d, 1H), 7,22 ppm (dxd, 1H), 6,61 ppm (s, 1H), 3,99 ppm (s, 3H), 2,82-3,01 ppm (m, 2H), 1,62-1,82 ppm (m, 2H), 1,25-1,45 ppm (m, 4H), 0,84-0,96 ppm (m, 3H). |
| 40 | $-(CH_2)_2-CH(CH_3)_2$ | Cl | H | $-CF_3$ | Oel; $^1$H-NMR. ($CDCl_3$, 200 MHz): 7,56 ppm (d, 1H), 7,31 ppm (d, 1H), 7,22 ppm (dxd, 1H), 6,62 ppm (s, 1H), 3,99 ppm (s, 3H), 2,82-3,04 ppm (m, 2H), 1,51-1,73 ppm (m, 3H), 0,82-0,98 ppm (m, 6H). |
| 41 | 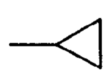 | Cl | H | $-CF_3$ | Smp. 105-106°C |

Tabelle 2b: (Fortsetzung)

| Beispiel | $R_6$ | $R_2$ | $R_3$ | $R_5$ | Phys.-chem. Daten |
|---|---|---|---|---|---|
| 42 | 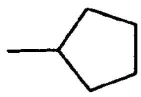 | Cl | H | $-CF_3$ | Smp. 96-98°C |
| 43 | $-CH_3$ | F | H | $-CF_3$ | Smp. 161-163°C |
| 44 | $-CH_3$ | Br | H | $-CF_3$ | Smp. 162-163°C |
| 45 | $-CH_2CH_3$ | Cl | H | $-CF_2CF_3$ | Oel; $^1$H-NMR. ($d_6$-DMSO, 200 MHz): 7,85 ppm (d, 1H), 7,71 ppm (d, 1H), 7,62 ppm (dxd, 1H), 6,81 ppm (s, 1H), 3,90 ppm (s, 3H), 2,94 ppm (quartett, 2H), 1,08 ppm (t, 3H). |
| 46 | $-CH(CH_3)_2$ | Cl | H | $-CF_2CF_3$ | Oel; $^1$H-NMR. ($d_6$-DMSO, 200 MHz): 7,78 ppm (d, 1H), 7,72 ppm (d, 1H), 7,60 ppm (dxd, 1H), 6,80 ppm (s, 1H), 3,89 ppm (s, 3H), 3,26 ppm (septett, 1H). |

Beispiel 47: 2-Chlor-5-[3-allyl-3,6-dihydro-2,6-dioxo-4-trifluormethyl-1(2H)-pyrimidinyl]-propiophenon

0,5 g Natriumhydrid (50-60%ige Suspension in Weissöl) wird in 20 ml N,N-Dimethylformamid vorgelegt. Dazu wird innerhalb 15 Minuten eine Lösung aus 3,5 g 2-Chlor-5-[3,6-dihydro-2,6-dioxo-4-trifluormethyl-1(2H)-pyrimidinyl]-propiophenon in 30 ml N,N-Dimethylformamid bei 10-20°C zugetropft und während 30 Minuten bei 20°C nachgerührt. Darauf wird 2,4 g Allylbromid zugegeben und während 16 Stunden bei 20°C gerührt. Das Reaktionsgemisch wird dann auf 400 ml Wasser gegossen und diese wässrige Mischung zweimal mit je 300 ml Essigsäureethylester extrahiert. Die vereinigten organischen Phasen werden zweimal mit je 100 ml gesättigter Natriumchloridlösung gewaschen, über Natriumsulfat getrocknet und eingeengt. Die Reindarstellung des erhaltenen Rückstandes erfolgt mittels Kieselgelsäule und Essigsäureethylester/n-Hexan 1/7 als Laufmittel und Umkristallisation aus Diethylether/n-Hexan; Smp.: 165-169°C.

Analog zu Beispiel 47 erhält man 2-Chlor-5-[3,6-dihydro-2,6-dioxo-3-propargyl-4-trifluormethyl-1(2H)-pyrimidinyl]-propiophenon; Smp.: 138-141°C (Beispiel 48).

Beispiel 49: 2-Chlor-5-[3,6-dihydro-2,6-dioxo-3-methyl-4-trifluormethyl-1(2H)pyrimidinyl]-acetophenon

1,10 g 2-Chlor-5-[3,6-dihydro-2,6-dioxo-3-methyl-4-trifluormethyl-1(2H)-pyrimidinyl]benzoesäurechlorid werden in 200 ml absolutem Tetrahydrofuran unter Stickstoffatmosphäre gelöst. Dazu tropft man unter Rühren bei -70°C 1,0 ml einer 3 molaren Lösung Methylmagnesiumchlorid in Tetrahydrofuran innerhalb 30 Minuten. Es wird noch während 2 Stunden bei -70°C nachgerührt, und darauf weitere 0,2 ml der angegebenen Grignardlösung zugetropft und 1 Stunde bei -70°C nachgerührt. Anschliessend lässt man die Reaktionstemperatur auf 0°C ansteigen und gibt 20 ml einer 2N Salzsäurelösung dazu. Dieses Gemisch wird auf 200 ml Wasser gegossen und zweimal mit je 100 ml Diethylether extrahiert. Die vereinigten Extrakte werden mit Wasser gewaschen, über Natriumsulfat getrocknet und am Rotovap eingeengt. Die Reindarstellung erfolgt über Kieselgel mit n-Hexan/Essigsäureethylester als Eluenten. Das erhaltene Produkt zeigt einen Smp.: 177-178°C (Siehe auch Beispiel 19).

Beispiel 50: 2-Chlor-4-fluor-5-[1,2,4,5,6,7-hexahydro-1-methyl-2,4-dioxo-3H-cyclopenta-[d]pyrimidin-3-yl]-acetophenon

0,95 g 2-Chlor-4-fluor-5-[1,2,4,5,6,7-hexahydro-1-methyl-2,4-dioxo-3H-cyclopenta[d]-pyrimidin-3-yl]-N-methoxy-N-methylbenzamid werden in 10 ml absolutem Tetrahydrofuran bei 0°C vorgelegt. 0,85 ml einer 22%igen Lösung Methylmagnesiumchlorid in Tetrahydrofuran werden unter Rühren langsam zugetropft und nachfolgend noch 1 Stunde nachgerührt. Das Reaktionsgemisch wird auf 10 g Eis und 5 ml 2N Schwefelsäure gegeben und dieses Gemisch zweimal mit je 25 ml Essigsäureethylester extrahiert. Nach dem Neutralwaschen der organischen Phase wird diese eingeengt und der ölige Rückstand über 100 g Kieselgel G mit dem Lösungsmittelgemisch n-Hexan/Essigsäureethylester 8/2 rein dargestellt: farbloses Oel.
$^1$H-NMR. ($CDCl_3$, 400 MHz): 7,60 ppm (d, 1H), 7,31 ppm (d, 1H), 3,41 ppm (s, 2H), 2,94 ppm (m, 2H), 2,80 ppm (s, 3H), 2,65 ppm (s, 3H), 2,17 ppm (m, 2H).

Analog zu Beispiel 50 werden die in der Tabelle 3 zusammengestellten Verbindungen der Formel Ih hergestellt.

Tabelle 3: Verbindungen der Formel Ih

| Beispiel | Struktur | Phys.-chem. Daten |
|---|---|---|
| 51 | 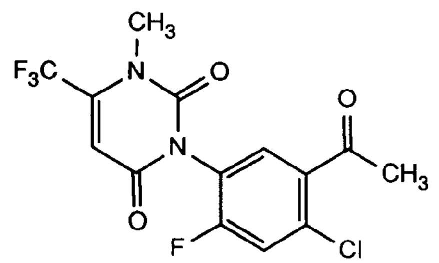 | $^1$H-NMR. ($CDCl_3$, 60 MHz): 7,65 ppm (d, 1H), 7,40 ppm (d, 1H), 6,61 ppm (s, 1H), 2,70 ppm (s, 3H). |
| 52 | 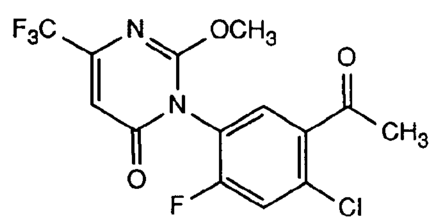 | $^1$H-NMR. ($CDCl_3$, 60 MHz): 7,62 ppm (d, 1H), 7,40 ppm (d, 1H), 6,65 ppm (s, 1H), 4,05 ppm (s, 3H), 2,70 ppm (s, 3H). |

Beispiel 53: 2-Chlor-5-[3,6-dihydro-2,6-dioxo-3-methyl-4-trifluormethyl-1(2H)-pyrimidinyl]-benzaldehyd

Zu einer Suspension von 19,38 g Pyridiniumchlorchromat in 250 ml Dichlormethan wird während 30 Minuten unter Rühren bei Raumtemperatur eine Lösung von 20,08 g 2-Chlor-5-[3,6-dihydro-2,6-dioxo-3-methyl-4-trifluormethyl-1(2H)-pyrimidinyl]-benzylalkohol in 250 ml Dichlormethan zugetropft und das Reaktionsgemisch während 3 Stunden bei 25-28°C nachgerührt. Die klare Lösung wird dekantiert, der braune Rückstand mit 100 ml Dichlormethan gewaschen und die Dichlormethanlösung über 150 g Kieselgel filtriert. Das Filtrat wird eingeengt und der Rückstand aus n-Hexan/Essigsäureethylester umkristallisiert; Smp.: 168-169°C.

Analog zu Beispiel 53 erhält man 2-Chlor-5-[3,6-dihydro-2,6-dioxo-3-methyl-4-trifluormethyl-1(2H)-pyrimidinyl]-4-fluorbenzaldehyd; Smp.: 180-181°C (Beispiel 54).

Beispiel 55: 2-Chlor-5-[3,6-dihydro-2,6-dioxo-3-methyl-4-trifluormethyl-1(2H)-pyrimidinyl] -acetophenonoxim

2,5 g 2-Chlor-5-[3,6-dihydro-2,6-dioxo-3-methyl-4-trifluormethyl- 1(2H)-pyrimidinyl]-acetophenon und 1,0 g Hydroxylamin·Hydrochlorid werden in 20 ml Ethanol während 4 Stunden am Rückfluss erhitzt, und anschliessend mit 200 ml Essigsäureethylester verdünnt. Dieses Gemisch wäscht man mit 100 ml gesättigter Natriumchloridlösung, trocknet über Natriumsulfat und engt am Rotovap ein. Die Reindarstellung des Rückstandes erfolgt über Kieselgel mit Essigsäureethylester/n-Hexan 3/7 als Eluenten und anschliessend durch Umkristallisation aus Diethylether/n-Hexan; Smp.: 215-218°C.

Analog zu Beispiel 55 werden die in der Tabelle 4 zusammengestellten Verbindungen der Formel Ii hergestellt.

Tabelle 4: Verbindungen der Formel Ii

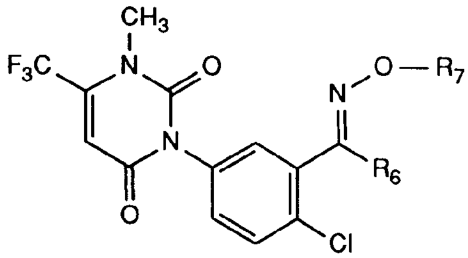

(Ii)

| Beispiel | $R_6$ | $R_7$ | Phys.-chem. Daten |
|---|---|---|---|
| 56 | $-CH_3$ | $-CH_3$ | Oel; $^1$H-NMR. ($d_6$-DMSO, 200 MHz): 7,62-7,70 ppm (m, 1H), 7,32-7,41 ppm (m, 2H), 6,54 ppm (s, 1H), 3,90 ppm (s, 3H), 3,40 ppm (s, 3H), 2,16 ppm (s, 3H). |
| 57 | $-CH_3$ | $-CH_2CH_3$ | Smp. 101-103°C |
| 58 | $-CH_3$ | $-C(CH_3)_3$ | Harz; $^1$H-NMR. ($d_6$-DMSO, 200 MHz): 7,58-7,68 ppm (m, 1H), 7,20-7,39 ppm (m, 2H), 6,54 ppm (s, 1H), 3,39 ppm (s, 3H), 2,13 ppm und 2,10 ppm (s, 3H), 1,29 ppm und 1,19 ppm (s, 9H). |
| 59 | $-CH_3$ | $-CH_2CH=CH_2$ | Harz; $^1$H-NMR. ($d_6$-DMSO, 200 MHz): 7,60-7,71 ppm (m, 2H), 7,30-7,41 ppm (m, 2H), 6,54 ppm (s, 1H), 5,90-6,12 ppm (m, 1H), 5,16-5,39 ppm (m, 2H), 4,60-4,68 ppm (m, 2H), 3,39 ppm (s, 3H), 2,19 ppm (s, 3H). |
| 60 | $-CH_3$ | 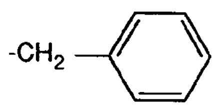 | Oel; $^1$H-NMR. ($d_6$-DMSO, 200 MHz): 7,60-7,70 ppm (m, 1H), 7,22-7,44 ppm (m, 7H), 6,54 ppm und 6,53 ppm (s, 1H), 5,19 ppm und 5,03 ppm (s, 2H), 3,39 ppm (s, 3H), 2,21 ppm und 2,09 ppm (s, 3H). |
| 61 | H | H | Smp. 213-215°C |
| 62 | $-CH_3$ | $-CH_2-CH(CH_3)_2$ | Oel; $^1$H-NMR. ($d_6$-DMSO, 200 MHz): 7,59-7,70 ppm (m, 1H), 7,19-7,40 ppm (m, 2H), 6,52 ppm (s, 1H), 3,90-3,72 ppm (d, 2H), 3,39 ppm (s, 3H), 2,09-2,18 ppm (s, 3H), 1,73-2,06 ppm (m, 1H), 0,92 ppm und 0,80 ppm (d, 6H). |

Tabelle 4: (Fortsetzung)

| Bsp. | $R_6$ | $R_7$ | Phys.-chem. Daten |
|---|---|---|---|
| 63 | $-CH(CH_3)_2$ | $-CH_3$ | Oel; $^1$H-NMR. ($d_6$-DMSO, 200 MHz): 7,59-7,70 ppm (m, 1H), 7,14-7,39 ppm (m, 2H), 6,52 ppm (s, 1H), 3,73 ppm und 3,86 ppm (s, 3H), 3,40 ppm (s, 3H), 2,69 ppm (septett, 1H), 1,08 ppm und 1,02 ppm (d, 6H). |
| 64 | $-CH(CH_3)_2$ | $-CH_2CH_3$ | Oel; $^1$H-NMR. ($d_6$-DMSO, 200 MHz): 7,58-7,67 ppm (m, 1H), 7,14-7,39 ppm (m, 2H), 6,51 ppm (s, 1H), 4,00 ppm und 4,11 ppm (quartett, 2H), 3,40 ppm (s, 3H), 2,69 ppm (septett, 1H), 1,13 ppm und 1,23 ppm (t, 3H), 1,08 ppm und 1,03 ppm (d, 6H). |
| 65 | $-CH(CH_3)_2$ | $-CH_2-CH=CH_2$ | Oel; $^1$H-NMR. ($d_6$-DMSO, 200 MHz): 7,59-7,68 ppm (m, 1H), 7,15-7,39 ppm (m, 2H), 6,51 ppm (s, 1H), 5,76-6,11 ppm (m, 1H), 5,06-5,38 ppm (m, 2H), 4,41-4,64 ppm (m, 2H), 3,39 ppm (s, 3H), 2,70 ppm (septett, 1H), 1,09 ppm und 1,04 ppm (d, 6H). |
| 66 | $-CH(CH_3)_2$ | $-CH_2-CH(CH_3)_2$ | Oel; $^1$H-NMR. ($d_6$-DMSO, 200 MHz): 7,62 ppm (d, 1H), 7,30 ppm (dxd, 1H), 7,16 ppm (d, 1H), 6,51 ppm (s, 1H), 3,39 ppm (s, 3H), 3,73 ppm (d, 2H), 2,68 ppm (septett, 1H), 1,85 ppm (septett, 1H), 1,08 ppm (d, 6H), 0,81 ppm (d, 6H). |
| 67 | $-CH(CH_3)_2$ | $-CH_2$ 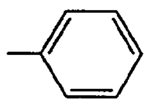 | Oel; $^1$H-NMR. ($d_6$-DMSO, 200 MHz): 7,57-7,70 ppm (m, 1H), 7,14-7,42 ppm (m, 7H), 6,52 ppm (s, 1H), 5,03 ppm und 5,15 ppm (s, 2H) 3,40 ppm (s, 3H), 2,70 ppm (septett, 1H), 1,07 ppm und 1,04 ppm (d, 6H). |

Tabelle 4: (Fortsetzung)

| Bsp. | $R_6$ | $R_7$ | Phys.-chem. Daten |
|---|---|---|---|
| 68 | 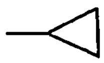 | $-CH_3$ | Oel; $^1$H-NMR. ($CDCl_3$, 200 MHz): 7,45-7,56 ppm (m, 1H), 6,91-7,21 ppm (m, 2H), 6,36 ppm und 6,34 ppm (s, 1H), 3,79 ppm und 3,97 ppm (s, 3H), 3,54 ppm (s, 3H), 1,73-1,92 ppm und 2,37-2,56 ppm (m, 1H), 0,46-0,97 ppm (m, 4H). |
| 69 | 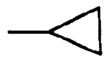 | $-CH_2-CH=CH_2$ | Oel; $^1$H-NMR. ($CDCl_3$, 200 MHz): 7,46-7,55 ppm (m, 1H), 6,92-7,21 ppm (m, 2H), 6,36 ppm und 6,34 ppm (s, 1H), 5,81-6,17 ppm (m, 1H), 5,06-5,42 ppm (m, 2H), 4,44-4,70 ppm (m, 2H), 3,55 ppm (s, 3H), 2,46-2,62 ppm und 1,75-1,93 ppm (m, 1H), 0,48-0,99 ppm (m, 4H). |
| 70 | $-(CH_2)_4-CH_3$ | $-CH_2-CH=CH_2$ | Oel; $^1$H-NMR. ($d_6$-DMSO, 200 MHz): 7,59-7,69 ppm (m, 1H), 7,17-7,40 ppm (m, 2H), 6,52 ppm (s, 1H), 5,78-6,11 ppm (m, 1H), 5,07-5,37 ppm (m, 2H), 4,44-4,65 ppm (m, 2H), 3,40 ppm (s, 3H), 2,68 ppm und 2,42 ppm (t, 2H) 1,12-1,52 ppm (m, 6H), 0,73-0,91 ppm (m, 3H). |
| 71 | $-(CH_2)_2-CH(CH_3)_2$ | $-CH_2-CH=CH_2$ | Oel; $^1$H-NMR. ($CDCl_3$, 200 MHz): 7,47-7,56 ppm (m, 1H), 6,96-7,20 ppm (m, 2H), 6,36 ppm (s, 1H), 5,82-6,14 ppm (m, 1H), 5,06-5,39 ppm (m, 2H), 4,48-4,59 ppm (m, 2H), 3,54 ppm (s, 3H), 2,69-2,81 ppm und 2,45-2,57 ppm (m, 2H), 1,24-1,69 ppm (m, 3H), 0,88 ppm und 0,89 ppm (d, 6H). |
| 72 | $-(CH_2)_4-CH_3$ | 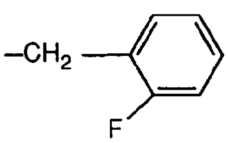 | Oel; $^1$H-NMR. ($CDCl_3$, 200 MHz): 6,90-7,60 ppm (m, 7H), 6,36 ppm und 6,35 ppm (s, 1H), 3,53 ppm (s, 3H), 5,15 ppm und 5,26 ppm (s, 2H) 2,68-2,82 ppm und 2,42-2,54 ppm (m, 2H), 1,16-1,62 ppm (m, 6H), 0,76-0,95 ppm (m, 3H). |

Analog zu Beispiel 55 erhält man ausgehend von 2-Chlor-5-[3,6-dihydro-2,6-dioxo-3-methyl-4-pentafluorethyl-1(2H)-pyrimidinyl]-isobutyrophenon und O-Allyl-hydroxylamin Hydrochlorid O-Allyl-(2-chlor-5-[3,6-dihydro-2,6-dioxo-3-methyl-4-pentafluor-1(2H)-pyrimidinyl]-isobutyrophenon)-oxim als Oel, $^1$H-NMR. ($d_6$-DMSO, 200 MHz): 7,60-7,69 ppm (m, 1H), 7,18-7,38 ppm (m, 2H), 6,40 ppm (s, 1H), 5,77-6,01 ppm (m, 1H), 5,05-5,27 ppm (m, 2H), 4,43-4,64 ppm (m, 2H), 3,40 ppm (s, 3H), 2,70 ppm (septett, 1H), 1,00- 1,15 ppm (m, 6H) (Beispiel 73).

Beispiel 74: 2-Chlor-5-[3,6-dihydro-2,6-dioxo-3-methyl-4-trifluormethyl-1(2H)-pyrimidinyl]-acetophenon-N,N-dimethyl-hydrazon

2,5 g 2-Chlor-5-[3,6-dihydro-2,6-dioxo-3-methyl-4-trifluormethyl-1(2H)-pyrimidinyl]acetophenon und 0,7 g N,N-Dimethylhydrazin werden in 20 ml Ethanol während 24 Stunden am Rückfluss erhitzt und das Reaktionsgemisch anschliessend in 300 ml Wasser eingetragen. Es wird zweimal mit je 300 ml Essigsäureethylester extrahiert und die vereinigten organischen Phasen mit 200 ml gesättigter Natriumchloridlösung gewaschen, über Natriumsulfat getrocknet und am Rotovap eingeengt. Der erhaltene Rückstand wird mittels Kieselgelsäule und Essigsäureethylester/n-Hexan 1/1 chromatographisch rein dargestellt.
$^1$H-NMR. ($CDCl_3$, 200 MHz): 7,44-7,58 ppm (m, 1H), 7,02-7,38 ppm (m, 2H), 6,36 ppm (s, 1H), 3,55 ppm (m,

3H), 2,62 ppm und 2,42 ppm (s, 6H), 2,30 ppm und 2,25 ppm (s, 3H).

Analog zu Beispiel 74 erhält man 2-Chlor-5-[3,6-dihydro-2,6-dioxo-3-methyl-4-trifluormethyl-1(2H)-pyrimidinyl]-benzaldehyd-N,N-dimethylhydrazon; Smp.: 170-171 °C (Beispiel 75).

Beispiel 76: 2-Chlor-5-[3,6-dihydro-2,6-dioxo-3-methyl-4-trifluormethyl-1(2H)pyrimidinyl]-acetophenon ethylenacetal

2,5 g 2-Chlor-5-[3,6-dihydro-2,6-dioxo-3-methyl-4-trifluormethyl-1(2H)-pyrimidinyl]acetophenon, 0,7 g Ethylenglykol und 0,2 g p-Toluolsulfonsäure werden in 60 ml Benzol in einer Dean-Stark-Apparatur während 24 Stunden erhitzt. Nach Beendigung des Wasserabscheidens wird das Reaktionsgemisch auf 100 g Eis gegeben und anschliessend mit 100 ml Essigsäureethylester extrahiert. Die organische Phase wird mit 100 ml gesättigter Natriumchloridlösung gewaschen, über Natriumsulfat getrocknet und eingeengt. Der erhaltene Rückstand wird aus Diethylether/n-Hexan umkristallisiert; Smp.: 121-123°C.

Analog zu Beispiel 76 werden die in der Tabelle 5 zusammengestellten Verbindungen der Formel Ij hergestellt.

Tabelle 5: Verbindungen der Formel Ij

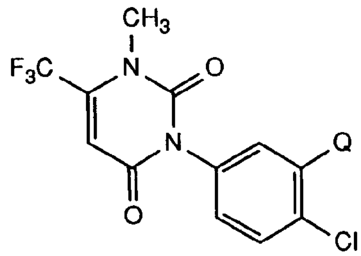

(Ij)

| Beispiel | Q | Phys.-chem. Daten |
|---|---|---|
| 77 | 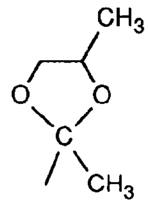 | Smp. 132-135°C |
| 78 | 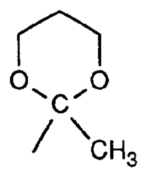 | Smp. 137-139°C |
| 79 | 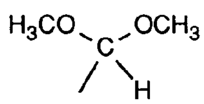 | $^1$H-NMR. ($CDCl_3$, 200 MHz): 7,52 ppm (d, 1H), 7,51 ppm (d, 1H), 7,15 ppm (q, 1H), 6,36 ppm (s, 1H), 5,66 ppm (s, 1H), 3,54 ppm (m, 3H), 3,35 ppm (s, 3H). |
| 80 | 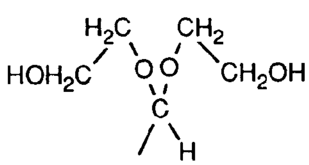 | $^1$H-NMR. ($CDCl_3$, 200 MHz): 7,57 ppm (d, 1H), 7,52 ppm (d, 1H), 7,17 ppm (q, 1H), 6,37 ppm (s, 1H), 5,83 ppm (s, 1H), 3,66-3,82 ppm (m, 8H), 3,55 ppm (m, 3H), 2,50-2,80 ppm (s, 1H), 1,60-2,00 ppm (s, 1H). |
| 81 | 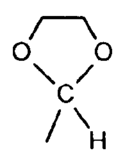 | Smp. 146-148°C |

Beispiel 82: 2-Chlor-5-[3,6-dihydro-2,6-dioxo-3-methyl-4-trifluormethyl-1(2H)-pyrimidinyl]-acetophenon-propylendithioacetal

2,5 g 2-Chlor-5-[3,6-dihydro-2,6-dioxo-3-methyl-4-trifluormethyl-1(2H)-pyrimidinyl]acetophenon und 1,1 g 1,3-Propandithiol werden in 20 ml Chloroform gelöst und anschliessend trockenes Chlorwasserstoffgas bis zur Sättigung eingeleitet. Während 18 Stunden wird das Reaktionsgemisch stehen gelassen und darauf während 1 Stunde auf 40°C erwärmt. Diese Reaktionslösung wird auf 300 ml Eiswasser gegossen und zweimal mit je 100 ml Essigsäureethylester extrahiert. Die vereinigten organischen Phasen werden zweimal mit je 100 ml Natriumhydrogencarbonatlösung und zweimal mit je 100 ml gesättigter Natriumchloridlösung gewaschen, über Natriumsulfat getrocknet und eingeengt. Der Rückstand wird aus Essigsäureethylester/n-Hexan umkristallisiert; Smp.: 186-188°C.

Analog zu Beispiel 82 werden die in der Tabelle 6 zusammengestellten Verbindungen der Formel Ik hergestellt.

Tabelle 6: Verbindungen der Formel Ik

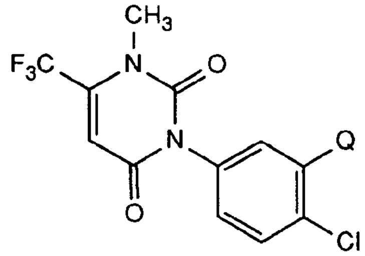

(Ik)

| Beispiel | Q | Phys.-chem. Daten |
|---|---|---|
| 83 | 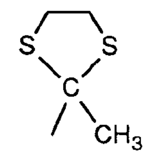 | Smp. 147-149°C |
| 84 | 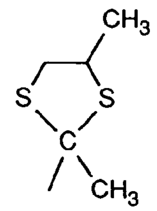 | Harz; $^1$H-NMR. ($d_6$-DMSO, 200 MHz): 7,97-8,04 ppm (m, 1H), 7,57-7,65 ppm (m, 1H), 7,19-7,28 ppm (m, 1H), 6,52 ppm (s, 1H), 3,93-4,14 ppm und 3,65-3,82 ppm (m, 1H), 3,40 ppm (s, 3H), 3,49 ppm und 3,25 ppm und 3,12 ppm und 2,81 ppm (dxd, 2H), 2,20 ppm und 2,17 ppm (s, 3H), 1,40 ppm und 1,28 ppm (d, 3H). |

Beispiel 85: 3-[4-Chlor-3-(3-methylbutyryl)phenyl]-1-methyl-6-trifluormethyl-2,4(1H,3H)-pyrimidindion

Zu 0,44 g einer 55%igen Natriumhydrid-Dispersion in 50 ml absolutem Tetrahydrofuran wird bei 25°C unter Rühren eine Lösung von 2,02 g Isopropylmalonsäure-diethylester in 10 ml Tetrahydrofuran während 5 Minuten zugetropft und 30 Minuten unter Rückfluss gerührt. Nach beendeter Wasserstoffentwicklung wird bei Raumtemperatur unter Rühren während 5 Minuten in inerter Atmosphäre eine Lösung von 3,67 g 2-Chlor-5-[3,6-dihydro-2,6-dioxo-3-methyl-4-trifluormethyl-1(2H)-pyrimidinyl]-benzoesäure-chlorid in 40 ml Tetrahydrofuran zugetropft und zwei Stunden bei 23-30°C nachgerührt. Das Reaktionsgemisch wird am Rotovap zur Trockene eingedampft, der Rückstand in 200 ml Diethylether gelöst und zweimal mit Wasser gewaschen. Die organische Phase wird über Natriumsulfat getrocknet und eingeengt. Die Reindarstellung des erhaltenen Rückstandes erfolgt mittels Kieselgelsäule und Methylenchlorid/Diethylether 39/1 als Eluenten und mittels Umkristallisation aus Diethylether/n-Hexan. Auf diese Weise erhält man 2-Chlor-5-[3,6-dihydro-2,6-dioxo-3-methyl-4-trifluormethyl-1(2H)-pyrimidinyl]-benzoyl-isopropylmalonsäure-diethylester mit Smp.: 105-107°C.

3,40 g dieses Zwischenproduktes werden in 50 ml Essigsäure und 25 ml 48%iger Bromwasserstoffsäure während 48 Stunden am Rückfluss erhitzt. Das Reaktionsgemisch wird anschliessend unter vermindertem Druck weitgehend eingedampft und der Rückstand in 200 ml Diethylether aufgenommen, mit wässriger Natriumhydrogencarbonat-Lösung gewaschen, über Natriumsulfat getrocknet und zur Trockene eingedampft. Der erhaltene Rückstand wird aus Diethylether/n-Hexan umkristallisiert; Smp. 119-120°C.

Beispiel 86: 2-Chlor-5-[3,6-dihydro-2,6-dioxo-3-methyl-4-trifluormethyl-1(2H)-pyrimidinyl]-benzaldehyd-isopropyloxim

Zu einer Suspension von 0,13 g einer 55%igen Natriumhydrid-Dispersion in 50 ml Dimethylformamid wird bei Raumtemperatur unter Rühren 1,00 g 2-Chlor-5-[3,6-dihydro-2,6-dioxo-3-methyl-4-trifluormethyl-1(2H)-pyrimidinyl]-benzaldehydoxim zugefügt und 2 Stunden bis zur Beendigung der Wasserstoffentwicklung nachgerührt. Die gelbe Lösung wird mit 0,75 g Isopropyljodid versetzt, 1 Stunde nachgerührt und das Reaktionsgemisch anschliessend auf eine Lösung von 300 ml Wasser und 20 ml 2N Salzsäure gegossen und zweimal

mit 50 ml Essigsäureethylester extrahiert. Die organische Phase wird mit Wasser neutral gewaschen, über Natriumsulfat getrocknet und eingedampft. Die Reindarstellung des Rückstandes erfolgt mittels Kieselgelsäule und n-Hexan/Essigsäureethylester 2/1 als Eluenten und durch Umkristallisation aus n-Hexan; Smp.: 105-107°C.

B. Herstellung der Zwischenprodukte der Formeln IIb, IV, XVI, XVII, XXIV, XXIX und XXX:

Beispiel 87: 2-Chlorphenyl-n-pentylketon

Zu einer Lösung aus 68,8 g o-Chlorbenzonitril in 400 ml Diethylether wird bei Raumtemperatur eine zuvor hergestellte Grignardlösung aus 36,5 g Magnesium und 157 ml 1-Brompentan in 600 ml Diethylether während 2 Stunden zugetropft. Nach beendeter Zugabe wird das Reaktionsgemisch während 3 Stunden rückflussiert und anschliessend mit einem Eisbad auf 10°C gekühlt. 800 ml halbkonzentrierte Salzsäure werden zugetropft und das erhaltene Gemisch während 36 Stunden bei Raumtemperatur stehengelassen. Die wässrige Phase wird abgetrennt und mit 400 ml Diethylether extrahiert. Die vereinigten organischen Phasen werden mit 300 ml 2N Salzsäurelösung und zweimal mit je 300 ml gesättigter Kochsalzlösung gewaschen, über Magnesiumsulfat getrocknet und eingedampft. Der Rückstand wird im Hochvakuum fraktioniert destilliert; das gewünschte Produkt erhält man als farbloses Oel mit Sdp. 98-101°C/0,8 Torr; $n_D^{23}$ 1,5163.

Analog zu Beispiel 87 werden die in der Tabelle 7 aufgeführten Verbindungen der Formel XXX hergestellt.

Tabelle 7: Verbindungen der Formel XXX

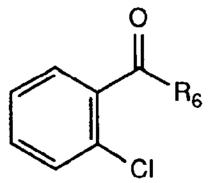

(XXX)

| Beispiel | $R_6$ | Phys.-chem. Daten |
|---|---|---|
| 88 | $-CH_2CH_3$ | Sdp. 74-76°C/0,25 Torr; $n_D^{23}$ 1,5340 |
| 89 | $-CH(CH_3)_2$ | Sdp. 81-83°C/0,2 Torr; $n_D^{23}$ 1,5228 |
| 90 | $-(CH_2)_3CH_3$ | Sdp. 84-87°C/0,07 Torr; $n_D^{23}$ 1,5204 |
| 91 | $-CH_2CH_2CH(CH_3)_2$ | Sdp. 106-108°C/0,1 Torr; $n_D^{23}$ 1,5161 |
| 92 | 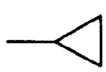 | Sdp. 89-94°C/0,14 Torr; $n_D^{23}$ 1,5573 |
| 93 | 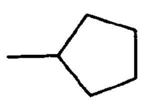 | $n_D^{23}$ 1,5475 |
| 94 | 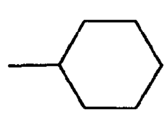 | Sdp. 124-128°C/0,15 Torr; $n_D^{23}$ 1,5452 |
| 95 | $-CH_2CH_2CH_3$ | Sdp. 89-90°C/0,16 Torr; $n_D^{23}$ 1,5267 |

Auf analoge Weise wird durch Reaktion von 2-Chlor-4-fluor-benzonitril mit Ethylmagnesiumbromid 2-Chlor-4-fluor-propiophenon als Oel erhalten; Sdp. 44°C/0,07 Torr (Beispiel 96).

Beispiel 97: 2-Chlor-5-nitro-propiophenon

Zu 31 ml vorgelegter 100%iger Salpetersäure (d 1,52) werden bei -10°C 6 g o-Chlorpropiophenon während 15 Minuten so zugetropft, dass die Reaktionstemperatur zwischen -5°C und -10°C liegt. Zur Vervollständigung der Reaktion wird bei 0°C noch während 45 Minuten nachgerührt. Anschliessend wird das Reaktionsgemisch auf 300 g Eis gegossen und die wässrige Mischung zweimal mit je 200 ml Diethylether extrahiert. Die organische Phase wird mit 100 ml Wasser, zweimal mit je 100 ml 2N Kaliumhydrogencarbonat-Lösung und zum Schluss mit gesättigter Kochsalzlösung gewaschen, über Magnesiumsulfat getrocknet und eingeengt. Der Rückstand wird aus Diisopropylether/n-Hexan umkristallisiert; Smp.: 55-56°C.

Analog zu Beispiel 97 werden die in der Tabelle 8 aufgeführten Verbindungen der Formel XXIX hergestellt.

Tabelle 8: Verbindungen der Formel XXIX

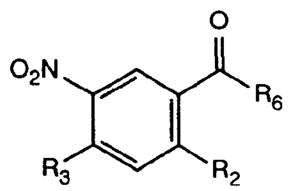

(XXIX)

| Bsp. | $R_6$ | $R_2$ | $R_3$ | Phys.-chem. Daten |
|---|---|---|---|---|
| 98 | $-CH(CH_3)_2$ | Cl | H | Oel; $^1$H-NMR. ($CDCl_3$, 200 MHz): 8,22-8,28 ppm (m, 2H), 7,61-7,69 ppm (m, 1H), 3,36 ppm (m, 1H), 1,24 ppm (d, 6H). |
| 99 | $-(CH_2)_3CH_3$ | Cl | H | Oel; $^1$H-NMR. ($d_6$-DMSO, 200 MHz): 8,49 ppm (d, 1H), 8,32 ppm (dxd, 1H), 7,84 ppm (d, 1H), 3,01 ppm (t, 2H), 1,54-1,70 ppm (m, 2H), 1,26-1,47 ppm (m, 2H), 0,92 ppm (t, 3H). |
| 100 | $-(CH_2)_4CH_3$ | Cl | H | Smp. 38-40°C |
| 101 | $-CH_2CH_2CH(CH_3)_2$ | Cl | H | Smp. 37-39°C |
| 102 | 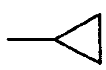 | Cl | H | Oel; $^1$H-NMR. ($CDCl_3$, 200 MHz): 8,38 ppm (d, 1H), 8,24 ppm (dxd, 1H), 7,64 ppm (d, 1H), 2,41-2,55 ppm (m, 1H), 1,15-1,46 ppm (m, 4H). |
| 103 | 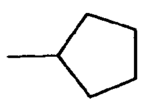 | Cl | H | Smp. 61-64°C |
| 104 | 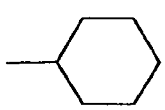 | Cl | H | Smp. 66-67°C |
| 105 | $-CH_2CH_2CH_3$ | Cl | H | Oel; $^1$H-NMR. ($d_6$-DMSO, 200 MHz): 8,49 ppm (d, 1H), 8,32 ppm (dxd, 1H), 7,85 ppm (d, 1H), 2,99 ppm (t, 2H), 1,64 ppm (sextett, 2H), 0,96 ppm (t, 3H). |

Tabelle 8: (Fortsetzung)

| Bsp. | $R_6$ | $R_2$ | $R_3$ | Phys.-chem. Daten |
|---|---|---|---|---|
| 106 | $-CH_3$ | F | H | Smp. 55-57°C |
| 107 | $-CH_3$ | Br | H | Smp. 154-156°C |
| 108 | $-CH_2CH_3$ | Cl | F | Oel; $^1$H-NMR. ($d_6$-DMSO, 200 MHz): 8,53 ppm (d, 1H), 8,03 ppm (d, 1H), 3,00 ppm (quartett, 2H), 1,09 ppm (t, 3H). |

Beispiel 109: 5-Amino-2-chloracetophenon

Zum Gemisch aus 5,6 g Eisenpulver, 40 ml Ethanol, 9 ml Wasser und 1 ml konzentrierter Salzsäure werden unter Rühren bei 65°C 5 g festes 2-Chlor-5-nitroacetophenon (siehe J. Am. Chem. Soc. 76, 5482 (1954)) portionenweise eingetragen. Das Reaktionsgemisch wird während 1 Stunde bei 70°C nachgerührt. Nach dem Abkühlen auf Raumtemperatur wird mit 300 ml Wasser verdünnt und durch Zugabe von 2N Kaliumcarbonatlösung der pH-Wert auf 9 eingestellt. Es wird über Celite abgenutscht und das Filtergut mit 200 ml Essigsäureethylester nachgewaschen. Das Filtrat wird in einen Scheidetrichter transferiert und die Wasserphase verworfen. Die organische Phase wird mit gesättigter Natriumchlorid-Lösung gewaschen, über Natriumsulfat getrocknet und eingeengt.

$^1$H-NMR. ($CDCl_3$, 200 MHz): 7,15 ppm (d, 1H), 6,82 ppm (d, 1H), 6,58 ppm (dxd, 1H), 3,82 ppm (s, 2H), 2,62 ppm (s, 3H).

Analog zu Beispiel 109 werden die in der Tabelle 9 aufgeführten Verbindungen der Formel XXIV hergestellt.

Tabelle 9: Verbindungen der Formel XXIV

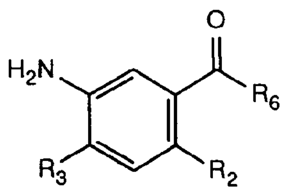

(XXIV)

| Bsp. | $R_6$ | $R_2$ | $R_3$ | Phys.-chem. Daten |
|---|---|---|---|---|
| 110 | $-CH(CH_3)_2$ | Cl | H | Oel; $^1$H-NMR. ($d_6$-DMSO, 200 MHz): 7,07-7,16 ppm (m, 1H), 6,58-6,68 ppm (m, 2H), 5,45 ppm(s, 2H), 3,20 ppm (m, 1H), 1,08 ppm (d, 6H). |
| 111 | $-(CH_2)_3CH_3$ | Cl | H | Oel; $^1$H-NMR. ($d_6$-DMSO, 200 MHz): 7,10 ppm (d, 1H), 6,69 ppm (d, 1H), 6,65 ppm (dxd, 1H), 5,47 ppm (s, 2H), 2,83 ppm (t, 2H), 1,48-1,65 ppm (m, 2H), 1,21-1,42 ppm (m, 2H), 0,89 ppm (t, 3H). |
| 112 | $-(CH_2)_4CH_3$ | Cl | H | Oel; $^1$H-NMR. ($CDCl_3$, 200 MHz): 7,12 ppm (d, 1H), 6,69 ppm (d, 1H), 6,65 ppm (dxd, 1H), 3,67 ppm (s, 2H), 2,89 ppm (t, 2H), 1,50-1,80 ppm (m, 2H), 1,22-1,45 ppm (m, 4H), 0,82-0,98 ppm (m, 3H). |
| 113 | $-CH_2CH_2CH(CH_3)_2$ | Cl | H | Oel; $^1$H-NMR. ($CDCl_3$, 200 MHz), 7,07-1,17 ppm (m, 1H), 6,60-6,70 ppm (m, 2H), 3,83 ppm (s, 2H), 2,83-2,97 ppm (m, 2H), 1,50-1,70 ppm (m, 3H), 0,84-0,94 ppm (m, 6H). |
| 114 | 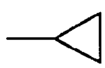 | Cl | H | Oel |
| 115 | 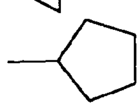 | Cl | H | Oel |
| 116 | 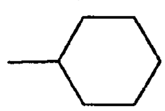 | Cl | H | Oel |
| 117 | $-CH_2CH_2CH_3$ | Cl | H | Oel; $^1$H-NMR. ($d_6$-DMSO, 200 MHz): 7,11 ppm (d, 1H), 6,61-6,74 ppm (m, 2H), 5,46 ppm (s, 2H), 2,82 ppm (t, 2H), 1,61 ppm (sextett, 2H), 0,91 ppm (t, 3H). |

Tabelle 9: (Fortsetzung)

| Bsp. | $R_6$ | $R_2$ | $R_3$ | Phys.-chem. Daten |
|---|---|---|---|---|
| 118 | $-CH_3$ | F | H | Smp. 76-78°C |
| 119 | $-CH_3$ | Br | H | Oel |
| 120 | $-CH_2CH_3$ | Cl | F | Oel; $^1$H-NMR. ($d_6$-DMSO, 200 MHz): 7,24 ppm (d, 1H), 6,99 ppm (d, 1H), 5,54 ppm (s, 2H), 2,86 ppm (quartett, 2H), 1,06 ppm (t, 3H). |

Beispiel 121: 3-Acetyl-4-chlor-carbanilsäure-ethylester

Zu 34 g vorgelegtem, rohem 5-Amino-2-chloracetophenon in 200 ml Pyridin werden bei 0°C 27 g Chlorameisensäureethylester zugetropft und anschliessend während 2 Stunden bei Raumtemperatur nachgerührt. Das Reaktionsgemisch wird auf 1,0 kg zerstossenes Eis gegeben und dann zweimal mit je 300 ml Essigsäureethylester extrahiert. Die organische Phase wird fünfmal mit je 300 ml 2N Salzsäure und zweimal mit je 300 ml gesättigter Natriumchlorid-Lösung nachgewaschen, über Natriumsulfat getrocknet und eingedampft. Der Rückstand wird aus Ether/n-Hexan umkristallisiert; Smp. 77-78°C.

Analog zu Beispiel 121 werden die in der Tabelle 10 aufgeführten Verbindungen der Formel XVII hergestellt.

Tabelle 10: Verbindungen der Formel XVII

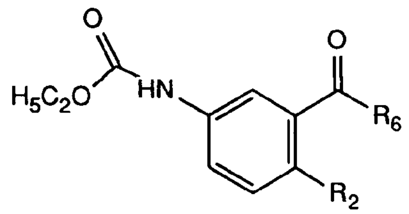

(XVII)

| Bsp. | $R_6$ | $R_2$ | Phys.-chem. Daten |
|---|---|---|---|
| 122 | $-CH(CH_3)_2$ | Cl | Smp. 73-75°C |
| 123 | $-(CH_2)_3CH_3$ | Cl | Oel; $^1$H-NMR. ($d_6$-DMSO, 200 MHz): 9,94 ppm (s, 1H), 7,72 ppm (d, 1H), 7,58 ppm (dxd, 1H), 7,43 ppm (d, 1H), 4,16 ppm (q, 2H), 2,89 ppm (t, 2H), 1,50-1,68 ppm (m, 2H), 1,20-1,46 ppm (m, 5H), 0,89 ppm (t, 3H). |
| 124 | $-(CH_2)_4CH_3$ | Cl | Smp. 32-34°C |
| 125 | $-CH_2CH_2CH(CH_3)_2$ | Cl | Smp. 49-50°C |
| 126 | 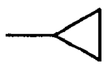 | Cl | Smp. 98-100°C |
| 127 | $-CH_2CH_3$ | Cl | Smp. 58-60°C |
| 128 | 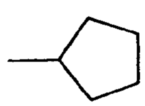 | Cl | Oel; $^1$H-NMR. ($d_6$-DMSO, 200 MHz): 9,94 ppm (s, 1H), 7,72 ppm (d, 1H), 7,56 ppm (dxd, 1H), 7,43 ppm (d, 1H), 4,16 ppm (q, 2H), 3,53 ppm (quintett, 1H), 1,51-1,93 ppm (m, 8H), 1,27 ppm (t, 3H). |
| 129 | 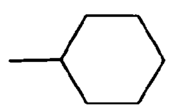 | Cl | Smp. 94-96°C |
| 130 | $-CH_2CH_2CH_3$ | Cl | Oel; $^1$H-NMR. ($d_6$-DMSO, 200 MHz): 9,92 ppm (s, 1H), 7,71 ppm (d, 1H), 7,56 ppm (dxd, 1H), 7,43 ppm (d, 1H), 4,16 ppm (quartett, 2H), 2,86 ppm (t, 2H), 1,63 ppm (sextett, 2H), 1,27 ppm (t, 3H), 0,93 ppm (t, 3H). |
| 131 | $-CH_3$ | F | Smp. 166-118°C |
| 132 | $-CH_3$ | Br | Smp. 60-62°C |

Beispiel 133: 3-Acetyl-4-chlor-6-fluorphenylisocyanat

Zu 100 ml einer 2M Phosgenlösung in Toluol werden bei Raumtemperatur 18,7 g 2-Chlor-4-fluor-5-aminoacetophenon (siehe EP-A-0,207,884) in 100 ml Essigsäureethylester zugetropft und während 2 Stunden gerührt. Ueberschüssiges Phosgen und Lösungsmittel werden abdestilliert. Aus dem Rückstand wird beim Sdp. 170°C/0,2 Torr das gewünschte Produkt erhalten.

Beispiel 134: Ethyl-1-[[[4-chlor-6-fluor-3-acetophenyl]carbamoyl]methylen-ethyl]carbamat

Zu 21,0 g 2-Chlor-4-fluor-5-amino-acetophenon und 10,4 g Diketen in 100 ml Benzol werden bei Raumtemperatur 0,28 g 4-Dimethylaminopyridin zugegeben, wobei die Temperatur auf +77°C ansteigt. Nach 30 Minuten werden 22,5 g Urethan und 1,0 g p-Toluolsulfonsäure zugegeben und diese Reaktionslösung bei Rückflusstemperatur während 5 Stunden in der Dean-Stark-Apparatur erhitzt. Anschliessend wird das Gemisch auf 400 ml Wasser gegossen und mit Essigsäureethylester extrahiert. Die organische Phase wird neutral gewaschen und eingedampft; dabei kristallisiert das Produkt aus. $^1$H-NMR. ($CDCl_3$, 60 MHz): 9,90 ppm (s, 1H), 8,30 ppm (d, 1H), 7,51 ppm (d, 1H), 5,31 ppm (s, 1H), 3,30 ppm (s, 3H), 2,20 ppm (s, 3H), 1,15 ppm (t, 3H).

Beispiel 135: 5-(Acetoacetylamino)-2-chlor-4-fluorpropiophenon

19,5 g 2,2-Dimethyl-1,3-dioxan-4,6-dion (Meldrum's Säure) und 21,5 ml Pyridin werden in 100 ml Dichlormethan bei 0°C vorgelegt. Dazu werden während 1 Stunde 12,5 ml Acetylchlorid zugetropft und während 1 Stunde bei Raumtemperatur nachgerührt. Nach dem Eintragen des Reaktionsgemisches in 200 ml eiskalte 2N Salzsäure wird zweimal mit je 400 ml Diethylether extrahiert. Die organischen Phasen werden zweimal mit je 200 ml gesättigter Natriumchloridlösung gewaschen und über Natriumsulfat getrocknet und eingedampft. Der erhaltene Rückstand wird zusammen mit 27,3 g 5-Amino-2-chlor-4-fluorpropiophenon in 200 ml Toluol während 4 Stunden am Rückfluss erhitzt. Nach dem Abkühlen wird das Reaktionsgemisch mit 300 ml 2N Salzsäure versetzt und zweimal mit je 300 ml Diethylether extrahiert. Die organischen Phasen werden zweimal mit je 200 ml gesättigter Natriumchloridlösung gewaschen, über Natriumsulfat getrocknet und eingeengt. Der Rückstand wird mittels Kieselgelsäule und Essigsäureethylester/n-Hexan 2/3 als Eluenten gereinigt. Das erwünschte Produkt ist ein Oel; $^1$H-NMR. ($d_6$-DMSO, 200 MHz): 10,17 ppm (s, 1H), 8,30 ppm (d, 1H), 7,63 ppm (d, 1H), 3,68 ppm (s, 2H), 2,92 ppm (quartett, 2H), 2,22 ppm (s, 3H), 1,08 ppm (t, 3H).

Beispiel 136: 2-Chlor-5-[3,6-dihydro-2,6-dioxo-3-methyl-4-trifluormethyl-1(2H)-pyrimidinyl]-benzylalkohol

Zu einer Lösung von 2,0 g 2-Chlor-5-[3,6-dihydro-2,6-dioxo-3-methyl-4-trifluormethyl-1(2H)-pyrimidinyl]-benzoesäure in 40 ml trockenem Tetrahydrofuran wird unter Rühren bei 0°C unter Stickstoff 4 ml einer 0,5 molaren Lösung von Diboran in Tetrahydrofuran zugegeben und 30 Minuten bei 0°-25°C nachgerührt. Anschliessend wird das Reaktionsgemisch noch zweimal bei 25°C mit 4 ml der 0,5 molaren Diboranlösung versetzt, wobei das erste Mal 40 Minuten, das zweite Mal 1,5 Stunden nachgerührt wird. Darauf wird das Reaktionsgemisch mit 20 ml 2N Salzsäure versetzt und 5 Minuten nachgerührt. Dann wird auf 500 ml Wasser gegossen und zweimal mit je 100 ml Diethylether extrahiert. Die organische Phase wird mit Wasser gewaschen und über Natriumsulfat getrocknet. Unter vermindertem Druck wird das Lösungsmittel abgedampft und der erhaltene, feste Rückstand über eine Kieselgelsäule mit Essigsäureethylester/n-Hexan 1/1 als Laufmittel rein dargestellt; Smp.: 192-194°C.

Auf analoge Weise erhält man aus 2-Chlor-5-[3,6-dihydro-2,6-dioxo-3-methyl-4-trifluormethyl-1(2H)-pyrimidinyl]-4-fluorbenzoesäure den 2-Chlor-5-[3,6-dihydro-2,6-dioxo-3-methyl-4-trifluormethyl-1(2H)-pyrimidinyl] -4-fluor-benzylalkohol; Smp.: 114-116°C (Beispiel 137).

C. Formulierungsbeispiele

Beispiel F1: Zur Herstellung eines 25%igen Spritzpulvers werden die nachstehend aufgeführten Bestandteile miteinander vermischt:

| | Gewichtsprozent |
|---|---|
| Verbindung der Formel I (Wirkstoff) | 25 |
| Hydratisierte Kieselsäure (Trägerstoff, Mahlhilfsmittel) | 20 |
| Natrium-laurylsulfat (Netzmittel) | 2 |
| Natrium-lignosulfonat (Dispergiermittel) | 4 |
| Kaolin (Trägerstoff) | 49 |
| | 100 |

Es wird zuerst der flüssige bzw. geschmolzene Wirkstoff in einer Mahleinrichtung auf die vorgelegte Kieselsäure aufgetragen. Anschließend werden die weiteren Bestandteile zugemischt. Das Gemisch wird unter Verwendung einer Stiftmühle oder einer vergleichbaren Mahleinrichtung fein gemahlen.

Das resultierende Spritzpulver ergibt beim Einrühren in Wasser eine feine Suspension, die sich als gebrauchsfertige Spritzbrühe eignet.

Als Wirkstoff für diese Formulierung eignen sich inbesondere Verbindungen der Formel I, die flüssig sind oder einen niedrigen Schmelzpunkt, d.h. bis etwa +100°C, aufweisen.

Beispiel F2: Verbindungen der Formel I mit hohem Schmelzpunkt. d.h. ab etwa +100°C, können vorzugsweise als Wirkstoffe in konzentrierten Spritzpulvern verwendet werden, beispielsweise wie folgt:

| | Gewichtsprozent |
|---|---|
| Verbindung der Formel I (Wirkstoff) | 75 |
| Hydratisierte Kieselsäure (Trägerstoff, Mahlhilfsmittel) | 1 |
| Alkylnaphthalensulfonat und Alkylcarboxylatsulfat als Natriumsalze, beispielsweise "Morowett EFW" (Handelsname der De Soto) (Netzmittel) | 2 |
| Sulfoniertes Naphthalen-Formaldehyd-Kondensat als Natriumsalz, beispielsweise "Morowett D-425" (Handelsname der De Soto) (Dispergiermittel) | 10 |
| Polyvinylpyrrolidon, beispielsweise PVP-K-30 (GAF Corp.) (Bindemittel) | 1 |
| Kaolin (Trägerstoff) | 11 |
| | 100 |

Die Bestandteile werden miteinander gemischt und unter Verwendung einer Stiftmühle oder einer vergleichbaren Mahleinrichtung, insbesondere einer Strahlmühle, fein gemahlen.

Beim Einrühren in Wasser ergibt das resultierende Spritzpulver eine feine Suspension beliebiger Konzentration, die sich als gebrauchsfertige Spritzbrühe eignet.

Beispiel F3: Ein Spritzpulver auf der Basis der Zusammensetzung des Beispiels 49 kann auch in ein dispergierbares Granulat übergeführt werden. Dazu wird das gemahlene Pulver in einer geeigneten Granuliervorrichtung (beispielsweise Granulierteller, Mischtrommel, Intensivmischer oder Wirbelschichtgranulator) mit

einer wäßrigen Lösung des Bindemittels besprüht, bis sich Agglomerate gebildet haben. Danach wird das zugefügte Wasser in einem Trocknungsprozeß wieder entfernt. Die Granulate der gewünschten Größe werden ausgesiebt.

Das resultierende Granulat hat gegenüber dem Spritzpulver verschiedene Vorteile (keine Staubbildung bei der Applikation, leichtere Dosierbarkeit infolge besserer Fließeigenschaften). Die Applikation erfolgt nach Einrühren des Präparates in Wasser und nach vollständigem Zerfall der Granulate in die Primärpartikel genau gleich wie beim Spritzpulver.

Beispiel F4: Die Verbindungen der Formel I sind in den üblichen organischen Lösungsmitteln beschränkt löslich. Demzufolge sind emulgierbare Konzentrate von verhältnismäßig niedriger Konzentration möglich; beispielsweise:

| | |
|---|---|
| Verbindung der Formel I (Wirkstoff) | 125 g/l |
| "Sorprophor BSU" (Handelsname der Rhône-Poulenc) (Emulgator) | 300 g/l |
| N-Methyl-2-pyrrolidon (Lösungsmittel) | ad 1000 ml |

Der Wirkstoff und der Emulgator werden unter Rühren ins Lösungsmittel eingetragen Das Gemisch wird gerührt, bis eine homogene Lösung entstanden ist.

Das resultierende emulgierbare Konzentrat läßt sich in Wasser emulgieren und ergibt dabei eine gebrauchsfertige Spritzbrühe der gewünschten Konzentration.

Beispiel F5: Verbindungen der Formel I mit einem Schmelzpunkt ab etwa +60°C können auch als sogenannte Suspensionskonzentrate ("Flowables") formuliert werden; Beispielsweise:

| | |
|---|---|
| Verbindung der Formel I (Wirkstoff) | 250 g/l |
| Ethylenglykol (Frostschutz) | 80 g/l |
| Kieselsäure (Antiabsetzmittel) | 5 g/l |
| Xanthan gum., beispielsweise "Kelzan" (Handelsname der Kelco) (Verdickungsmittel) | 2 g/l |
| Silicon-Entschäumer, beispielsweise "Rhodorsil 426" (Handelsname der Rhône-Poulenc) | 5 g/l |
| Nonylphenol-polyethoxylat (Netzmittel) | 20 g/l |
| Sulfoniertes Naphthalen-Formaldehyd-Kondensat als Natriumsalz, beispielsweise "Morowett D-425" (Handelsname der De Soto) (Dispergiermittel) | 40 g/l |
| Wasser | ad 1000 ml |

Die Formulierungshilfsstoffe werden in Wasser gelöst. In der Lösung wird der vorgemahlene Wirkstoff unter Rühren dispergiert. Die resultierende grobe Suspension wird nun einer Naßmahlung unterzogen (beispielsweise in einer Kolloid-Mühle oder einer Rührwerkkugelmühle). Gegebenenfalls sind nun noch weitere Zusätze in kleinen Mengen möglich, wie Antischaummittel, Antiabsetzmittel und Biozide.

Das resultierende "Flowable" läßt sich zur Anwendung mit Wasser beliebig verdünnen und ergibt dabei eine gebrauchsfertige Spritzbrühe der gewünschten Konzentration.

D. Biologische Beispiele

Beispiel B1: Pre-emergente Herbizid-Wirkung

Im Gewächshaus wird unmittelbar nach der Einsaat der Versuchspflanzen in Saatschalen die Erdobefläche mit einer wässrigen Spritzbrühe entsprechend einer Aufwandmenge von 0,3 kg Wirksubstanz/Hektar behandelt. Die Saatschalen werden im Gewächshaus bei 22-25°C und 50-70 % relativer Luftfeuchtigkeit gehalten.

Nach 4 Wochen wird die Herbizidwirkung mit einem mehrstufigen (10 = 100 % Schädigung, 5 = 50 % Schädigung, 0 = keine Schädigung) Boniturschema im Vergleich mit einer unbehandelten Kontrollgruppe bewertet.

In diesem Versuch zeigen die Verbindungen der Formel I gemäß den Beispielen 53, 56, 81 und 85 starke Herbizidwirkung.

Beispiele für die gute herbizide Wirksamkeit der Verbindungen der Formel I gibt die Tabelle B1:

Tabelle B1: Preemergente Herbizid-Wirkung

| Verb. gemäss Beispiel | Unkraut: Sorghum | Echinochloa | Digitaria | Bromus | Chenopodium | Amaranthus | Stellaria | Sinapis | Galium |
|---|---|---|---|---|---|---|---|---|---|
| 53 | 10 | 9 | 10 | 10 | 10 | 10 | 10 | 10 | 10 |
| 56 | 9 | 10 | 10 | 7 | 10 | 10 | 10 | 10 | 10 |
| 81 | 10 | 10 | 10 | 10 | 10 | 10 | 10 | 10 | 10 |
| 85 | 10 | 10 | 10 | 10 | 10 | 10 | 10 | 10 | 10 |

Beispiel B2: Postemergente Herbizid-Wirkung (Kontaktherbizid)

Eine Anzahl Unkräuter, sowohl monokotyle wie dikotyle, wurden nach dem Auflaufen (im 1- bis 2-Blattstadium) mit einer wässrigen Wirkstoffdispersion in einer Dosierung von 1 kg Wirksubstanz pro Hektar gespritzt und diese bei 24°-26°C und 45-60 % relativer Luftfeuchtigkeit gehalten. 21 Tage nach der Behandlung wird der Versuch mit einem mehrstufigen Bonitiersystem (10 = 100 % Schädigung, 0 = keine Schädigung) ausgewertet. Auch in diesem Versuch zeigen die Verbindungen der Formel I gemäß den Beispielen 56, 81 und 85 gute Herbizidwirkung. Beispiele für die gute herbizide Wirksamkeit der Verbindungen der Formel I gibt die Tabelle B2:

Tabelle B2: Postemergente Herbizid-Wirkung

| Verb. gemäss Beispiel | Unkraut: Chenopodium | Amaranthus | Stellaria | Sinapis | Galium |
|---|---|---|---|---|---|
| 56 | 10 | 9 | 6 | 10 | 10 |
| 81 | 10 | 10 | 10 | 10 | 10 |
| 85 | 10 | 10 | 10 | 10 | 10 |

Beispiel B3: Herbizidwirkung für Wasserreis (paddy)

Das Wasserunkraut Echinochloa crus-galli wird in Plastikbechern (60 $cm^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat durch eine Spritzung der Prüfsubstanzen auf die Gefäße. Die verwendete Dosis entspricht einer Wirkstoffmenge von 3 kg AS pro Hektar. Die Pflanzenbecher werden dann im Gewächshaus unter oprimalen Wachstumsbedingungen für die Reisunkräuter aufgestellt, d.h. bei 25°-30°C und hoher Luftfeuchtigkeit.

Die Auswertung der Versuche findet 3 Wochen nach Applikation statt. Die Verbindungen der Formel I gemäß den Beispielen 53, 56, 81 und 85 schädigen dabei die Unkräuter.

## Patentansprüche

1. 3-Aryluracil-Derivate, dadurch gekennzeichnet, dass diese der Formel I

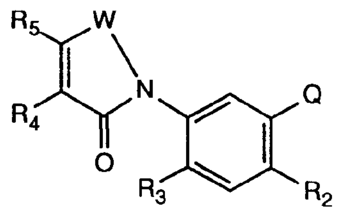

(I),

entsprechen, worin
-W- die Gruppe

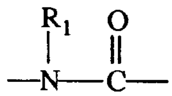 oder 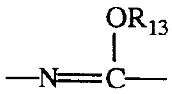

bedeutet, wobei die Bindung an das Ring-Stickstoffatom über das C-Atom erfolgt;

$R_1$ Wasserstoff, $C_1$-$C_4$-Alkyl, $C_1$-$C_4$-Haloalkyl, $C_3$- oder $C_4$-Alkenyl oder $C_3$- oder $C_4$-Alkinyl;
$R_2$ Halogen oder Cyano;
$R_3$ Wasserstoff oder Halogen;
$R_4$ Wasserstoff, Halogen oder $C_1$-$C_4$-Alkyl;
$R_5$ $C_1$-$C_4$-Alkyl oder $C_1$-$C_4$-Haloalkyl ist; oder $R_4$ und $R_5$ zusammen $-(CH_2)_n-$ sind;
n die Zahl 3 oder 4;
$R_{13}$ $C_1$-$C_4$-Alkyl, $C_3$- oder $C_4$-Alkenyl oder $C_3$- oder $C_4$-Alkinyl ist; und
Q eine der Gruppen a) bis e) bedeutet:

a)

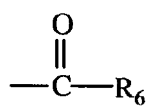 (1),

wobei $R_5$ Wasserstoff, $C_1$-$C_8$-Alkyl, $C_3$-$C_7$-Cycloalkyl oder $C_3$-$C_7$-Cycloalkyl-$C_1$-$C_4$-alkyl;

b)

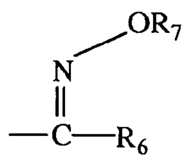 (2),

wobei $R_5$ die unter a) angegebene Bedeutung hat; und

$R_7$ Wasserstoff, $C_1$-$C_8$-Alkyl, $C_2$-$C_8$-Alkenyl, $C_2$-$C_8$-Alkinyl, $C_3$-$C_7$-Cycloalkyl, Phenyl, mit Halogen, $C_1$-$C_4$-Alkyl oder $C_1$-$C_4$-Alkoxy substituiertes Phenyl, Benzyl oder mit Halogen, $C_1$-$C_4$-Alkyl oder $C_1$-$C_4$-Alkoxy substituiertes Benzyl;

c)

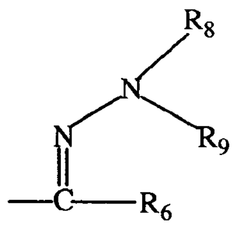 (3),

wobei $R_6$ die unter a) angegebene Bedeutung hat;
$R_6$ und $R_9$, unabhängig voneinander, Wasserstoff, $C_1$-$C_8$-Alkyl, $C_2$-$C_8$-Alkenyl, $C_2$-$C_8$-Alkinyl, Phenyl, mit Halogen, $C_1$-$C_4$-Alkyl oder $C_1$-$C_4$-Alkoxy substituiertes Phenyl, Benzyl, mit Halogen, $C_1$-$C_4$-Alkyl oder $C_1$-$C_4$-Alkoxy substituiertes Benzyl; oder $R_8$ und $R_9$ zusammen $-(CH_2)_m-$ sind; und
m die Zahl 3, 4 oder 5 ist;
d)

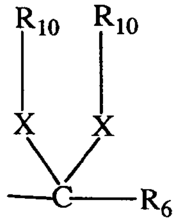 (4),

wobei $R_6$ die unter a) angegebene Bedeutung hat;
$R_{10}$ $C_1$-$C_8$-Alkyl, $C_2$-$C_8$-Alkenyl, $C_2$-$C_8$-Alkinyl, $C_1$-$C_4$-Alkoxy-$C_1$-$C_6$-alkyl, Hydroxy-$C_1$-$C_6$-alkyl oder $C_1$-$C_8$-Haloalkyl; und
X Sauerstoff oder Schwefel;
e)

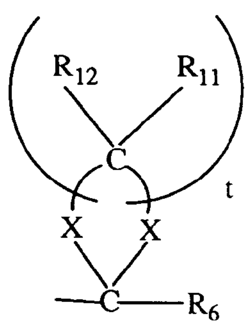 (5),

wobei $R_6$ die unter a) angegebene Bedeutung hat;
$R_{11}$ und $R_{12}$, unabhängig voneinander, Wasserstoff oder $C_1$-$C_4$-Alkyl sind;
t die Zahl 2, 3 oder 4; und
X Sauerstoff oder Schwefel bedeutet; und sofern $R_1$ Wasserstoff ist, auch die agrochemisch verträglichen Salze von Verbindungen der Formel I.

**2.** 3-Aryluracil-Derivate gemäss Anspruch 1, dadurch gekennzeichnet, dass diese der Formel Ia

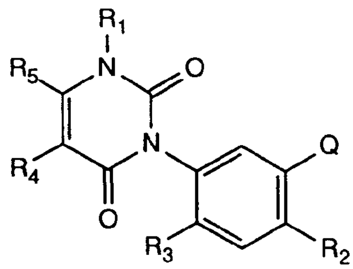

(Ia),

entsprechen, worin $R_1$, $R_2$, $R_3$, $R_4$, $R_5$ und Q die in Anspruch 1 angegebenen Bedeutungen besitzen.

**3.** 3-Aryluracil-Derivate gemäss Anspruch 1, dadurch gekennzeichnet, dass diese der Formel Ib

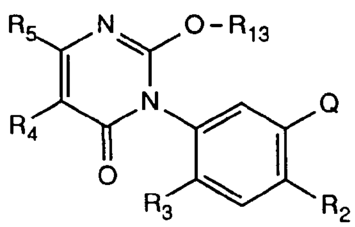

(Ib),

entsprechen, worin $R_2$, $R_3$, $R_4$, $R_5$, $R_{13}$ und Q die in Anspruch 1 angegebenen Bedeutungen besitzen.

**4.** 3-Aryluracil-Derivate gemäss einem der Ansprüche 1 oder 2, worin $R_1$ $C_1$-$C_4$-Alkyl, $C_1$-$C_4$-Haloalkyl, $C_3$- oder $C_4$-Alkenyl oder $C_3$- oder $C_4$-Alkinyl bedeutet.

**5.** 3-Aryluracil-Derivate gemäss einem der Ansprüche 1 bis 4, worin

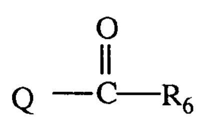

(1)

ist, wobei $R_6$ die in Anspruch 1 angegebene Bedeutung hat.

**6.** 3-Aryluracil-Derivate gemäss einem der Ansprüche 1 bis 4, worin

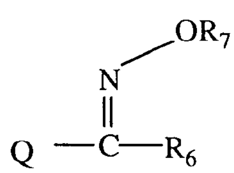

(2)

ist, wobei $R_6$ und $R_7$ die in Anspruch 1 angegebenen Bedeutungen besitzen.

**7.** 3-Aryluracil-Derivate gemäss einem der Ansprüche 1 bis 4, worin Q

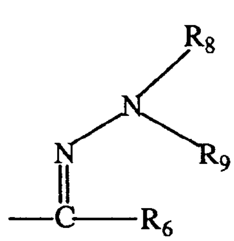

(3) ist,

wobei $R_6$, $R_8$ und $R_9$ die in Anspruch 1 angegebenen Bedeutungen besitzen.

**8.** 3-Aryluracil-Derivate gemäss einem der Ansprüche 1 bis 4, worin

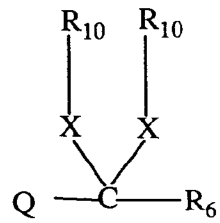 (4)

ist, wobei $R_6$, $R_{10}$ und X die in Anspruch 1 angegebenen Bedeutungen besitzen.

**9.** 3-Aryluracil-Derivate gemäss einem der Ansprüche 1 bis 4, worin Q

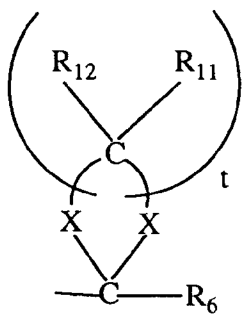 (5) ist,

wobei $R_6$, $R_{11}$, $R_{12}$, t und X die in Anspruch 1 angegebenen Bedeutungen besitzen.

**10.** 3-Aryluracil-Derivate gemäss Anspruch 5, worin $R_6$ Wasserstoff, $C_1$-$C_5$-Alkyl oder $C_3$-$C_6$-Cycloalkyl bedeutet.

**11.** 3-Aryluracil-Derivate gemäss Anspruch 6, worin $R_6$ Wasserstoff oder $C_1$-$C_5$-Alkyl; und $R_7$ Wasserstoff, $C_1$-$C_5$-Alkyl, $C_3$-$C_5$-Alkenyl, $C_3$-$C_5$-Alkinyl oder Benzyl, bedeuten.

**12.** 3-Aryluracil-Derviate gemäss Anspruch 7, worin $R_6$ Wasserstoff oder $C_1$-$C_5$-Alkyl; und $R_8$ und $R_9$ je Methyl, bedeuten.

**13.** 3-Aryluracil-Derivate gemäss Anspruch 8, worin $R_6$ Wasserstoff, $C_1$-$C_5$-Alkyl oder $C_3$-$C_6$-Cycloalkyl; und $R_{10}$ $C_1$-$C_5$-Alkyl, $C_3$-$C_5$-Alkenyl, $C_3$-$C_5$-Alkinyl oder Hydroxy-$C_2$-$C_4$-alkyl, bedeuten.

**14.** 3-Aryluracil-Derivate gemäss Anspruch 9, worin $R_6$ Wasserstoff, $C_1$-$C_5$-Alkyl oder $C_3$-$C_6$-Cycloalkyl ist;
$R_{11}$ und $R_{12}$, unabhängig voneinander, Wasserstoff oder Methyl sind; und
t die Zahl 2 oder 3 bedeutet.

**15.** 3-Aryluracil-Derivate gemäss Anspruch 14, worin $R_{11}$ und $R_{12}$ Wasserstoff bedeuten.

**16.** 3-Aryluracil-Derivate gemäss Anspruch 2, worin $R_1$ Wasserstoff, Methyl, Allyl oder Propargyl;
$R_2$ Fluor, Chlor, Brom oder Cyano;
$R_3$ Wasserstoff oder Fluor;
$R_4$ Wasserstoff, Fluor oder Methyl;
$R_5$ Methyl, Trifluormethyl oder Pentafluorethyl ist; oder $R_4$ und $R_5$ zusammen $-(CH_2)_n-$ sind; und
n die Zahl 3 oder 4 bedeutet.

**17.** 3-Aryluracil-Derivate gemäss Anspruch 16, worin
$R_1$ Wasserstoff oder Methyl,
$R_2$ Fluor, Chlor, Brom oder Cyano,
$R_4$ Wasserstoff oder Fluor ist, oder $R_4$ und $R_6$ zusammen $-(CH_2)_n-$ sind, wobei n die Zahl 3 bedeutet.

**18.** 3-Aryluracil-Derivate gemäss Anspruch 3, worin

| | |
|---|---|
| $R_2$ | Fluor, Chlor, Brom oder Cyano; |
| $R_3$ | Wasserstoff oder Fluor, |
| $R_4$ | Wasserstoff, Fluor oder Methyl; |
| $R_5$ | Methyl, Trifluormethyl oder Pentafluorethyl ist; oder $R_4$ und $R_5$ zusammen $-(CH_2)_n-$ sind; |
| n | die Zahl 3 oder 4; und |
| $R_{13}$ | Methyl, Ethyl, Allyl oder Propargyl bedeutet. |

**19.** 3-Aryluracil-Derivate gemäss Anspruch 18, worin
$R_2$ Fluor, Chlor, Brom oder Cyano, $R_4$ Wasserstoff oder Fluor und n die Zahl 3 bedeutet.

**20.** 3-Aryluracil-Derivate gemäss Anspruch 1, worin -W- die Gruppe

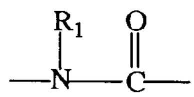

bedeutet;

| | |
|---|---|
| $R_1$ | Wasserstoff oder Methyl; |
| $R_2$ | Fluor, Chlor, Brom oder Cyano; |
| $R_3$ | Wasserstoff oder Fluor, |
| $R_4$ | Wasserstoff; |
| $R_5$ | Methyl, Trifluormethyl, Pentafluorethyl ist; oder |

$R_4$ und $R_5$ zusammen $-(CH_2)_n-$ sind;
n die Zahl 3; und
Q eine der Gruppen a) bis e) bedeutet:

a)

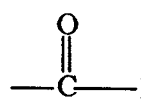
(1),

wobei $R_6$ Wasserstoff, $C_1$-$C_5$-Alkyl oder $C_3$-$C_6$-Cycloalkyl;

b)

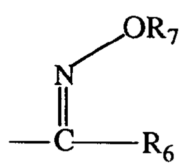
(2),

wobei $R_6$ die unter a) angegebene Bedeutung besitzt; und $R_7$ Wasserstoff, $C_1$-$C_4$-Alkyl, Allyl oder Benzyl;

c)

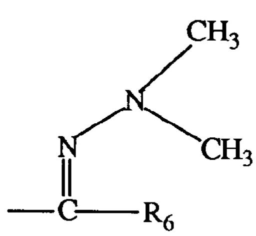
(3),

wobei $R_6$ die unter a) angegebene Bedeutung besitzt;

d)

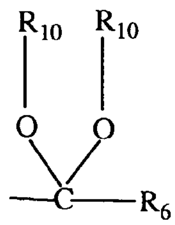 (4),

wobei $R_6$ die unter a) angegebene Bedeutung besitzt; und $R_{10}$ $C_1$-$C_5$-Alkyl oder Hydroxy-ethyl;
e)

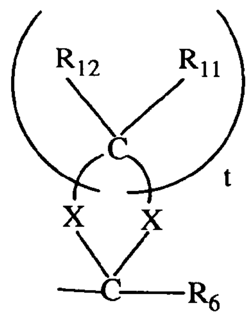 (5),

wobei $R_6$ die unter a) angegebene Bedeutung besitzt; $R_{11}$ und $R_{12}$ je Wasserstoff sind; t die Zahl 2 oder 3; oder $R_{11}$ Wasserstoff und $R_{12}$ Methyl; n die Zahl 2; und X Sauerstoff oder Schwefel bedeutet.

**21.** Eine Verbindung gemäss Anspruch 1 ausgewählt aus:
2-Chlor-5-[3,6-dihydro-2,6-dioxo-3-methyl-4-trifluormethyl-1(2H)-pyrimidinyl]-benzaldehyd,
2-Chlor-5-[3,6-dihydro-2,6-dioxo-3-methyl-4-trifluormethyl-1(2H)-pyrimidinyl]-acetophenon-oxim-methylether,
2-Chlor-5-[3,6-dihydro-2,6-dioxo-3-methyl-4-trifluormethyl-1(2H)-pyrimidinyl]-acetophenonethylenketal,
2-Chlor-5-[3,6-dihydro-2,6-dioxo-3-methyl-4-trifluormethyl-1(2H)-pyrimidinyl]-benzophenonethylenketal,
3-[4-Chlor-3-(3-methylbutyryl)phenyl]-1-methyl-6-trifluormethyl-2,4(1H,3H)-pyrimidindion,
2-Chlor-5-[3,6-dihydro-2,6-dioxo-3-methyl-4-trifluormethyl-1(2H)-pyrimidinyl]-acetophenon,
2-Chlor-5-[3,6-dihydro-2,6-dioxo-3-methyl-4-trifluormethyl-1(2H)-pyrimidinyl]-phenyl-1-(i)propyl-keton,
2-Chlor-5-[3,6-dihydro-2,6-dioxo-3-methyl-4-trifluormethyl-1(2H)-pyrimidinyl]-phenyl-1-(n)butyl-keton,
2-Chlor-5-[3,6-dihydro-2,6-dioxo-3-methyl-4-trifluormethyl-1(2H)-pyrimidinyl]-phenyl-1-(3-methylbutyl)-keton,
2-Chlor-5-[3,6-dihydro-2,6-dioxo-3-methyl-4-trifluormethyl-1(2H)-pyrimidinyl]-phenyl-1-cyclopropyl-keton,
2-Chlor-5-[3,6-dihydro-2,6-dioxo-3-methyl-4-trifluormethyl-1(2H)-pyrimidinyl]-phenyl-1-cyclopentyl-keton,
2-Chlor-5-[3,6-dihydro-2,6-dioxo-3-methyl-4-trifluormethyl-1(2H)-pyrimidinyl]-phenyl-1-cyclohexyl-keton,
2-Chlor-4-fluor-5-[3,6-dihydro-2,6-dioxo-3-methyl-4-trifluormethyl-1(2H)-pyrimidinyl]-acetophenon,
2-Chlor-4-fluor-5-[3,6-dihydro-2,6-dioxo-3,4-dimethyl-1(2H)-pyrimidinyl]-acetophenon, 2,4-Difluor-5-[3,6-dihydro-2,6-dioxo-3-methyl-4-trifluormethyl-1(2H)-pyrimidinyl]-acetophenon,
2-Chlor-5-[3,6-dihydro-2,6-dioxo-3-methyl-4-pentafluorethyl-1(2H)-pyrimidinyl]phenyl-1-(i)propyl-keton,
2-Chlor-5-[3,6-dihydro-2,6-dioxo-3-methyl-4-trifluormethyl-1(2H)-pyrimidinyl]-propiophenon,
2-Chlor-5-[3,6-dihydro-2,6-dioxo-3-methyl-4-pentafluorethyl-1(2H)-pyrimidinyl]-propiophenon,
2-Chlor-5-[3,6-dihydro-2,6-dioxo-3-methyl-4-trifluormethyl-1(2H)-pyrimidinyl]-phenyl-1-(n)propyl-keton,
2-Brom-5-[3,6-dihydro-2,6-dioxo-3-methyl-4-trifluormethyl-1(2H)-pyrimidinyl]-acetophenon,
2-Fluor-5-[3,6-dihydro-2,6-dioxo-3-methyl-4-trifluormethyl-1(2H)-pyrimidinyl]-acetophenon,
2-Chlor-4-fluor-5-[3,6-dihydro-2,6-dioxo-3,4-dimethyl-1(2H)-pyrimidinyl]-propiophenon,
2-Chlor-4-fluor-5-[1,2,4,5,6,7-hexahydro-1-methyl-2,4-dioxo-3H-cyclopenta[d]-pyrimidin-3-yl]-acetophenon,
2-Chlor-5-[3,6-dihydro-2,6-dioxo-3-methyl-4-trifluormethyl- 1(2H)-pyrimidinyl]-4-fluorbenzaldehyd,
2-Chlor-5-[3,6-dihydro-2,6-dioxo-3-methyl-4-trifluormethyl-1(2H)-pyrimidinyl]-acetophenonoxim,
2-Chlor-5-[3,6-dihydro-2,6-dioxo-3-methyl-4-trifluormethyl-1(2H)-pyrimidinyl] -acetophenonoxim-ethylether,
2-Chlor-5-[3,6-dihydro-2,6-dioxo-3-methyl-4-trifluormethyl-1(2H)-pyrimidinyl]-acetophenonoxim-isopropylether,
2-Chlor-5-[3,6-dihydro-2,6-dioxo-3-methyl-4-trifluormethyl-1(2H)-pyrimidinyl]-acetophenonoxim-allylether,

2-Chlor-5-[3,6-dihydro-2,6-dioxo-3-methyl-4-trifluormethyl-1(2H)-pyrimidinyl]-acetophenonoxim-benzylether,
2-Chlor-5-[3,6-dihydro-2,6-dioxo-3-methyl-4-trifluormethyl-1(2H)-pyrimidinyl]-benzaloxim
2-Chlor-5-[3,6-dihydro-2,6-dioxo-3-methyl-4-trifluormethyl-1(2H)-pyrimidinyl]-acetophenonoxim-(i)butylether,
2-Chlor-5-[3,6-dihydro-2,6-dioxo-3-methyl-4-trifluormethyl-1(2H)-pyrimidinyl]-acetophenon-N,N-dimethylhydrazon,
2-Chlor-5-[3,6-dihydro-2,6-dioxo-3-methyl-4-trifluormethyl-1(2H)-pyrimidinyl]-benzaldehyd-N,N-dimethylhydrazon,
2-Chlor-5-[3,6-dihydro-2,6-dioxo-3-methyl-4-trifluormethyl-1(2H)-pyrimidinyl]-acetophenon-methylethylenacetal,
2-Chlor-5-[3,6-dihydro-2,6-dioxo-3-methyl-4-trifluormethyl-1(2H)-pyrimidinyl]-acetophenon-propylenacetal,
2-Chlor-5-[3,6-dihydro-2,6-dioxo-3-methyl-4-trifluormethyl-1(2H)-pyrimidinyl]-benzaldehyd-dimethoxyacetal,
2-Chlor-5-[3,6-dihydro-2,6-dioxo-3-methyl-4-trifluormethyl-1(2H)-pyrimidinyl]-benzaldehyd-ethylenacetal,
2-Chlor-5-[3,6-dihydro-2,6-dioxo-3-methyl-4-trifluormethyl-1(2H)-pyrimidinyl]-acetophenon-propylendithioacetal,
2-Chlor-5-[3,6-dihydro-2,6-dioxo-3-methyl-4-trifluormethyl-1(2H)-pyrimidinyl]-acetophenon-ethylendithioacetal,
2-Chlor-5-[3,6-dihydro-2,6-dioxo-3-methyl-4-trifluormethyl-1(2H)-pyrimidinyl]-acetophenon-methylethylendithioacetal,
2-Chlor-5-[3,6-dihydro-2,6-dioxo-3-methyl-4-trifluormethyl-1(2H)-pyrimidinyl]-benzaldehyd-isopropyloxim.

**22.** Verbindungen gemäss der Formel IIa oder IIb

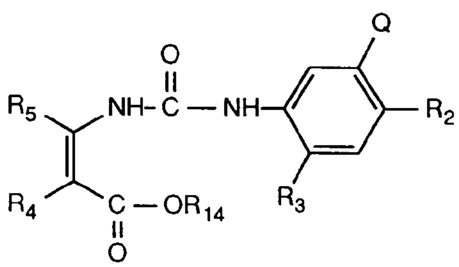 (IIa) oder 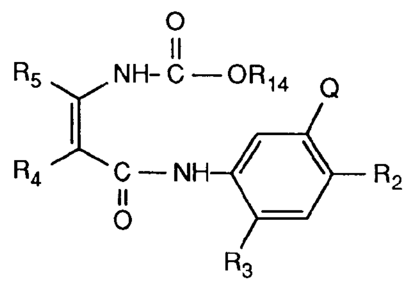 (IIb),

worin

| | |
|---|---|
| $R_2$ | Halogen oder Cyano; |
| $R_3$ | Wasserstoff oder Halogen; |
| $R_4$ | Wasserstoff, Halogen oder $C_1$-$C_4$-Alkyl; |
| $R_5$ | $C_1$-$C_4$-Alkyl oder $C_1$-$C_4$-Haloalkyl ist; oder |

$R_4$ und $R_5$ zusammen $-(CH_2)_n-$ sind;

| | |
|---|---|
| n | die Zahl 3 oder 4; |
| $R_{14}$ | $C_1$-$C_6$-Alkyl, insbesondere $C_1$-$C_4$-Alkyl; und |
| Q | eine der Gruppen a) bis e) bedeuten: |

a)

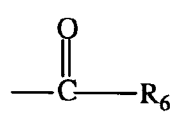 (1),

wobei $R_6$ Wasserstoff, $C_1$-$C_8$-Alkyl, $C_3$-$C_7$-Cycloalkyl oder $C_3$-$C_7$-Cycloalkyl-$C_1$-$C_4$-alkyl ist;

b)

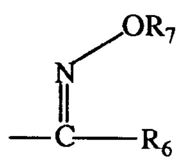 (2),

wobei $R_6$ die unter a) angegebene Bedeutung hat; und $R_7$ Wasserstoff, $C_1$-$C_8$-Alkyl, $C_2$-$C_8$-Al-

kenyl, $C_2$-$C_8$-Alkinyl, $C_3$-$C_7$-Cycloalkyl, Phenyl, mit Halogen, $C_1$-$C_4$-Alkyl oder $C_1$-$C_4$-Alkoxy substituiertes Phenyl, Benzyl oder mit Halogen, $C_1$-$C_4$-Alkyl oder $C_1$-$C_4$-Alkoxy substituiertes Benzyl ist;

c)

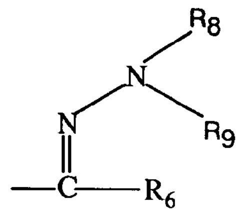

(3),

wobei $R_6$ die unter a) angegebene Bedeutung hat; $R_8$ und $R_9$, unabhängig voneinander, Wasserstoff, $C_1$-$C_8$-Alkyl, $C_2$-$C_8$-Alkenyl, $C_2$-$C_8$-Alkinyl, Phenyl, mit Halogen, $C_1$-$C_4$-Alkyl oder $C_1$-$C_4$-Alkoxy substituiertes Phenyl, Benzyl oder mit Halogen, $C_1$-$C_4$-Alkyl oder $C_1$-$C_4$-Alkoxy substituiertes Benzyl; oder $R_8$ und $R_9$ zusammen $-(CH_2)_m-$ sind; und m die Zahl 3, 4 oder 5 ist,

d)

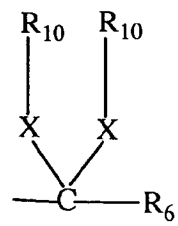

(4),

wobei $R_6$ die unter a) angegebene Bedeutung hat; $R_{10}$ $C_1$-$C_8$-Alkyl, $C_2$-$C_8$-Alkenyl, $C_2$-$C_8$-Alkinyl, $C_1$-$C_4$-Alkoxy-$C_1$-$C_6$-alkyl, Hydroxy-$C_1$-$C_6$-alkyl oder $C_1$-$C_8$-Haloalkyl; und X Sauerstoff oder Schwefel ist;

e)

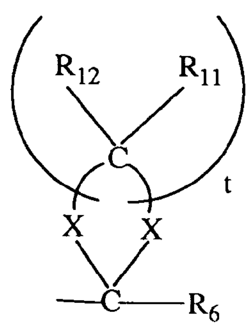

(5),

wobei $R_{11}$ und $R_{12}$, unabhängig voneinander, Wasserstoff oder $C_1$-$C_4$-Alkyl sind; t die Zahl 2, 3 oder 4; X Sauerstoff oder Schwefel ist; und $R_6$ die unter a) angegebene Bedeutung hat.

**23.** Verbindungen gemäss der Formel IV

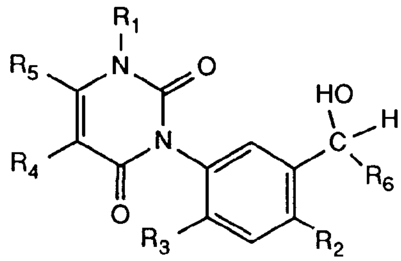

(IV),

worin

$R_1$ Wasserstoff, $C_1$-$C_4$-Alkyl, $C_1$-$C_4$-Haloalkyl, $C_3$- oder $C_4$-Alkenyl oder $C_3$- oder $C_4$-Alkinyl;

$R_6$ Wasserstoff, $C_1$-$C_8$-Alkyl, $C_3$-$C_7$-Cycloalkyl oder $C_3$-$C_7$-Cycloalkyl-$C_1$-$C_4$-alkyl ist; und

$R_2$, $R_3$, $R_4$ und $R_5$ die in Anspruch 22 angegebenen Bedeutungen besitzen;

**24.** Verbindungen gemäss der Formel V

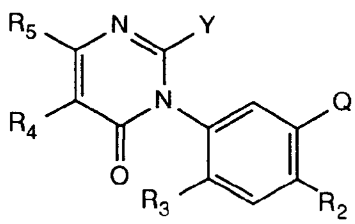

(V),

worin Y Halogen; und $R_2$, $R_3$, $R_4$, $R_5$ und Q die in Anspruch 22 angegebenen Bedeutungen besitzen.

**25.** Verbindungen gemäss der Formel VI

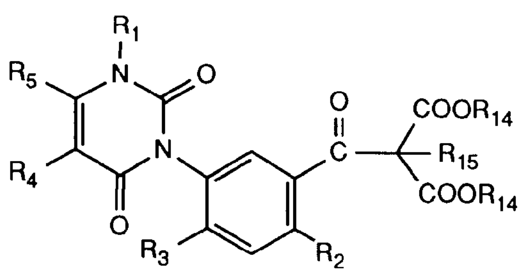

(VI),

worin $R_2$, $R_3$, $R_4$ und $R_5$ die in Anspruch 22 angegebenen Bedeutungen besitzen;

$R_1$ Wasserstoff, $C_1$-$C_4$-Alkyl, $C_1$-$C_4$-Haloalkyl, $C_3$- oder $C_4$-Alkenyl oder $C_3$- oder $C_4$-Alkinyl;

$R_{14}$ $C_1$-$C_6$-Alkyl, insbesondere $C_1$-$C_4$-Alkyl; und

$R_{15}$ Wasserstoff, $C_1$-$C_7$-Alkyl, $C_3$-$C_7$-Cycloalkyl oder $C_3$-$C_7$-Cycloalkyl-$C_1$-$C_3$-alkyl bedeutet.

**26.** Verfahren zur Herstellung von Verbindungen der Formel I gemäss Anspruch 1, dadurch gekennzeichnet, dass man eine Verbindung der Formel IIa oder IIb

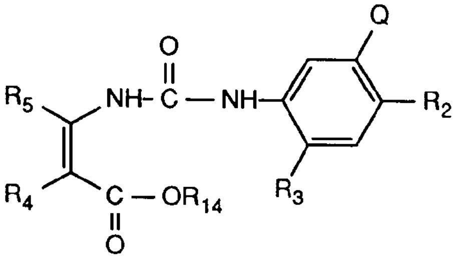

(IIa) oder

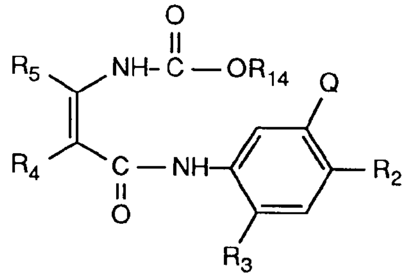

(IIb),

unter basischen Bedingungen zu einem Salz der Formel IIc

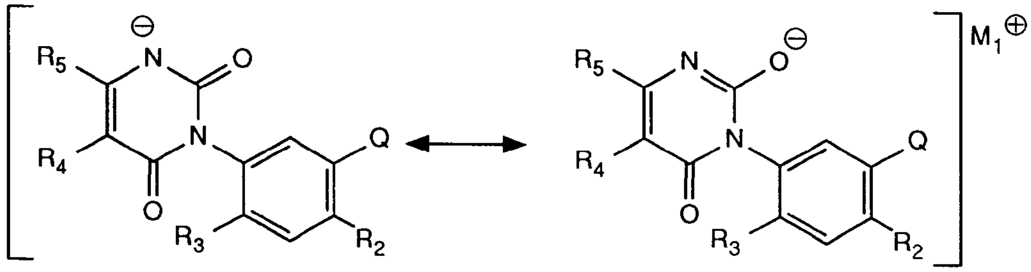

(IIc)

cyclisiert, wobei in den Formeln IIa, IIb und IIc die Reste $R_2$, $R_3$, $R_4$, $R_5$, $R_{14}$ und Q die in Anspruch 22 angegebenen Bedeutungen besitzen, $M_1^{\oplus}$ ein Kation ist, und gegebenenfalls die erhaltenen Salze in Gegenwart einer Säure in die Verbindungen mit $R_1$ Wasserstoff, oder in Gegenwart eines Alkylierungsmittels in die 3-Aryluracil-Derivate, worin $R_1$ $C_1$-$C_4$-Alkyl, $C_3$-$C_4$-Alkenyl oder $C_3$-$C_4$-Alkinyl bedeutet, überführt.

**27.** Verfahren zur Herstellung von Verbindungen der Formel I gemäss Anspruch 1, dadurch gekennzeichnet, dass man eine Verbindung der Formel III

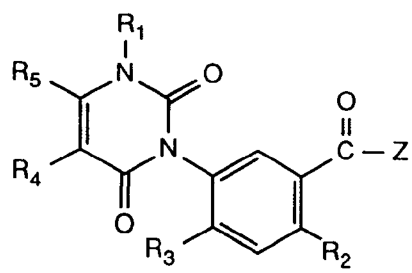

(III),

worin $R_1$ Wasserstoff, $C_1$-$C_4$-Alkyl, $C_1$-$C_4$-Haloalkyl, $C_3$- oder $C_4$-Alkenyl oder $C_3$- oder $C_4$-Alkinyl; Z Halogen, vorzugsweise Chlor, oder eine Abgangsgruppe, vorzugsweise N,O-Dimethyl-hydroxyl-amino ist; und $R_2$, $R_3$, $R_4$ und $R_5$ die in Anspruch 22 angegebenen Bedeutungen besitzen, mit einer metallorganischen Verbindung der Formel VIIa oder VIIb

$R_{5'}$-$M_2$ (VIIa) oder $R_{6'}$-$M_3$-G (VIIb),

worin $R_{5'}$ $C_1$-$C_8$-Alkyl, $C_3$-$C_7$-Cycloalkyl oder $C_3$-$C_7$-Cycloalkyl-$C_1$-$C_4$-alkyl;

$M_2$ ein Alkalimetallion, insbesondere Lithium;

$M_3$ ein Erdalkalimetallion oder ein Metall der ersten oder zweiten Nebengruppe des Periodensystems, insbesondere Magnesium; und

G Halogen, insbesondere Chlor oder Brom, bedeuten, umsetzt.

**28.** Verfahren zur Herstellung von Verbindungen der Formel I gemäss Anspruch 1, dadurch gekennzeichnet, dass man eine Verbindung der Formel IV

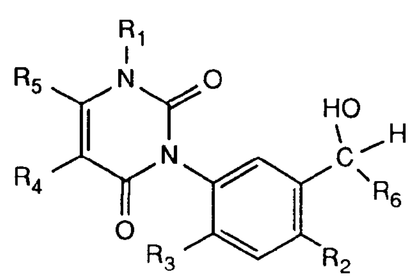

(IV),

worin $R_1$, $R_2$, $R_3$, $R_4$, $R_5$ und $R_5$ die in Anspruch 23 angegebenen Bedeutungen besitzen, oxydiert.

**29.** Unkrautbekämpfungsmittel, gekennzeichnet durch einen Gehalt an mindestens einer Verbindung der Formel I oder deren Salze gemäss Anspruch 1.

30. Verfahren zur Bekämpfung von Unkräutern, dadurch gekennzeichnet, dass man die gegen Unkräuter zu schützenden Kulturpflanzen und/oder die Unkräuter mit einem Wirkstoff der Formel I gemäss einem der Ansprüche 1 bis 21 oder mit einem diesen Wirkstoff enthaltenden Mittel gemäss Anspruch 29 behandelt.

Europäisches Patentamt

# EUROPÄISCHER RECHERCHENBERICHT

Nummer der Anmeldung
EP 92 81 0853

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | WO-A-8 902 891 (F. HOFFMANN-LA ROCHE & CO. A.G.)<br>* das ganze Dokument *<br>--- | 1-30 | C07D239/54<br>C07D239/70<br>C07D405/10<br>C07D239/36<br>C07C275/42<br>C07C237/18<br>A01N43/54 |
| A | WO-A-8 903 825 (F. HOFFMANN-LA ROCHE & CO. A.G.)<br>* das ganze Dokument *<br>--- | 1-30 | |
| A,D | EP-A-0 195 346 (F. HOFFMANN-LA ROCHE & CO. A.G.)<br>* das ganze Dokument *<br>--- | 1-30 | |
| A | EP-A-0 408 382 (NISSAN CHEMICAL INDUSTRIES LTD.)<br>* Seite 50; Tabelle I.c *<br>--- | 1-30 | |
| A,D | EP-A-0 260 621 (F. HOFFMANN-LA ROCHE & CO. A.G.)<br>* das ganze Dokument *<br>--- | 1-30 | |
| A | WO-A-9 100 278 (CIBA-GEIGY A.G.)<br>* das ganze Dokument *<br>--- | 1-30 | RECHERCHIERTE SACHGEBIETE (Int. Cl.5) |
| A,P | EP-A-0 473 551 (CIBA-GEIGY A.G.)<br>* das ganze Dokument *<br>----- | 1-30 | C07D<br>C07C<br>A01N |

Der vorliegende Recherchenbericht wurde für alle Patentansprüche erstellt

| Recherchenort | Abschlußdatum der Recherche | Prüfer |
|---|---|---|
| BERLIN | 16 DEZEMBER 1992 | FRELON D. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)